# EUROPEAN PATENT SPECIFICATION

(11) **EP 1 224 297 B1**
(45) Date of publication and mention of the grant of the patent: **13.05.2009**
(21) Application number: 00965338.7
(22) Date of filing: 22.09.2000
(51) Int. Cl.: C12N 15/57, C12N 9/64, G01N 33/68, C12Q 1/37, C12N 1/21, C07K 14/47, C12N 5/10

(54) **ALZHEIMER'S DISEASE SECRETASE, APP SUBSTRATES THEREFOR, AND USES THEREFOR**
ALZHEIMER KRANKHEIT SEKRETASE, APP (AMYLOID VORLAÜFER-PROTEIN) SUBSTRATE DAFÜR UND VERWENDUNGEN
SECRETASE DE LA MALADIE D'ALZHEIMER, SUBSTRATS DE LA PROTEINE PRECURSEUR D'AMYLASE (APP) APPROPRIES ET PROCEDES D'UTILISATION

(30) Priority: 23.09.1999 US 404133 P; 23.09.1999 US 155493 P; 13.10.1999 US 416901; 06.12.1999 US 169232 P
(43) Date of publication of application: 24.07.2002
(60) Divisional application: 02015537.0
(73) Proprietor: Pharmacia & Upjohn Company LLC, Kalamazoo, MI 49001 (US)
(72) Inventor: GURNEY, Mark, 110 Reykjavik (IS); BIENKOWSKI, Michael, Jerome, Portage, MI 49024 (US)
(74) Representative: Perry, Robert Edward
(86) International application number: PCT/US2000/026080
(87) International publication number: WO 2001/023533

(56) References cited:
- EP-A- 0 783 104
- EP-A- 0 848 062
- WO-A-00/17369
- WO-A-99/33963
- LANNFELT L ET AL: "DECREASED ALPHA-SECRETASE-CLEAVED AMYLOID PRECURSOR PROTEIN AS A DIAGNOSTIC MARKER FOR ALZHEIMER'S DISEASE" NATURE MEDICINE,US,NATURE PUBLISHING, CO, vol. 1, no. 8, 1 August 1995 (1995-08-01), pages 829-832, XP000673085 ISSN: 1078-8956

## Description

### FIELD OF THE INVENTION

The present invention relates to a method for assaying human Aspartyl Protease 1 (hu-Asp1) α-secretase activity.

### BACKGROUND OF THE INVENTION

Alzheimer's disease (AD) causes progressive dementia with consequent formation of amyloid plaques, neurofibrillary tangles, gliosis and neuronal loss. The disease occurs in both genetic and sporadic forms whose clinical course and pathological features are quite similar. Three genes have been discovered to date which, when mutated, cause an autosomal dominant form of Alzheimer's disease. These encode the amyloid protein precursor (APP) and two related proteins, presenilin-1 (PS1) and presenilin-2 (PS2), which, as their names suggest, are structurally and functionally related. Mutations in any of the three proteins have been observed to enhance proteolytic processing of APP via an intracellular pathway that produces amyloid beta peptide (Aβ peptide, or sometimes here as Abeta), a 40-42 amino acid long peptide that is the primary component of amyloid plaque in AD.

Dysregulation of intracellular pathways for proteolytic processing may be central to the pathophysiology of AD. In the case of plaque formation, mutations in APP, PS1 or PS2 consistently alter the proteolytic processing of APP so as to enhance formation of Aβ 1-42, a form of the Aβ peptide which seems to be particularly amyloidogenic, and thus very important in AD. Different forms of APP range in size from 695-770 amino acids, localize to the cell surface, and have a single C-terminal transmembrane domain. Examples of specific isotypes of APP which are currently known to exist in humans are the 695-amino acid polypeptide described by Kang et. al. (1987), Nature 325: 733-736 which is designated as the "normal" APP; the 751 amino acid polypeptide described by Ponte et al. (1988), Nature 331: 525-527 (1988) and Tanzi et al. (1988), Nature 331: 528-530; and the 770 amino acid polypeptide described by Kitaguchi et. al. (1988), Nature 331: 530-532. The Abeta peptide is derived from a region of APP adjacent to and containing a portion of the transmembrane domain. Normally, processing of APP at the α-secretase site cleaves the midregion of the Aβ sequence adjacent to the membrane and releases the soluble, extracellular domain of APP from the cell surface. This α-secretase APP processing creates soluble APP- α, (sAPPα) which is normal and not thought to contribute to AD.

Pathological processing of APP at the β- and γ-secretase sites, which are locatcd N-terminal and C-terminal to the α-secretase site, respectively, produces a very different result than processing at the α site. Sequential processing at the β- and γ-secretase sites releases the Aβ peptide, a peptide possibly very important in AD pathogenesis. Processing at the β- and γ-secretase sites can occur in both the endoplasmic reticulum (in neurons) and in the endosomal/lysosomal pathway after reinternalization of cell surface APP (in all cells).

### SUMMARY OF THE INVENTION

According to the present invention, a method for assaying hu-Asp1 α-secretase activity, comprises the steps of:
(a) contacting a hu-Aspl polypeptide which comprises an amino acid sequence at least 95% identical to a fragment, including the aspartyl protease active site tripeptides DTG and DSG, of the amino acid sequence of SEQ ID NO:2, wherein the polypeptide and the fragment retain α-secretase activity, with an amyloid precursor protein (APP) substrate which contains an α-secretase cleavage site; and
(b) measuring cleavage of the APP substrate at the hu-Asp1 α-secretase cleavage site.

The invention provides for methods for assaying for human Asp1(hu-Asp1) α-secretase activity comprising contacting the hu-Asp 1 protein with an amyloid precursor protein (APP) substrate, wherein the substrate contains an α-secretase cleavage site; and measuring cleavage of the APP substrate at the α-secretase cleavage site, thereby assaying hu-Asp1 α-secretase activity. An example of α-secretase activity is APP processing wherein the APP substrate is cleaved at a site adjacent to the cell membrane (at residues Phe^{20 ₁}Ala²¹ in relation to the Aβ peptide). This cleavage results in the release of a soluble, extracellular domain of APP, known as amyloid alpha peptide (sAPPα), from the cell surface into the cytoplasm. The sAPPα within the cytoplasm can be detected and quantitated, thereby measuring α-secretase activity.

The hu-Asp1 enzyme used in the methods of the invention can be purified and isolated from a cell which is transfected or transformed with a polynucleotide that encodes hu-Asp 1, such as SEQ ID NO: 1, or a polynucleotide sequence that encodes the the amino acid sequence of SEQ ID NO: 2. Further, the hu-Asp1 protein used in the methods may be a fragment of the amino acid sequence of SEQ ID NO: 2 which retains α-secretase activity. Possible fragments that may be of use for the methods include those lacking the transmembrane domain amino acids 469-492 of SEQ ID NO: 2, those fragments that lack the cytoplasmic amino acids 493-492 of SEQ ID NO: 2, those fragments that lack the amino terminal amino acids 1-62 of SEQ ID NO: 2 or combinations thereof.

The invention also encompasses methods of assaying for α-secretase activity where hu-Asp1 protein and its substrate are brought into contact by a growing cell transfected or transformed with a polynucleotide encoding the hu-Asp1 protein or a fragment thereof that retains α-secretase activity under conditions where the cell expresses hu-Asp1 protein in the presence of the APP substrate. The APP substrate in such circumstances can be exogenously introduced, or more preferably, is expressed by the cell that expresses Asp 1. These methods also encompass contacting hu-Asp 1 protein with a cell that expresses a polynucleotide that encodes an APP substrate containing an α-secretase cleavage site. For example, the cell may express a polynucleotide that encodes a polypeptide having an α-secretase cleavage site comprising the amino acid sequence LVFFAEDF or KLVFFAED. In addition, the APP substrate may comprise any human isoform of APP, such as "normal" APP (APP695), APP 751, or APP770. These APP substrates can be further modified to comprise a carboxy-terminal di-lysine motif.

To measure the cleavage of the substrates for the methods of assaying for α-secretase activity of the invention, the substrates of the method can be further modified to comprise detectable labels such as radioactive, enzymatic, chemilumenescent or flourescent labels. In particular, shorter peptide substrates preferably comprise internally quenched labels that result in increased detectability after cleavage of the peptide substrates. The peptide substrates may be modified to have attached a paired flurophore and quencher including but not limited to 7-amino-4-methyl coumarin and dinitrophenol, respectively, such that cleavage of the peptide by the hu-Asp1 results in increased fluorescence due to physical separation of the flurophore and quencher. Other paired flurophores and quenchers include bodipy-tetramethylrhodamine and QSY-5 (Molecular Probes, Inc.) In a variant of this assay, biotin or another suitable tag may be placed on one end of the peptide to anchor the peptide to a substrate assay plate and a flurophore may be placed at the other end of the peptide. Useful flurophores include those listed above as well as Europium labels such as W8044 (EG&G Wallac, Inc.). A preferred label is oregon green that may be attached to a Cys residue. Cleavage of the peptide by Asp1 will release the flurophore or other tag from the plate, allowing compounds to be assayed for inhibition of Asp1 proteolytic cleavage as shown by an increase in retained fluorescence. Preferred colorimetric assays of hu-Asp1 proteolytic activity utilize other suitable substrates that include the P₂ and P₁ amino acids comprising the recognition site for cleavage linked to o-nitrophenol through an amide linkage, such that cleavage by the hu-Asp1 results in an increase in optical density after altering the assay buffer to alkaline pH.

### DETAILED DESCRIPTION OF THE INVENTION

A few definitions used in this invention follow, most definitions to be used are those that would be used by one ordinarily skilled in the art.

The term "β amyloid peptide" means any peptide resulting from beta secretase cleavage of APP. This includes peptides of 39, 40, 41, 42 and 43 amino acids, extending from the β-secretase cleavage site to 39, 40, 41, 42 and 43 amino acids C-terminal to the β-secretase cleavage site. β amyloid peptide also includes sequences 1-6, SEQ ID NOs. 1-6 of US 5,750,349, issued 12 May 1998 (incorporated into this document by reference). A β-secretase cleavage fragment disclosed here is called CTF-99, which extends from β-secretase cleavage site to the carboxy terminus of APP.

When an isoform of APP is discussed then what is meant is any APP polypeptide, including APP variants (including mutations), and APP fragments that exists in humans such as those described in US 5,766,846, col 7, lines 45-67, incorporated into this document by reference.

The term "β-amyloid precursor protein" (APP) as used herein is defined as a polypeptide that is encoded by a gene of the same name localized in humans on the long arm of chromosome 21 and that includes "βAP - here "β-amyloid protein" see above, within its carboxyl third. APP is a glycosylated, single-membrane spanning protein expressed in a wide variety of cells in many mammalian tissues. Examples of specific isotypes of APP which are currently known to exist in humans are the 695 amino acid polypeptide described by Kang et. al. (1987) Nature 325:733-736 which is designated as the "normal" APP (SEQ ID NOs: 9-10); the 751 amino acid polypeptide described by Ponte et al. (1988) Nature 331:525-527 (1988) and Tanzi et al. (1988) Nature 331:528-530 (SEQ ID NOs: 56-57); and the 770-amino acid polypeptide described by Kitaguchi et. al. (1988) Nature 331:530-532 (SEQ ID NOs: 54-55). Examples of specific variants of APP include point mutation which can differ in both position and phenotype (for review of known variant mutation see Hardy (1992) Nature Genet. 1:233-234). The term "APP fragments" as used herein refers to fragments of APP other than those which consist solely of βAP or βAP fragments. That is, APP fragments will include amino acid sequences of APP in addition to those which form intact βAP or a fragment of βAP.

When the term "any amino acid" is used, the amino acids referred to are to be selected from the following, three letter and single letter abbreviations - which may also be used, are provided as follows:
Alanine, Ala, A; Arginine, Arg, R; Asparagine, Asn, N; Aspartic acid, Asp, D; Cysteine, Cys, C; Glutamine, Gln, Q; Glutamic Acid, Glu, E; Glycine, Gly, G; Histidine, His, H; Isoleucine, Ile, I; Leucine, Leu, L; Lysine, Lys, K; Methionine, Met, M; Phenylalanine, Phe, F; Proline, Pro, P; Serine, Ser, S; Threonine, Thr, T; Tryptophan, Trp, W; Tyrosine, Tyr, Y; Valine, Val, V; Aspartic acid or Asparagine, Asx, B; Glutamic acid or Glutamine, Glx, Z; Any amino acid, Xaa, X.

The predicted amino acid sequence of Hu-Asp1, shares significant homology with previously identified mammalian aspartyl proteases such as pepsinogen A, pepsinogen B, cathepsin D, cathepsin E, and renin. P.B.Szecs, Scand. J. Clin. Lab. Invest. 52:(Suppl. 210 5-22 (1992)). These enzymes are characterized by the presence of a duplicated DTG/DSG sequence motif. The Hu-Asp1 polypeptide disclosed herein also exhibits extremely high homology with the ProSite consensus motif for aspartyl proteases extracted from the SwissProt database.

The nucleotide sequence given as residues 1-1554 of SEQ ID NO:1 corresponds to the nucleotide sequence encoding Hu-Asp 1. The isolation and sequencing of DNA encoding Hu-Asp1 is described below in Examples 1 and 2.

As is described in Examples 1 and 2, automated sequencing methods were used to obtain the nucleotide sequence of Hu-Asp1. The Hu-Asp nucleotide sequences were obtained for both DNA strands, and are believed to be 100% accurate. However, as is known in the art, nucleotide sequence obtained by such automated methods may contain some errors. Nucleotide sequences determined by automation are typically at least about 90%, more typically at least about 95% to at least about 99.9% identical to the actual nucleotide sequence of a given nucleic acid molecule. The actual sequence may be more precisely determined using manual sequencing methods, which are well known in the art. An error in sequence which results in an insertion or deletion of one or more nucleotides may result in a frame shift in translation such that the predicted amino acid sequence will differ from that which would be predicted from the actual nucleotide sequence of the nucleic acid molecule, starting at the point of the mutation.

Suitable host cells for expression of Hu-Asp polypeptides includes prokaryotes, yeast, and higher eukaryotic cells. Suitable prokaryotic hosts to be used for the expression of Hu-Asp include bacteria of the genera *Escherichia, Bacillus, and Salmonella,* as well as members of the genera *Pseudomonas, Streptomyces,* and *Staphylococcus.* For expression in, *e.g., E. coli,* a Hu-Asp polypeptide may include an N-terminal methionine residue to facilitate expression of the recombinant polypeptide in a prokaryotic host. The N-terminal Met may optionally then be cleaved from the expressed Hu-Asp polypeptide. Other N-terminal amino acid residues can be added to the Hu-Asp polypeptide to facilitate expression in Escherichia coli including but not limited to the T7 leader sequence, the T7-caspase 8 leader sequence, as well as others leaders including tags for purification such as the 6-His tag (Example 9). Hu-Asp polypeptides expressed in E. coli may be shortened by removal of the cytoplasmic tail, the transmembrane domain, or the membrane proximal region. Hu-Asp polypeptides expressed in E. coli may be obtained in either a soluble form or as an insoluble form which may or may not be present as an inclusion body. The insoluble polypeptide may be rendered soluble by guanidine HCl, urea or other protein denaturants, then refolded into a soluble form before or after purification by dilution or dialysis into a suitable aqueous buffer. If the inactive proform of the Hu-Asp was produced using recombinant methods, it may be rendered active by cleaving off the prosegment with a second suitable protease such as human immunodeficiency virus protease.

Expression vectors for use in prokaryotic hosts generally comprises one or more phenotypic selectable marker genes. Such genes generally encode, *e.g*., a protein that confers antibiotic resistance or that supplies an auxotrophic requirement. A wide variety of such vectors are readily available from commercial sources. Examples include pSPORT vectors, pGEM vectors (Promega), pPROEX vectors (LTI, Bethesda, MD), Bluescript vectors (Stratagene), pET vectors (Novagen) and pQE vectors (Qiagen).

Hu-Asp may also be expressed in yeast host cells from genera including *Saccharomyces, Pichia,* and *Kluveromyces.* Preferred yeast hosts are *S. cerevisiae* and *P. pasioris.* Yeast vectors will often contain an origin of replication sequence from a 2T yeast plasmid, an autonomously replicating sequence (ARS), a promoter region, sequences for polyadenylation, sequences for transcription termination, and a selectable marker gene. Vectors replicable in both yeast and *E. coli* (termed shuttle vectors) may also be used. In addition to the above-mentioned features of yeast vectors, a shuttle vector will also include sequences for replication and selection in *E. coli.* Direct secretion of Hu-Asp polypeptides expressed in yeast hosts may be accomplished by the inclusion of nucleotide sequence encoding the yeast I-factor leader sequence at the 5' end of the Hu-Asp-encoding nucleotide sequence.

Insect host cell culture systems may also be used for the expression of Hu-Asp polypeptides. In a preferred embodiment, the Hu-Asp polypeptides of the invention are expressed using an insect cell expression system (*see* Example 10). Additionally, a baculovirus expression system can be used for expression in insect cells was reviewed by Luckow and Summers, Bio/Technology 6:47 (1988).

In another preferred embodiment, the Hu-Asp polypeptide is expressed in mammalian host cells. Nonlimiting examples of suitable mammalian cell lines include the COS7 line of monkey kidney cells (Gluzman et al., Cell 23:175 (1981)), human embyonic kidney cell line 293, and Chinese hamster ovary (CHO) cells. Preferably, Chinese hamster ovary (CHO) cells are used for expression of Hu-Asp proteins.

The choice of a suitable expression vector for expression of the Hu-Asp polypeptides of the invention will of course depend upon the specific mammalian host cell to be used, and is within the skill of the ordinary artisan. Examples of suitable expression vectors include pcDNA3 (Invitrogen) and pSVL (Pharmacia Biotech). A preferred vector for expression of Hu-Asp polypeptides is pcDNA3.1-Hygro (Invitrogen). Expression vectors for use in mammalian host cells may include transcriptional and translational control sequences derived from viral genomes. Commonly used promoter sequences and enhancer sequences which may be used in the present invention include, but are not limited to, those derived from human cytomegalovirus (CMV), Adenovirus 2, Polyoma virus, and Simian virus 40 (SV40). Methods for the construction of mammalian expression vectors are disclosed, for example, in Okayama and Berg (Mol. Cell. Biol. 3:280 (1983)); Cosman et al. (Mol. Immunol. 23:935 (1986)); Cosman et al. (Nature 312:768 (1984)); EP-A-0367566; and WO 91/18982.

Having generally described the invention, the same will be more readily understood by reference to the following examples, which are provided by way of illustration and are not intended as limiting.

### Example 1

### Development of a Search Algorithm Useful for the Identification of Aspartyl Proteases, and Identification of C. elegans Aspartyl Protease Genes in Wormpep 12

### Materials and Methods:

Classical aspartyl proteases such as pepsin and renin possess a two-domain structure which folds to bring two aspartyl residues into proximity within the active site. These are embedded in the short tripeptide motif DTG, or more rarely, DSG. The DTG or DSG active site motif appears at about residue 25-30 in the enzyme, but at about 65-70 in the proenzyme (prorenin, pepsinogen). This motif appears again about 150-200 residues downstream. The proenzyme is activated by cleavage of the N-terminal prodomain. This pattern exemplifies the double domain structure of the modem day aspartyl enzymes which apparently arose by gene duplication and divergence. Thus;

NH₂ ------------X------ D²⁵TG ---------------Y-------D^{Y+25}TG ----------------C

where X denotes the beginning of the enzyme, following the N-terminal prodomain, and Y denotes the center of the molecule where the gene repeat begins again.

In the case of the retroviral enzymes such as the HIV protease, they represent only a half of the two-domain structures of well-known enzymes like pepsin, cathepsin D, renin, etc. They have no prosegment, but are carved out of a polyprotein precursor containing the *gag* and *pol* proteins of the virus. They can be represented by:

NH₂--------- D²⁵TG --------------------- C100

This "monomer" only has about 100 aa, so is extremely parsimonious as compared to the other aspartyl protease "dimers" which have of the order of 330 or so aa, not counting the N-terminal prodomain.

The limited length of the eukaryotic aspartyl protease active site motif makes it difficult to search EST collections for novel sequences. EST sequences typically average 250 nucleotides, and so in this case would be unlikely to span both aspartyl protease active site motifs. Instead, we turned to the *C. elegans* genome. The *C. elegans* genome is estimated to contain around 13,000 genes. Of these, roughly 12,000 have been sequenced and the corresponding hypothetical open reading frame (ORF) has been placed in the database Wormpep12. We used this database as the basis for a whole genome scan of a higher eukaryote for novel aspartyl proteases, using an algorithm that we developed specifically for this purpose. The following AWK script for locating proteins containing two DTG or DSG motifs was used for the search, which was repeated four times to recover all pairwise combinations of the aspartyl motif.

```
 BEGIN {RS=">"} /* defines ">" as record separator for FASTA format */
 {
 pos = index($0,"DTG") /*finds "DTG" in record*/
 if (pos>0) {
       rest = substr($0,pos+3) /*get rest of record after first DTG*/
       pos2 = index(rest,"DTG") /*find second DTG*/
       if (pos2>0) printf ("%s%s\n",">",$0)} /*report hits*/
 }
 }
```

The AWK script shown above was used to search Wormpep12, which was downloaded from ftp.sanger.ac.uk/pub/databases/wormpep, for sequence entries containing at least two DTG or DSG motifs. Using AWK limited each record to 3000 characters or less. Thus, 35 or so larger records were eliminated manually from Wormpep12 as in any case these were unlikely to encode aspartyl proteases.

### Results and Discussion:

The Wormpep 12 database contains 12,178 entries, although some of these (<10%) represent alternatively spliced transcripts from the same gene. Estimates of the number of genes encoded in the *C*. *elegans* genome is on the order of 13,000 genes, so Wormpep12 may be estimated to cover greater than 90% of the *C. elegans* genome.

Eukaryotic aspartyl proteases contain a two-domain structure, probably arising from ancestral gene duplication. Each domain contains the active site motif D(S/T)G located from 20-25 amino acid residues into each domain. The retroviral (e.g., HIV protease) or retrotransposon proteases are homodimers of subunits which are homologous to a single eukaryotic aspartyl protease domain. An AWK script was used to search the Wormpep12 database for proteins in which the D(S/T)G motif occurred at least twice. This identified >60 proteins with two DTG or DSG motifs. Visual inspection was used to select proteins in which the position of the aspartyl domains was suggestive of a two-domain structure meeting the criteria described above.

In addition, the PROSITE eukaryotic and viral aspartyl protease active site pattern PS00141 was used to search Wormpep12 for candidate aspartyl proteases. (Bairoch A., Bucher P., Hofmann K., The PROSITE database: its status in 1997, Nucleic Acids Res. 24:217-221(1997)). This generated an overlapping set of Wormpep12 sequences. Of these, seven sequences contained two DTG or DSG motifs and the PROSITE aspartyl protease active site pattern. Of these seven, three were found in the same cosmid clone (F21F8.3, F21F8.4, and F21F8.7) suggesting that they represent a family of proteins that arose by ancestral gene duplication. Two other ORFs with extensive homology to F21F8.3, F21F8.4 and F21F8.7 are present in the same gene cluster (F21F8.2 and F21F8.6), however, these contain only a single DTG motif. Exhaustive BLAST searches with these seven sequences against Wormpep12 failed to reveal additional candidate aspartyl proteases in the *C. elegans* genome containing two repeats of the DTG or DSG motif.

BLASTX search with each *C. elegans* sequence against SWISS-PROT, GenPep and TREMBL revealed that R12H7.2 was the closest worm homologue to the known mammalian aspartyl proteases, and that T18H9.2 was somewhat more distantly related, while CEASP1, F21F8.3, F21F8.4, and F21F8.7 formed a subcluster which had the least sequence homology to the mammalian sequences.

### Discussion:

APP, the presenilins, and p35, the activator of cdk5, all undergo intracellular proteolytic processing at sites which conform to the substrate specificity of the HIV protease. Dysregulation of a cellular aspartyl protease with the same substrate specificity, might therefore provide a unifying mechanism for causation of the plaque and tangle pathologies in AD. Therefore, we sought to identify novel human aspartyl proteases. A whole genome scan in *C. elegans* identified seven open reading frames that adhere to the aspartyl protease profile that we had identified. These seven aspartyl proteases probably comprise the complete complement of such proteases in a simple, multicellular eukaryote. These include four closely related aspartyl proteases unique to *C. elegans* which probably arose by duplication of an ancestral gene. The other three candidate aspartyl proteases (T18H9.2, R12H7.2 and C11D2.2) were found to have homology to mammalian gene sequences.

### Example 2

### Identification of Novel Human Aspartyl Proteases Using Database Mining by Genome Bridging

### Materials and Methods:

### Computer-assisted analysis of EST databases, cDNA , and predicted polypeptide sequences:

Exhaustive homology searches of EST databases with the CEASP1, F21F8.3, F21F8.4, and F21F8.7 sequences failed to reveal any novel mammalian homologues. TBLASTN searches with R12H7.2 showed homology to cathepsin D, cathepsin E, pepsinogen A, pepsinogen C and renin, particularly around the DTG motif within the active site, but also failed to identify any additional novel mammalian aspartyl proteases. This indicates that the *C. elegans* genome probably contains only a single lysosomal aspartyl protease which in mammals is represented by a gene family that arose through duplication and consequent modification of an ancestral gene.

TBLASTN searches with T18H9.2, the remaining *C*. *elegans* sequence, identified several ESTs which assembled into a contig encoding a novel human aspartyl protease (Hu-ASP1). As is described above in Example 1, BLASTX search with the Hu-ASP1 contig against SWISS-PROT revealed that the active site motifs in the sequence aligned with the active sites of other aspartyl proteases. Exhaustive, repetitive rounds of BLASTN searches against LifeSeq, LifeSeqFL, and the public EST collections identified 102 EST from multiple cDNA libraries that assembled into a single contig. The 51 sequences in this contig found in public EST collections also have been assembled into a single contig (THC213329) by The Institute for Genome Research (TIGR). The TIGR annotation indicates that they failed to find any hits in the database for the contig. Note that the TIGR contig is the reverse complement of the LifeSeq contig that we assembled. BLASTN search of Hu-ASP1 against the rat and mouse EST sequences in ZooSeq revealed one homologous EST in each database (Incyte clone 700311523 and IMAGE clone 313341, GenBank accession number W10530, respectively).

TBLASTN searches with the assembled DNA sequence for Hu-ASP1 against both LifeSeqFL and the public EST databases identified a second, related human sequence (Hu-Asp2) represented by a single EST (2696295). Translation of this partial cDNA sequence reveals a single DTG motif which has homology to the active site motif of a bovine aspartyl protease, NM1.

BLAST searches, contig assemblies and multiple sequence alignments were performed using the bioinformatics tools provided with the LifeSeq, LifeSeqFL and LifeSeq Assembled databases from Incyte. Predicted protein motifs were identified using either the ProSite dictionary (Motifs in GCG 9) or the Pfam database.

### Full-length cDNA cloning of Hu-Asp1

The open reading frame of *C. elegans* gene T18H9.2CE was used to query Incyte LifeSeq and LifeSeq-FL databases and a single electronic assembly referred to as 1863920CE1 was detected. The 5' most cDNA clone in this contig, 1863920, was obtained from Incyte and completely sequenced on both strands. Translation of the open reading frame contained within clone 1863920 revealed the presence of the duplicated aspartyl protease active site motif (DTG/DSG) but the 5' end was incomplete. The remainder of the Hu-Asp1 coding sequence was determined by 5' Marathon RACE analysis using a human placenta Marathon ready cDNA template (Clontech). A 3'-antisense oligonucleotide primer specific for the 5' end of clone 1863920 was paired with the 5'-sense primer specific for the Marathon ready cDNA synthetic adaptor in the PCR. Specific PCR products were directly sequenced by cycle sequencing and the resulting sequence assembled with the sequence of clone 1863920 to yield the complete coding sequence of Hu-Asp-1 (SEQ ID No. 1).

Several interesting features are present in the primary amino acid sequence of Hu-Asp1 (Figure 1, SEQ ID No. 2). The sequence contains a signal peptide (residues 1-20 in SEQ ID No. 2), a pro-segment, and a catalytic domain containing two copies of the aspartyl protease active site motif (DTG/DSG). The spacing between the first and second active site motifs is about 200 residues which should correspond to the expected size of a single, eukaryotic aspartyl protease domain. More interestingly, the sequence contains a predicted transmembrane domain (residues 469-492 in SEQ ILL No.2) near its C-terminus which suggests that the protease is anchored in the membrane. This feature is not found in any other aspartyl protease.

### Cloning of a full-length Hu-Asp-2 cDNAs:

As is described above in Example 1, genome wide scan of the *Caenorhabditis elegans* database WormPep12 for putative aspartyl proteases and subsequent mining of human EST databases revealed a human ortholog to the *C. elegans* gene T18H9.2 referred to as Hu-Asp1. The assembled contig for Hu-Asp1 was used to query for human paralogs using the BLAST search tool in human EST databases and a single significant match (2696295CE1) with approximately 60% shared identity was found in the LifeSeq FL database. Similar queries of either gb105PubEST or the family of human databases available from TIGR did not identify similar EST clones. cDNA clone 2696295, identified by single pass sequence analysis from a human uterus cDNA library, was obtained from Incyte and completely sequence on both strands. This clone contained an incomplete 1266 bp open-reading frame that encoded a 422 amino acid polypeptide but lacked an initiator ATG on the 5' end. Inspection of the predicted sequence revealed the presence of the duplicated aspartyl protease active site motif DTG/DSG, separated by 194 amino acid residues. Subsequent queries of later releases of the LifeSeq EST database identified an additional ESTs, sequenced from a human astrocyte cDNA library (4386993), that appeared to contain additional 5' sequence relative to clone 2696295. Clone 4386993 was obtained from Incyte and completely sequenced on both strands. Comparative analysis of clone 4386993 and clone 2696295 confirmed that clone 4386993 extended the open-reading frame by 31 amino acid residues including two in-frame translation initiation codons. Despite the presence of the two in-frame ATGs, no in-frame stop codon was observed upstream of the ATG indicating that the 4386993 may not be full-length. Furthermore, alignment of the sequences of clones 2696295 and 4386993 revealed a 75 base pair insertion in clone 2696295 relative to clone 4386993 that results in the insertion of 25 additional amino acid residues in 2696295. The remainder of the Hu-Asp2 coding sequence was determined by 5' Marathon RACE analysis using a human

### Example 3

### Northern Blot Detection of HuAsp-1 Transcripts in Human Cell Lines

A variety of human cell lines was tested for their ability to produce Hu-Asp1 and Asp2 mRNA. Human embryonic kidney (HEK-293) cells, African green monkey (Cos-7) cells, Chinese hamster ovary (CHO) cells, HELA cells, and the neuroblastoma cell line IMR-32 were all obtained from the ATCC. Cells were cultured in DME containing 10% FCS except CHO cells which were maintained in α-MEM/10% FCS at 37°C in 5% CO₂ until they were near confluence. Washed monolayers of cells (3 X 10⁷) were lysed on the dishes and poly A⁺ RNA extracted using the Qiagen Oligotex Direct mRNA kit. Samples containing 2 µg of poly A⁺ RNA from each cell line were fractionated under denaturing conditions (glyoxal-treated), transferred to a solid nylon membrane support by capillary action, and transcripts visualized by hybridization with random-primed labeled (³²P) coding sequence probes derived from either Hu-Asp1 or Hu-Asp2. Radioactive signals were detected by exposure to X-ray film and by image analysis with a PhosphorImager.

The Hu-Asp1 DNA probe visualized a similar constellation of transcripts (2.6 kb and 3.5 kb) that were previously detected in human tissues. The relative abundance determined by quantification of the radioactive signal was Cos-7 > HEK 292 = HELA > IMR32.

### Example 4

### Modification of APP to increase Aβ processing for in vitro screening

Human cell lines that process Aβ peptide from APP provide a means to screen in cellular assays for inhibitors of β- and γ-secretase. Production and release of Aβ peptide into the culture supernatant is monitored by an enzyme-linked immunosorbent assay (EIA). Although expression of APP is widespread and both neural and non-neuronal cell lines process and release Aβ peptide, levels of endogenous APP processing are low and difficult to detect by EIA. Aβ processing can be increased by expressing in transformed cell lines mutations of APP that enhance Aβ processing. We made the serendipitous observation that addition of two lysine residues to the carboxyl terminus of APP695 increases Aβ processing still further. This allowed us to create a transformed cell line that releases Aβ peptide into the culture medium at the remarkable level of 20,000 pg/ml.

### Materials And Methods

### Materials:

Human embryonic kidney cell line 293 (HEK293 cells) were obtained internally. The vector pIRES-EGFP was purchased from Clontech. Oligonucleotides for mutation using the polymerase chain reaction (PCR) were purchased from Genosys. A plasmid containing human APP695 (SEQ ID No. 9 [nucleotide] and SEQ ID No. 10 [amino acid]) was obtained from Northwestern University Medical School. This was subcloned into pSK (Stratagene) at the *Not*I site creating the plasmid pAPP695.

### Mutagenesis protocol:

The Swedish mutation (K670N, M671L) was introduced into pAPP695 using the Stratagene Quick Change Mutagenesis Kit to create the plasmid pAPP695NL (SEQ ID No. 11 [nucleotide] and SEQ ID No. 12 [amino acid]). To introduce a di-lysine motif at the C-terminus of APP695, the forward primer #276 5' GACTGACCACTCGACCAGGTTC (SEQ ID No. 47) was used with the "patch" primer #274 5' CGAATTAAATTCCAGCACACTGGCTACTTCTTGTTCTGCATCTCAAAGAAC (SEQ ID No. 48) and the flanking primer #275 CGAATTAAATTCCAGCACACTGGCTA (SEQ ID No. 49) to modify the 3' end of the APP695 cDNA (SEQ ID No. 15 [nucleotide] and SEQ ID No. 16 [amino acid]). This also added a BstX1 restriction site that will be compatible with the BstX1 site in the multiple cloning site of pIRES-EGFP. PCR amplification was performed with a Clontech HF Advantage cDNA PCR kit using the polymerase mix and buffers supplied by the manufacturer. For "patch" PCR, the patch primer was used at 1/20th the molar concentration of the flanking primers. PCR amplification products were purified using a QIAquick PCR purification kit (Qiagen). After digestion with restriction enzymes, products were separated on 0.8% agarose gels and then excised DNA fragments were purified using a QIAquick gel extraction kit (Qiagen).

To reassemble a modified APP695-Sw cDNA, the 5' Not1-Bgl2 fragment of the APP695-Sw cDNA and the 3' Bgl2-BstX1 APP695 cDNA fragment obtained by PCR were ligated into pIRES-EGFP plasmid DNA opened at the Not1 and BstX1 sites. Ligations were performed for 5 minutes at room temperature using a Rapid DNA Ligation kit (Boehringer Mannheim) and transformed into Library Efficiency DH5a Competent Cells (GibcoBRL Life Technologies). Bacterial colonies were screened for inserts by PCR amplification using primers #276 and #275. Plasmid DNA was purified for mammalian cell transfection using a QIAprep Spin Miniprep kit (Qiagen). The construct obtained was designated pMG125.3 (APPSW-KK, SEQ ID No. 17 [nucleotide] and SEQ ID No. 18 [amino acid]).

### Mammalian Cell Transfection:

HEK293 cells for transfection were grown to 80% confluence in Dulbecco's modified Eagle's medium (DMEM) with 10% fetal bovine serum. Cotransfections were performed using LipofectAmine (Gibco-BRL) with 3 µg pMG125.3 DNA and 9 µg pcDNA3.1 DNA per 10 x 10⁶ cells. Three days posttransfection, cells were passaged into medium containing G418 at a concentration of 400 µg/ml. After three days growth in selective medium, cells were sorted by their fluorescence.

### Clonal Selection of 125.3 cells by FACS:

Cell samples were analyzed on an EPICS Elite ESP flow cytometer (Coulter, Hialeah, FL) equipped with a 488 nm excitation line supplied by an air-cooled argon laser. EGFP emission was measured through a 525 nm band-pass filter and fluorescence intensity was displayed on a 4-decade log scale after gating on viable cells as determined by forward and right angle light scatter. Single green cells were separated into each well of one 96 well plate containing growth medium without G418. After a four day recovery period, G418 was added to the medium to a final concentration of 400 µg/ml. After selection, 32% of the wells contained expanding clones. Wells with clones were expanded from the 96 well plate to a 24 well plate and then a 6 well plate with the fastest growing colonies chosen for expansion at each passage. The final cell line selected was the fastest growing of the final six passaged. This clone, designated 125.3, has been maintained in G418 at 400 ug/ml with passage every four days into fresh medium. No loss of Aβ production of EGFP fluorescence has been seen over 23 passages.

### Aβ EIA Analysis (Double Antibody Sandwich ELISA for hAβ 1-40/42):

Cell culture supernatants harvested 48 hours after transfection were analyzed in a standard Aβ EIA as follows. Human Aβ 1-40 or 1-42 was measured using monoclonal antibody (mAb) 6E10 (Senetek, St. Louis, MO) and biotinylated rabbit antiserum 162 or 164 (New York State Institute for Basic Research, Staten Island, NY) in a double antibody sandwich ELISA. The capture antibody 6E10 is specific to an epitope present on the N-terminal amino acid residues 1-16 of hAβ. The conjugated detecting antibodies 162 and 164 are specific for hAβ 1-40 and 1-42, respectively. Briefly, a Nunc Maxisorp 96 well immunoplate was coated with 100 µl/well of mAb 6E10 (5µg/ml) diluted in 0.1M carbonate-bicarbonate buffer, pH 9.6 and incubated at 4°C overnight. After washing the plate 3x with 0.01M DPBS (Modified Dulbecco's Phosphate Buffered Saline (0.008M sodium phosphate, 0.002M potassium phosphate, 0.14M sodium chloride, 0.01 M potassium chloride, pH 7.4) from Pierce, Rockford, II) containing 0.05% of Tween-20 (DPBST), the plate was blocked for 60 minutes with 200 µl of 10% normal sheep serum (Sigma) in 0.01M DPBS to avoid non-specific binding. Human Aβ 1-40 or 1-42 standards 100 µl/well (Bachem, Torrance, CA) diluted, from a 1mg/ml stock solution in DMSO, in culture medium was added after washing the plate, as well as 100 µl/well of sample, *e.g*., conditioned medium of transfected cells.

The plate was incubated for 2 hours at room temperature and 4°C overnight. The next day, after washing the plate, 100 µl/well biotinylated rabbit antiserum 162 1:400 or 164 1:50 diluted in DPBST + 0.5% BSA was added and incubated at room temperature for 1 hour, 15 minutes. Following washes, 100 µl/well neutravidin-horseradish peroxidase (Pierce, Rockford, II) diluted 1:10,000 in DPBST was applied and incubated for 1 hour at room temperature. After the last washes 100 µl/well of o-phenylnediamine dihydrochloride (Sigma Chemicals, St. Louis, MO) in 50mM citric acid/100mM sodium phosphate buffer (Sigma Chemicals, St. Louis, MO), pH 5.0, was added as substrate and the color development was monitored at 450nm in a kinetic microplate reader for 20 minutes using Soft max Pro software. All standards and samples were run in triplicates. The samples with absorbance values falling within the standard curve were extrapolated from the standard curves using Soft max Pro software and expressed in pg/ml culture medium.

### Results:

Addition of two lysine residues to the carboxyl terminus of APP695 greatly increases Aβ processing in HEK293 cells as shown by transient expression (Table 1). Addition of the di-lysine motif to APP695 increases Aβ processing to that seen with the APP695 containing the Swedish mutation. Combining the di-lysine motif with the Swedish mutation further increases processing by an additional 2.8 fold.

Cotransformation of HEK293 cells with pMG125.3 and pcDNA3.1 allowed dual selection of transformed cells for G418 resistance and high level expression of EGFP. After clonal selection by FACS, the cell line obtained, produces a remarkable 20,000 pg Aβ peptide per ml of culture medium after growth for 36 hours in 24 well plates. Production of Aβ peptide under various growth conditions is summarized in Table 2.

**TABLE 1**

| Release of Aβ peptide into the culture medium 48 hours after transient transfection of HEK293 cells with the indicated vectors containing wildtype or modified APP. Values tabulated are mean + SD and P-value for pairwise comparison using Student's t-test assuming unequal variances. | | | |
|---|---|---|---|
| APP Construct | Aβ 1-40 peptide (pg/ml) | Fold Increase | P-value |
| pIRES-EGFP vector | 147 + 28 | 1.0 | |
| wt APP695 (142.3) | 194 + 15 | 1.3 | 0.051 |
| wt APP695-KK (124.1) | 424 + 34 | 2.8 | 3 x 10-5 |
| APP695-Sw (143.3) | 457 + 65 | 3.1 | 2 x 10-3 |
| APP695-SwKK (125.3) | 1308 + 98 | 8.9 | 3 x 10-4 |

**TABLE 2**

| Release of Aβ peptide from HEK125.3 cells under various growth conditions. | | | | |
|---|---|---|---|---|
| Type of Culture Plate | Volume of Medium | Duration of Culture | Aβ 1-40 (pg/ml) | Aβ 1-42 (pg/ml) |
| 24 well plate | 400 ul | 36 hr | 28,036 | 1,439 |

### Example 5

### Demonstration that Asp1 processes APP at the α-secretase site

Increased expression of an α-secretase candidate gene in human cells would be expected to increase basal release of sAPPα and to decrease release of Aβ peptides. This the effect was observed when full length human Asp1 is co-expressed with APP in HEK293 cells. The experiment utilized the APP 695 amino acid isoform which had been modified by the addition of a pair of lysine residues to the C-terminus (APP-KK). The C-terminal di-lysine motif increases the intracellular half-life of glycosylated APP and consequently the production of both sAPPα and Aβ. As shown in Table 3, cotransfection of HEK293 cells with APP-KK with Asp1 increased the production of sAPPα by 3.5 fold (p<0.001) and decreased the production of Aβ40 by 2.8 fold. Thus, Asp1 acts directly or indirectly to facilitate constitutive α-secretase cleavage and this effect is competitive with the amyloidogenic processing of APP to Aβ peptides. This implies that mutations or genetic polymorphisms in Asp1 may affect Aβ production by affecting the balance between the competing pathways for constitutive α-secretase cleavage and Aβ peptide production.

**Table 3.**

| **Asp1 stimulates basal release of sAPPα from HEK293 cells after cotransfection with APP-KK.** | | | | |
|---|---|---|---|---|
| Transfection | sAPPα µg/ml | Fold Increase | Aβ40 pg/ml | Fold Decrease |
| Asp 1 | 3.5 + 1.1 | +3.5 | 113 + 7 | -2.8 |
| pcDNA | 1.0 + 0.2 | | 321 + 18 | |

Specific methods used were as follows. The full length Asp1 cDNA was cloned into the vector pcDNA3. 1/hygro+(Invitrogen) for transfection studies as previously described (Yan et al., (1999) Nature 402: 533-537). The APP-KK cDNA was cloned into the vector pIRES (Clontech) also as previously described. HEK293 cells were transfected with expression constructs using the Lipofectamine Plus reagent from GibcoBRL. Cells were seeded in 24 well tissue culture plates to a density of 70-80% confluence. Four wells per plate were transfected with 2 µg DNA (3:1, APP:Asp1 or empty pcDNA3.1./hygro+ vector), 8µl Plus reagent, and 4µl Lipofectamine in OptiMEM. OptiMEM was added to a total volume of 1 ml, distributed 200 µl per well and incubated 3 hours. Care was taken to hold constant the ratios of the two plasmids used for cotransfection as well as the total amount of DNA used in the transfection. The transfection media was replaced with DMEM supplemented with 10% FBS and NaPyruvate, with antibiotic/antimycotic and the cells were incubated under normal conditions (37°, 5% CO₂) for 48 hours. The conditioned media were removed to polypropylene tubes and stored at -80°C until assayed for the content of sAPPα or Aβ40/Aβ42 by enzyme-linked immunosorbent assay (EIA) as described above in Example 4. The Aβ EIA followed the protocol of Pirttila et al. (Neuro. Lett. (1999) 249: 21-4) using the 6E10 monoclonal antibody (Senetek) as a capture antibody and biotinylated rabbit antiserum 162 or 165 (New York State Institute for Basic Research, Staten Island, NY) for detection of Aβ40 and Aβ42, respectively. The 6E10 antibody recognizes residues 1-16 of the Aβ peptide. The sAPPα EIA used LN27 antibody as a capture antibody and biotinylated 6E10 for detection as described previously (Yan *et al.,* (1999) *supra.).* The LN27 antibody recognized the first 20 amino acids of the human APP peptide.

Increased α-secretase activity and concomitant reduction of Aβ production *in vivo* represents an effect that may be desirable for the prevention, treatment (*e.g*., to show the progression of), or cure of Azheimer's disease. Thus, the activities demonstrated in this example provide an indication that modulators of Asp1 activity, that achieve the same effects *in vivo,* will have utility for Alzheimer's disease therapy. Screening methods for such modulators are contemplated as an aspect of the invention.

### Example 6

### Expression of Pre-pro-Hu-Asp1 and Derivatives in Insect Cells

### Expression of hu-Asp-1TM(His)⁶ by baculovirus infection.

The coding sequence of pre-pro-Hu-Asp1 was engineered for production as a soluble, secreted form by insect cells. PCR primers were designed to (1) delete the predicted transmembrane domain and cytoplasmic tail of Asp1 and (2) to introduce a Kozak consensus sequence for efficient translational initiation. The primers sequences were are follows: sense CGCTTTAAGCTTGCCACCATGGGCGCA CTGGCCCGGGCG (SEQ ID NO: 74) and antisense CGCTTTCTCGAGCTAA. TGGTGATGGTGATGGTGCCACAAAATGGGCTCGCTCAAAGA (SEQ ID NO: 75) which replaced the deleted C-terminal transmembrane and cytoplasmic domains with a hexahistidine purification tag.

PCR reactions were carried out with 100 ng of full length Asp1 pcDNA 3.1 hygro+construct, 200 M NTPs, 300 nM of each primer, 1x reaction buffer containing 2 mM MgSO₄, and 5 units of Pwo I DNA polymerase (Roche Biochemicals). The reactions were cycled under the following conditions: 94°C for 5 minutes followed by 15 cycles of 94°C for 30 seconds and 72°C for 30 seconds, and then a final extension reaction at 72°C for 10 minutes. The predicted amino acid sequence of this PCR generated derivative (denoted as Asp-1ΔTM(His)₆) is set out as SEQ ID NO: 66.

The reaction product was digested to completion with HindIII-XhoI and ligated into the expression vector pIB (Invitrogen) to yield the pIB/Asp-1ΔTM(His)₆ construct. Creation of recombinant baculovirus and infection of sf9 insect cells was performed using standard methods known in the art. Sf9 cells were transfected with either the pIB vector alone or the pIB/Asp-1ΔTM(His)₆ construct utilizing Insectin Plus reagent (Invitrogen) according to the manufacturer's instructions. After the transfection, the cells were cultured in High Five serum-free media (Invitrogen) for 3 days. Subsequently, the conditioned medium was harvested and subjected to Western blot analysis. This analysis revealed specific expression and secretion of immunoreactive Asp-1ΔTM(His)₆ polypeptide into the extracellular medium. The secreted proteins were detected on the Western blot with either the India probe (Pierce Chemicals) specific for the hexahistidine sequence tag or using a rabbit polyclonal antiserum. The polyclonal antisera (denoted as UP-199) was generated by injecting rabbits with recombinant Asp-1ΔTM(His)₆ (SEQ ID NO: 66). This recombinant peptide was prepared by heterologous expression in *E. coli.* The UP-199 antibody recognizes the processed form of Asp-1ΔTM.

Direct analysis with the polyclonal antiserum (UP-199) revealed an immunoreactive band of the expected molecular weight (50 kDa) only in pIB/Asp-1ΔTM(His)₆ transfected cells. This signal was significantly enhanced in concentrated conditioned medium. A similar pattern was obtained using the India probe. No signal was detected in conditioned medium derived from mock-transfected cells using either UP-199 antisera or the India probe.

Based on this result, transient and stable transfections of the pIB/Asp-1ΔTM(His)₆ construct in sf9 insect cells were carried out as described above. Four days post transient transfection, the culture medium was collected to provide material for further characterization. In parallel, sf9 cells were stably transfected with the pIB/Asp-1ΔTM(His)₆ construct and cultured in High Five serum-free medium (Invitrogen) supplemented with 50 µg/ml blasticidin for approximately 2 weeks. After blasticidin selection, the resistant pool of cells was expanded to provide a stable source of conditioned medium for Asp-1ΔTM(His)₆ purification.

### Purification of recombinant Asp-1ΔTM(His)₆

Conditioned media, from either transient or stably transfected sf9 cells, were concentrated approximately 10-fold using a stirred cell concentrator equipped with a 30,000 MWCO membrane (Spectrum Medical Industries). This concentrate was then subjected to ammonium sulfate precipitation to further concentrate the sample and provide partial purification. Material precipitating between 0-40% saturation was discarded and the resulting supernatant was brought to 80% saturation. Western blot analysis of the various ammonium sulfate precipitated fractions revealed that the majority of the immunoreactive material was contained within the 40-80% ammonium sulfate pellet. As a result, this material was subjected to further purification.

The 40-80% ammonium sulfate pellet was redisolved in approximately 1/20 the original volume of Ni+-NTA loading buffer (25 mM Tris-HCl (pH 8.5)/0.5 M NaCl/10 mM imidazole). Subsequently, the sample was applied to a Ni+-NTA column previously equilibrated in Ni+-NTA buffer. Following sample application, the column was washed with starting buffer (25 mM Tris-HCl (ph 8.5)/ 0.5 M NaCl/20 mM imidazole) until the A^{280nm} of the column effluent returned to zero. After washing, the bound recombinant protein was eluted off the column with a linear gradient ofNi+-NTA buffer containing increasing concentrations (10 mM, 50 mM, 100 mM, 250 mM, and 500 mM) of imidazole. The elution profile was monitored by Western blot analysis using the UP-199 antiserum. Immunoreactive Asp-1ΔTM(His)₆ was detected in the column load and eluted at 50 mM imidazole. NuPAGE gel analysis of the 50 mM imidazole fraction demonstrated a purity of Asp-1ΔTM(His)₆ of approximately 50%, therefore further purification was required.

The positive fractions, eluted off the Ni+-NTA column, were then pooled (denoted as post-IMAC pool), concentrated using a YM30 membrane (Amicron), and dialyzed with 25 mM Tris-HCl (pH 8.0). The dialyzed post-IMAC pool was fractionated by MonoQ anion exchange chromatography (Amersham-Pharmacia Biotech) gradient elution containing increasing concentrations (0 -0.5 M) of NaCI (Buffer A: 25 mM Tris-HCI (pH 8.0) and Buffer B: 25 mM Tris-HCI (pH 8.0)/ 0.5 M NaCl). The elution profile was determined by Western blot analysis which indicated immunoreactive fractions as those displaying immunoreactivity with the UP-199 antisera. NuPAGE gel analysis with silver staining demonstrated that the material prepared in this manner was >90% pure. The immunoactive fractions eluted off the MonoQ anion exchange column were pooled, dialyzed with 25 mM HEPES-Na+ (pH 8.0), and stored at 4°C until further analysis.

### Acid-activation of recombinant Asp-1 TM(His)₆

Recombinant Asp-1ΔTM(His)₆ migrated with an apparent molecular weight of 50 kD. Direct N-terminal sequence analysis carried out by automated Edman degradation for 20 cycles revealed a unique sequence beginning at Glu³ (SEQ NO: 67), confirming the identity of the recombinant protein. Computer assisted prediction of the signal peptidase cleavage site indicated that the pro-form should initiate at Ala¹, suggesting either an unusual processing site by the signal peptidase during secretion or an additional processing step that removes an additional two amino acid residues.

To investigate the mechanism of pro-Asp-1ΔTM(His)₆ activation, aliquots of the purified protein were incubated in various acidic environments with pH values ranging from 3.0-8.0 at 37°C for 2 hours. Subsequently; the recombinant proteins were analyzed by Western blot. A faster migrating polypeptide species was detected after incubation at pH values of 4.0, 4.5 and 5.0. The polypeptide migration was unaltered after incubation in environments which were either more acidic (pH 3.0 and 3.5) or more basic (pH 6.0, 7.0, and 8.0). Sequence analysis of this faster migrating species revealed that it initiated exclusively at Ala⁴³, consistent with removal of a 42 amino acid residue segment of the pro-peptide that was induced by treatment of the pro-enzyme at pH 4.5. The predicted amino acid sequence of the acid processed form of Asp-1ΔTM(His)₆ is set out as SEQ ID NO: 68.

To purify the acid-activated form of Asp-1ΔTM(His)₆, the Asp-1ΔTM(His)₆ post-IMAC pool (generated as described above) was dialyzed to pH 4.5 and then subjected to affinity chromatography on either pepstatin A agarose or sulfolink-PHA-292593E. Following sample application, the column was washed with 25 mM NaOAc (pH 4.5) and eluted with 50 mM Na-BO₃ (pH 9.5). The positive fractions eluted off the columns were dialyzed with 25 mM Hepes-Na (pH 7.5) overnight at 4°C which resulted in quantitative conversion of the pro-enzyme to the acid-processed form (SEQ ID NO: 68) described above. Western blot analysis of the elution profile revealed quantitative retention of immunoreactive Asp-1ΔTM(His)₆ on both affinity resins as evidenced by the lack of Asp-1ΔTM(His)₆ in the unbound fraction as detected by UP-199 immunoreactivity on a Western blot. Step elution 50 mM NaBO₃ at pH 9.5 resulted in elution of immunoreactive Asp-1 TM(His)₆, with variable recovery.

Asp1 undergoes efficient processing by the signal peptidase and additional processing to remove two additional amino acid residues from the N-terminus. Further processing of the pro-form of Asp1 was not detected in neutral pH.

In addition to providing valuable information about Asp1 activity, the discovery of a site of apparent autocatalytic processing of Asp1 provides an indication of a peptide sequences (surrounding Ala⁴³) that could be useful for performing screening assays to identify modulators of Asp1 activity. This idea is explored in greater detail in Example 7.

### Example 7

### Development of an enzymatic assay for Asp-1ΔTM(His)₆

The relationship between Asp1 and APP processing was explored by determining if APP α-secretase, APP β-secretase, or APP γ-secretase peptide substrates were cleaved by recombinant Asp-1ΔTM(His)₆. These peptide substrates included the α-secretase specific substrates Aβ₁₀₋₂₀ and Aβ₁₂₋₂₈, the β-secretase specific substrates PHA-95812E (SEVKMDAEFR; SEQ ID NO: 64) and PHA-247574E (SEVNLDAEFR; SEQ ID NO: 63), and γ-secretase specific substrate PHA-179111E (RRGGVVIATVIVGER; SEQ ID NO: 76). Each reaction consisted of incubating a peptide substrate (100 nM) with recombinant Asp-1ΔTM(His)₆ for 15 hours at pH 4.5 at 37°C. Reaction products were quantified by RP-HPLC at A^{214nm}. The elution profiles for Asp-1ΔTM(His)₆ were compared to those obtained from parallel Asp1 experiments. The identity of the cleavage products was determined by MADLI-TOF mass spectrometry. Table 4 summarizes the Asp1 substrates and indicates the cleavage site.

**Table 4.**

| **Substrate Preferences of Asp-1ΔTM** | | | | | | | | | | |
|---|---|---|---|---|---|---|---|---|---|---|
| P4 | P3 | P2 | P1 | | P1' | P2' | P3' | P4' | | SEQ ID NO: |
| G | L | A | L | ↓ | A | L | E | P | Self Activation | 69 |
| E | V | K | M | ↓ | D | A | E | F | β-Secretase, WT | 70 |
| E | V | N | L | ↓ | D | A | E | F | β-Secretase, Sw | 71 |
| L | V | F | F | ↓ | A | E | D | V | Aβ₁₂₋₂₈ (α-Secretase) | 72 |
| K | L | V | F | ↓ | F | A | E | D | Aβ₁₂₋₂₈ (α-Secretase) | 73 |

The peptides in Table 4 are described using the nomenclature by Schechter and Berger (Biochem. Biophys. Res. Commun. 27:157(1967) and Biochem. Biophys. Res. Commun. 32:898 (1968)), in which the amino acid residues in the peptide substrate that undergo the cleavage are defined as P₁ ... Pₙ toward the N-terminus and P₁' ... Pₙ' toward the C-terminus. Therefore, the scissile bond is between the P₁ and the P₁' residue of the peptide subunits and is denoted herein throughout with a hyphen between the P₁ and the P₁'.

Digestion of the α-secretase substrate (A₁₂₋₂₈) revealed two Asp1 cleavage sites. The major product was cleaved at Phe²⁰,Ala²¹ and the minor product was cleaved at Phe¹⁹Phe²⁰ (referring to the numbering convention in the APP Aβ) peptide. Analysis of the cleavage products obtained from the β-secretase peptide substrates revealed that both the wild-type (PHA-95812E) and the Swedish mutation (PHA-247574E) substrates were hydrolyzed exclusively at the β-secretase site. Also, the relative rates of Asp-1-dependent hydrolysis of the β-secretase peptide substrate containing the Swedish mutation was cleaved at least 10-times faster than the corresponding wild-type peptide. Conversion of the γ-secretase peptide substrate was not detected under these reaction conditions.

Measurement of the cleavage of the α-secretase and β-secretase substrates can also be carried out with substrates comprising detectable labels such as radioactive, enzymati , chemiluminescent or flourescent labels. For example, the peptide substrates could comprise internally quenched labels that result in increased detectability after cleavage of the peptide substrates due to separation of the labels upon cleavage. The peptide substrates can be modified to have attached a paired fluorprobe and quencher such as 7-amino-4-methyl courarin and dinitrophenol, respectively.

This example illustrates the α-secretase and β-secretase activity exhibited by Asp-1, confirming the APP processing activity of Asp1 shown in Example 5. The substrates described herein may be used in combination with recombinant Asp1 to measure Asp1-proteolytic activity at the α-secretase and β-secretase processing sites. These substrates are useful in screening assays for identification of modulators of Asp1 proteolytic activity.

In particular, production of Aβ species through the processing of APP at β-and γ-secretase sites may play a central role in Alzheimer's disease pathogenesis, and processing at the α-secretase site may have a protective role and may prevent Aβ production. Thus, a therapeutic and/or prophylactic indication exists for molecules that can increase Asp1 α-secretase activity and/or decrease Asp1 β-secretase activity *in vivo.*

## Claims

1. A method for assaying hu-Asp1 α-secretase activity, comprising the steps of:
(a) contacting a hu-Asp1 polypeptide which comprises an amino acid sequence at least 95% identical to a fragment, including the aspartyl protease active site tripeptides DTG and DSG, of the amino acid sequence of SEQ ID NO:2, wherein the polypeptide and the fragment retain α-secretase activity, with an amyloid precursor protein (APP) substrate which contains an α-secretase cleavage site; and
(b) measuring cleavage of the APP substrate at the hu-Aspl α-secretase cleavage site.

2. A method according to claim 1, wherein the hu-Aspl polypeptide is produced in a cell transformed or transfected with a polynucleotide encoding a hu-Asp1 polypeptide as defined in claim 1.

3. A method according to claim 2, wherein the hu-Asp1 polypeptide is purified and isolated from said cell.

4. A method according to claim 1, wherein the contacting step comprises growing a cell transfected or transformed with a polynucleotide encoding a hu-Asp1 polypeptide as defined in claim 1, under conditions in which the cell expresses the hu-Asp1 polypeptide in the presence of the APP substrate.

5. A method according to claim 4, wherein the cell expresses an APP substrate containing an α-secretase cleavage site, and wherein the growing is under conditions in which the cell expresses the hu-Asp1 polypeptide and the APP substrate.

6. A method according to any preceding claim, wherein the hu-Aspl polypeptide is encoded by a polynucleotide that hydridizes under stringent conditions to the complement of SEQ ID NO:1, wherein the stringent conditions are overnight incubation at about 42°C for about 2.5 hours in 6 x SSC/0.1% SDS, followed by washing of the filters four times for 15 minutes in 1.0 x SSC at 65°C, 0.1% SDS.

7. A method according to any of claims 1 to 6, wherein the hu-Asp1 polypeptide lacks a transmembrane domain.

8. A method according to claim 7, wherein the hu-Asp1 polypeptide lacks amino acids 469-492 of SEQ ID NO:2.

9. A method according to claim 8, wherein the hu-Asp1 polypeptide further lacks the cytoplasmic domain of amino acids 493-518 of SEQ ID NO:2.

10. A method according to any of claims 1 to 9, wherein the hu-Asp1 polypeptide lacks amino acids 1-62 of SEQ ID NO:2.

11. A method according to any of claims 1 to 10, wherein the cleavage site comprises the amino acid sequence LVFFAEDF or KLVFFAED.

12. A method according to any of claims 1 to 11, wherein the APP substrate comprises a human APP isoform and further comprises a carboxy-terminal di-lysine.

13. A method according to claim 12, wherein the human APP isoform has an amino acid sequence selected from SEQ ID NO:10, SEQ ID NO:12, SEQ ID NO:14, SEQ ID NO:55 and SEQ ID NO:57.

14. A method according to any of claims 11 to 13, wherein the APP substrate comprises a detectable label.

15. A method according to claim 14, wherein the detectable label is selected from radioactive labels, enzymatic labels, chemiluminescent labels and fluorescent labels.

16. A method according to any of claims 1 to 15, wherein the APP substrate comprises a human APP isoform, and the measuring step comprises measuring the production of amyloid alpha peptide.

## Patentansprüche

1. Verfahren zur Untersuchung von hu-Asp1-α-Sekretase-Aktivität, umfassend die Schritte:
(a) Inkontaktbringen eines hu-Asp1-Polypeptids, welches eine Aminosäuresequenz umfasst, die zu mindestens 95% identisch ist zu einem Fragment, beinhaltend die Tripeptide DTG und DSG der aktiven Stelle von Aspartyl-Protease, mit der Aminosäuresequenz von SEQ ID NO:2, wobei das Polypeptid und das Fragment α-Sekretase-Aktivität beibehalten, mit einem Amyloid-Vorläuferprotein (amyloid precursor protein; APP)-Substrat, das eine α-Sekretase-Schnittstelle enthält; und
(b) Messen der Spaltung des APP-Substrats an der hu-Asp1-α-Sekretase-Schnittstelle.

2. Verfahren nach Anspruch 1, wobei das hu-Asp1-Polypeptid in einer Zelle produziert wird, die mit einem Polynukleotid transformiert oder transfiziert ist, welches ein hu-Aspl-Polypeptid gemäß Definition in Anspruch 1 codiert.

3. Verfahren nach Anspruch 2, wobei das hu-Asp1-Polypeptid aus der Zelle gereinigt und isoliert wird.

4. Verfahren nach Anspruch 1, wobei der Schritt des Inkontaktbringens das Vermehren einer Zelle, die mit einem Polynukleotid transfiziert oder transformiert ist, welches ein hu-Asp1-Polypeptid gemäß Definition in Anspruch 1 codiert, unter Bedingungen umfasst, bei denen die Zelle das hu-Asp1-Polypeptid in Gegenwart des APP-Substrats exprimiert.

5. Verfahren nach Anspruch 4, bei dem die Zelle ein APP-Substrat exprimiert, das eine α-Sekretase-Schnittstelle enthält, und bei dem das Vermehren unter Bedingungen erfolgt, bei denen die Zelle das hu-Asp1-Polypeptid und das APP-Substrat exprimiert.

6. Verfahren nach einem der vorigen Ansprüche, bei dem das hu-Asp1-Polypeptid durch ein Polynukleotid codiert wird, das unter stringenten Bedingungen an das Komplement von SEQ ID NO:1 hybridisiert, wobei die stringenten Bedingungen in Übemacht-Inkubation bei etwa 42°C für etwa 2,5 Stunden in 6 x SSC/0,1% SDS, gefolgt von viermaligem Waschen der Filter für 15 Minuten in 1,0 x SSC bei 65°C, 0,1% SDS bestehen.

7. Verfahren nach einem der Ansprüche 1 bis 6, wobei dem hu-Asp1-Polypeptid eine Transmembran-Domäne fehlt.

8. Verfahren nach Anspruch 7, wobei dem hu-Asp1-Polypeptid die Aminosäuren 469-492 von SEQ ID NO:2 fehlen.

9. Verfahren nach Anspruch 8, wobei dem hu-Asp1-Polypeptid weiterhin die zytoplasmatische Domäne der Aminosäuren 493-518 von SEQ ID NO:2 fehlt.

10. Verfahren nach einem der Ansprüche 1 bis 9, wobei dem hu-Asp1-Polypeptid die Aminosäuren 1-62 von SEQ ID NO:2 fehlen.

11. Verfahren nach einem der Ansprüche 1 bis 10, wobei die Schnittstelle die Aminosäuresequenz LVFFAEDF oder KLVFFAED umfasst.

12. Verfahren nach einem der Ansprüche 1 bis 11, wobei das APP-Substrat eine humane APP-Isoform beinhaltet und weiterhin ein carboxyterminales Di-Lysin umfasst.

13. Verfahren nach Anspruch 12, wobei die humane APP-Isoform eine Aminosäuresequenz besitzt, die ausgewählt ist aus SEQ ID NO:10, SEQ ID NO:12, SEQ ID NO:14, SEQ ID NO:55 und SEQ ID NO:57.

14. Verfahren nach einem der Ansprüche 11 bis 13, wobei das APP-Substrat eine detektierbare Markierung umfasst.

15. Verfahren nach Anspruch 14, wobei die detektierbare Markierung ausgewählt ist aus radioaktiven Markierungen, enzymatischen Markierungen, chemilumineszenten Markierungen und fluoreszenten Markierungen.

16. Verfahren nach einem der Ansprüche 1 bis 15, wobei das APP-Substrat eine humane APP-Isoform beinhaltet, und wobei der Messschritt das Messen der Produktion von Amyloid-alpha-Peptid umfasst.

## Revendications

1. Procédé de dosage de l'activité α-sécrétase de hu-Asp1 comprenant les étapes consistant à :
(a) mettre en contact un polypeptide hu-Asp1 qui comprend une séquence d'acides aminés au moins identique à 95 % avec un fragment, y compris les tripeptides DTG et DSG du site actif des aspartyl-protéases, de la séquence d'acides aminés de SEQ ID NO : 2, où le polypeptide et le fragment conservent une activité α-sécrétase, avec le substrat de la protéine précurseur de l'amyloïde (APP) qui contient un site de clivage d'α-sécrétase ; et
(b) mesurer le clivage du substrat APP au niveau du site de clivage de l'α-sécrétase de hu-Aspl.

2. Procédé selon la revendication 1, dans lequel le polypeptide hu-Asp1 est produit dans une cellule transformée ou transfectée avec un polynucléotide codant pour un polypeptide hu-Asp1 tel que défini dans la revendication 1.

3. Procédé selon la revendication 2, dans lequel le polypeptide hu-Asp1 est purifié et isolé à partir de ladite cellule.

4. Procédé selon la revendication 1, dans lequel l'étape de mise en contact comprend la culture d'une cellule transfectée ou transformée avec un polynucléotide codant pour un polypeptide hu-Asp1 tel que défini dans la revendication 1 dans des conditions dans lesquelles la cellule exprime le polypeptide hu-Asp1 en présence du substrat APP.

5. Procédé selon la revendication 4, dans lequel la cellule exprime un substrat APP contenant un site de clivage d'α-sécrétase, et dans lequel la culture est réalisée dans des conditions dans lesquelles la cellule exprime le polypeptide hu-Asp1 et le substrat APP.

6. Procédé selon l'une quelconque des revendications précédentes, dans lequel le polypeptide hu-Asp1 est codé par un polynucléotide qui s'hybride dans des conditions stringentes au complémentaire de SEQ ID NO : 1, où les conditions stringentes sont une incubation pendant une nuit à environ 42°C pendant environ 2,5 heures dans du SSC 6X/0,1 % de SDS, suivie d'un lavage des filtres quatre fois pendant 15 minutes dans du SSC 1,0X à 65°C, 0,1 % de SDS.

7. Procédé selon l'une quelconque des revendications 1 à 6, dans lequel le polypeptide hu-Aspl manque d'un domaine transmembranaire.

8. Procédé selon la revendication 7, dans lequel le polypeptide hu-Aspl manque des acides aminés 469 à 492 de SEQ ID NO : 2.

9. Procédé selon la revendication 8, dans lequel le polypeptide hu-Asp1 manque en outre du domaine cytoplasmique des acides aminés 493 à 518 de SEQ ID NO : 2.

10. Procédé selon l'une quelconque des revendications 1 à 9, dans lequel le polypeptide hu-Asp1 manque des acides aminés 1 à 62 de SEQ ID NO : 2.

11. Procédé selon l'une quelconque des revendications 1 à 10, dans lequel le site de clivage comprend la séquence d'acides aminés LVFFAEDF et KLVFFAED.

12. Procédé selon l'une quelconque des revendications 1 à 11, dans lequel le substrat APP comprend une isoforme de l'APP humaine et comprend en outre une di-lysine carboxy-terminale.

13. Procédé selon la revendication 12, dans lequel l'isoforme de l'APP humaine a une séquence d'acides aminés choisie parmi SEQ ID NO : 10, SEQ ID NO : 12, SEQ ID NO : 14, SEQ ID NO : 55 et SEQ ID NO : 57.

14. Procédé selon l'une quelconque des revendications 11 à 13, dans lequel le substrat APP comprend un marqueur détectable.

15. Procédé selon la revendication 14, dans lequel le marqueur détectable est choisi parmi des marqueurs radioactifs, des marqueurs enzymatiques, des marqueurs chimioluminescents et des marqueurs fluorescents.

16. Procédé selon l'une quelconque des revendications 1 à 15, dans lequel le substrat APP comprend une isoforme de l'APP humaine, et l'étape de mesure comprend la mesure de la production du peptide alpha-amyloïde.


### SEQUENCE LISTING

<110> Pharmacia & Upjohn
<120> ALZHEIMER'S DISEASE SECRETASE, APP SUBSTRATES THEREFOR, AND USES THEREFOR
<130> 28341/6280.1
<140>
   <141>
<150> 60/169,232
   <151> 1999-12-06
<150> 09/416,901
   <151> 1999-10-13
<150> 60/155,493
   <151> 1999-09-23
<150> 09/404,133
   <151> 1999-09-23
<150> PCT/US99/20881
   <151> 1999-09-23
<150> 60/101,594
   <151> 1998-09-24
<160> 76
<170> PatentIn Ver. 2.0
<210> 1
   <211> 1804
   <212> DNA
   <213> Homo sapiens
<400> 1
   atgggcgcac tggcccgggc gctgctgctg cctctgctgg cccagtggct cctgcgcgcc 60
   gccccggagc tggcccccgc gcccttcacg ctgcccctcc gggtggccgc ggccacgaac 120
   cgcgtagttg cgcccacccc gggacccggg acccctgccg agcgccacgc cgacggcttg 180
   gcgctcgccc tggagcctgc cctggcgtcc cccgcgggcg ccgccaactt cttggccatg 240
   gtagacaacc tgcaggggga ctctggccgc ggctactacc tggagatgct gatcgggacc 300
   cccccgcaga agctacagat tctcgttgac actggaagca gtaactttgc cgtggcagga 360
   accccgcact cctacataga cacgtacttt gacacagaga ggtctagcac ataccgctcc 420
   aagggctttg acgtcacagt gaagtacaca caaggaagct ggacgggctt cgttggggaa 480
   gacctcgtca ccatccccaa aggcttcaat acttcttttc ttgtcaacat tgccactatt 540
   tttgaatcag agaatttctt tttgcctggg attaaatgga atggaatact tggcctagct 600
   tatgccacac ttgccaagcc atcaagttct ctggagacct tcttcgactc cctggtgaca 660
   caagcaaaca tccccaacgt tttctccatg cagatgtgtg gagccggctt gcccgttgct 720
   ggatctggga ccaacggagg tagtcttgtc ttgggtggaa ttgaaccaag tttgtataaa 780
   ggagacatct ggtatacccc tattaaggaa gagtggtact accagataga aattctgaaa 840
   ttggaaattg gaggccaaag ccttaatctg gactgcagag agtataacgc agacaaggcc 900
   atcgtggaca gtggcaccac gctgctgcgc ctgccccaga aggtgtttga tgcggtggtg 960
   gaagctgtgg cccgcgcatc tctgattcca gaattctctg atggtttctg gactgggtcc 1020
   cagctggcgt gctggacgaa ttcggaaaca ccttggtctt acttccctaa aatctccatc 1080
   tacctgagag atgagaactc cagcaggtca ttccgtatca caatcctgcc tcagctttac 1140
   attcagccca tgatgggggc cggcctgaat tatgaatgtt accgattcgg catttcccca 1200
   tccacaaatg cgctggtgat cggtgccacg gtgatggagg gcttctacgt catcttcgac 1260
   agagcccaga agagggtggg cttcgcagcg agcccctgtg cagaaattgc aggtgctgca 1320
   gtgtctgaaa tttccgggcc tttctcaaca gaggatgtag ccagcaactg tgtccccgct 1380
   cagtctttga gcgagcccat tttgtggatt gtgtcctatg cgctcatgag cgtctgtgga 1440
   gccatcctcc ttgtcttaat cgtcctgctg ctgctgccgt tccggtgtca gcgtcgcccc 1500
   cgtgaccctg aggtcgtcaa tgatgagtcc tctctggtca gacatcgctg gaaatgaata 1560
   gccaggcctg acctcaagca accatgaact cagctattaa gaaaatcaca tttccagggc 1620
   agcagccggg atcgatggtg gcgctttctc ctgtgcccac ccgtcttcaa tctctgttct 1680
   gctcccagat gccttctaga ttcactgtct tttgattctt gattttcaag ctttcaaatc 1740
   ctccctactt ccaagaaaaa taattaaaaa aaaaacttca ttctaaacca aaaaaaaaaa 1800
   aaaa                                                              1804
<210> 2
   <211> 518
   <212> PRT
   <213> Homo sapiens
<400> 2
   Met Gly Ala Leu Ala Arg Ala Leu Leu Leu Pro Leu Leu Ala Gln Trp
    1               5                  10                  15
   Leu Leu Arg Ala Ala Pro Glu Leu Ala Pro Ala Pro Phe Thr Leu Pro
               20                  25                  30
   Leu Arg Val Ala Ala Ala Thr Asn Arg Val Val Ala Pro Thr Pro Gly
           35                  40                  45
   Pro Gly Thr Pro Ala Glu Arg His Ala Asp Gly Leu Ala Leu Ala Leu
       50                  55                  60
   Glu Pro Ala Leu Ala Ser Pro Ala Gly Ala Ala Asn Phe Leu Ala Met
   65                  70                  75                  80
   Val Asp Asn Leu Gln Gly Asp Ser Gly Arg Gly Tyr Tyr Leu Glu Met
                   85                  90                  95
   Leu Ile Gly Thr Pro Pro Gln Lys Leu Gln Ile Leu Val Asp Thr Gly
               100                 105                 110
   Ser Ser Asn Phe Ala Val Ala Gly Thr Pro His Ser Tyr Ile Asp Thr
           115                 120                 125
   Tyr Phe Asp Thr Glu Arg Ser Ser Thr Tyr Arg Ser Lys Gly Phe Asp
       130                 135                 140
   Val Thr Val Lys Tyr Thr Gln Gly Ser Trp Thr Gly Phe Val Gly Glu
   145                 150                 155                 160
   Asp Leu Val Thr Ile Pro Lys Gly Phe Asn Thr Ser Phe Leu Val Asn
                   165                 170                 175
   Ile Ala Thr Ile Phe Glu Ser Glu Asn Phe Phe Leu Pro Gly Ile Lys
               180                 185                 190
   Trp Asn Gly Ile Leu Gly Leu Ala Tyr Ala Thr Leu Ala Lys Pro Ser
           195                 200                 205
   Ser Ser Leu Glu Thr Phe Phe Asp Ser Leu Val Thr Gln Ala Asn Ile
       210                 215                 220
   Pro Asn Val Phe Ser Met Gln Met Cys Gly Ala Gly Leu Pro Val Ala
   225                 230                 235                 240
   Gly Ser Gly Thr Asn Gly Gly Ser Leu Val Leu Gly Gly Ile Glu Pro
                   245                 250                 255
   Ser Leu Tyr Lys Gly Asp Ile Trp Tyr Thr Pro Ile Lys Glu Glu Trp
               260                 265                 270
   Tyr Tyr Gln Ile Glu Ile Leu Lys Leu Glu Ile Gly Gly Gln Ser Leu
           275                 280                 285
   Asn Leu Asp Cys Arg Glu Tyr Asn Ala Asp Lys Ala Ile Val Asp Ser
       290                 295                 300
   Gly Thr Thr Leu Leu Arg Leu Pro Gln Lys Val Phe Asp Ala Val Val
   305                 310                 315                 320
   Glu Ala Val Ala Arg Ala Ser Leu Ile Pro Glu Phe Ser Asp Gly Phe
                   325                 330                 335
   Trp Thr Gly Ser Gln Leu Ala Cys Trp Thr Asn Ser Glu Thr Pro Trp
               340                 345                 350
   Ser Tyr Phe Pro Lys Ile Ser Ile Tyr Leu Arg Asp Glu Asn Ser Ser
           355                 360                 365
   Arg Ser Phe Arg Ile Thr Ile Leu Pro Gln Leu Tyr Ile Gln Pro Met
       370                 375                 380
   Met Gly Ala Gly Leu Asn Tyr Glu Cys Tyr Arg Phe Gly Ile Ser Pro
   385                 390                 395                 400
   Ser Thr Asn Ala Leu Val Ile Gly Ala Thr Val Met Glu Gly Phe Tyr
                   405                 410                 415
   Val Ile Phe Asp Arg Ala Gln Lys Arg Val Gly Phe Ala Ala Ser Pro
               420                 425                 430
   Cys Ala Glu Ile Ala Gly Ala Ala Val Ser Glu Ile Ser Gly Pro Phe
           435                 440                 445
   Ser Thr Glu Asp Val Ala Ser Asn Cys Val Pro Ala Gln Ser Leu Ser
       450                 455                 460
   Glu Pro Ile Leu Trp Ile Val Ser Tyr Ala Leu Met Ser Val Cys Gly
   465                 470                 475                 480
   Ala Ile Leu Leu Val Leu Ile Val Leu Leu Leu Leu Pro Phe Arg Cys
                   485                 490                 495
   Gln Arg Arg Pro Arg Asp Pro Glu Val Val Asn Asp Glu Ser Ser Leu
               500                 505                 510
   Val Arg His Arg Trp Lys
           515
<210> 3
   <211> 2070
   <212> DNA
   <213> Homo sapiens
<400> 3
   atggcccaag ccctgccctg gctcctgctg tggatgggcg cgggagtgct gcctgcccac 60
   ggcacccagc acggcatccg gctgcccctg cgcagcggcc tggggggcgc ccccctgggg 120
   ctgcggctgc cccgggagac cgacgaagag cccgaggagc ccggccggag gggcagcttt 180
   gtggagatgg tggacaacct gaggggcaag tcggggcagg gctactacgt ggagatgacc 240
   gtgggcagcc ccccgcagac gctcaacatc ctggtggata caggcagcag taactttgca 300
   gtgggtgctg ccccccaccc cttcctgcat cgctactacc agaggcagct gtccagcaca 360
   taccgggacc tccggaaggg tgtgtatgtg ccctacaccc agggcaagtg ggaaggggag 420
   ctgggcaccg acctggtaag catcccccat ggccccaacg tcactgtgcg tgccaacatt 480
   gctgccatca ctgaatcaga caagttcttc atcaacggct ccaactggga aggcatcctg 540
   gggctggcct atgctgagat tgccaggcct gacgactccc tggagccttt ctttgactct 600
   ctggtaaagc agacccacgt tcccaacctc ttctccctgc acctttgtgg tgctggcttc 660
   cccctcaacc agtctgaagt gctggcctct gtcggaggga gcatgatcat tggaggtatc 720
   gaccactcgc tgtacacagg cagtctctgg tatacaccca tccggcggga gtggtattat 780
   gaggtcatca ttgtgcgggt ggagatcaat ggacaggatc tgaaaatgga ctgcaaggag 840
   tacaactatg acaagagcat tgtggacagt ggcaccacca accttcgttt gcccaagaaa 900
   gtgtttgaag ctgcagtcaa atccatcaag gcagcctcct ccacggagaa gttccctgat 960
   ggtttctggc taggagagca gctggtgtgc tggcaagcag gcaccacccc ttggaacatt 1020
   ttcccagtca tctcactcta cctaatgggt gaggttacca accagtcctt ccgcatcacc 1080
   atccttccgc agcaatacct gcggccagtg gaagatgtgg ccacgtccca agacgactgt 1140
   tacaagtttg ccatctcaca gtcatccacg ggcactgtta tgggagctgt tatcatggag 1200
   ggcttctacg ttgtctttga tcgggcccga aaacgaattg gctttgctgt cagcgcttgc 1260
   catgtgcacg atgagttcag gacggcagcg gtggaaggcc cttttgtcac cttggacatg 1320
   gaagactgtg gctacaacat tccacagaca gatgagtcaa ccctcatgac catagcctat 1380
   gtcatggctg ccatctgcgc cctcttcatg ctgccactct gcctcatggt gtgtcagtgg 1440
   cgctgcctcc gctgcctgcg ccagcagcat gatgactttg ctgatgacat ctccctgctg 1500
   aagtgaggag gcccatgggc agaagataga gattcccctg gaccacacct ccgtggttca 1560
   ctttggtcac aagtaggaga cacagatggc acctgtggcc agagcacctc aggaccctcc 1620
   ccacccacca aatgcctctg ccttgatgga gaaggaaaag gctggcaagg tgggttccag 1680
   ggactgtacc tgtaggaaac agaaaagaga agaaagaagc actctgctgg cgggaatact 1740
   cttggtcacc tcaaatttaa gtcgggaaat tctgctgctt gaaacttcag ccctgaacct 1800
   ttgtccacca ttcctttaaa ttctccaacc caaagtattc ttcttttctt agtttcagaa 1860
   gtactggcat cacacgcagg ttaccttggc gtgtgtccct gtggtaccct ggcagagaag 1920
   agaccaagct tgtttccctg ctggccaaag tcagtaggag aggatgcaca gtttgctatt 1980
   tgctttagag acagggactg tataaacaag cctaacattg gtgcaaagat tgcctcttga 2040
   attaaaaaaa aaaaaaaaaa aaaaaaaaaa 2070
<210> 4
   <211> 501
   <212> PRT
   <213> Homo sapiens
<400> 4
   Met Ala Gln Ala Leu Pro Trp Leu Leu Leu Trp Met Gly Ala Gly Val
    1               5                  10                  15
   Leu Pro Ala His Gly Thr Gln His Gly Ile Arg Leu Pro Leu Arg Ser
               20                  25                  30
   Gly Leu Gly Gly Ala Pro Leu Gly Leu Arg Leu Pro Arg Glu Thr Asp
           35                  40                  45
   Glu Glu Pro Glu Glu Pro Gly Arg Arg Gly Ser Phe Val Glu Met Val
       50                  55                  60
   Asp Asn Leu Arg Gly Lys Ser Gly Gln Gly Tyr Tyr Val Glu Met Thr
   65                  70                  75                  80
   Val Gly Ser Pro Pro Gln Thr Leu Asn Ile Leu Val Asp Thr Gly Ser
                   85                  90                  95
   Ser Asn Phe Ala Val Gly Ala Ala Pro His Pro Phe Leu His Arg Tyr
               100                 105                 110
   Tyr Gln Arg Gln Leu Ser Ser Thr Tyr Arg Asp Leu Arg Lys Gly Val
           115                 120                 125
   Tyr Val Pro Tyr Thr Gln Gly Lys Trp Glu Gly Glu Leu Gly Thr Asp
       130                 135                 140
   Leu Val Ser Ile Pro His Gly Pro Asn Val Thr Val Arg Ala Asn Ile
   145                 150                 155                 160
   Ala Ala Ile Thr Glu Ser Asp Lys Phe Phe Ile Asn Gly Ser Asn Trp
                   165                 170                 175
   Glu Gly Ile Leu Gly Leu Ala Tyr Ala Glu Ile Ala Arg Pro Asp Asp
               180                 185                 190
   Ser Leu Glu Pro Phe Phe Asp Ser Leu Val Lys Gln Thr His Val Pro
           195                 200                 205
   Asn Leu Phe Ser Leu His Leu Cys Gly Ala Gly Phe Pro Leu Asn Gln
       210                 215                 220
   Ser Glu Val Leu Ala Ser Val Gly Gly Ser Met Ile Ile Gly Gly Ile
   225                 230                 235                 240
   Asp His Ser Leu Tyr Thr Gly Ser Leu Trp Tyr Thr Pro Ile Arg Arg
                   245                 250                 255
   Glu Trp Tyr Tyr Glu Val Ile Ile Val Arg Val Glu Ile Asn Gly Gln
               260                 265                 270
   Asp Leu Lys Met Asp Cys Lys Glu Tyr Asn Tyr Asp Lys Ser Ile Val
           275                 280                 285
   Asp Ser Gly Thr Thr Asn Leu Arg Leu Pro Lys Lys Val Phe Glu Ala
       290                 295                 300
   Ala Val Lys Ser Ile Lys Ala Ala Ser Ser Thr Glu Lys Phe Pro Asp
   305                 310                 315                 320
   Gly Phe Trp Leu Gly Glu Gln Leu Val Cys Trp Gln Ala Gly Thr Thr
                   325                 330                 335
   Pro Trp Asn Ile Phe Pro Val Ile Ser Leu Tyr Leu Met Gly Glu Val
               340                 345                 350
   Thr Asn Gln Ser Phe Arg Ile Thr Ile Leu Pro Gln Gln Tyr Leu Arg
           355                 360                 365
   Pro Val Glu Asp Val Ala Thr Ser Gln Asp Asp Cys Tyr Lys Phe Ala
       370                 375                 380
   Ile Ser Gln Ser Ser Thr Gly Thr Val Met Gly Ala Val Ile Met Glu
   385                 390                 395                 400
   Gly Phe Tyr Val Val Phe Asp Arg Ala Arg Lys Arg Ile Gly Phe Ala
                   405                 410                 415
   Val Ser Ala Cys His Val His Asp Glu Phe Arg Thr Ala Ala Val Glu
               420                 425                 430
   Gly Pro Phe Val Thr Leu Asp Met Glu Asp Cys Gly Tyr Asn Ile Pro
           435                 440                 445
   Gln Thr Asp Glu Ser Thr Leu Met Thr Ile Ala Tyr Val Met Ala Ala
   450 455 460
   Ile Cys Ala Leu Phe Met Leu Pro Leu Cys Leu Met Val Cys Gln Trp
   465 470 475 480
   Arg Cys Leu Arg Cys Leu Arg Gln Gln His Asp Asp Phe Ala Asp Asp
   485 490 495
   Ile Ser Leu Leu Lys
   500
<210> 5
   <211> 1977
   <212> DNA
   <213> Homo sapiens
<400> 5
   atggcccaag ccctgccctg gctcctgctg tggatgggcg cgggagtgct gcctgcccac 60
   ggcacccagc acggcatccg gctgcccctg cgcagcggcc tggggggcgc ccccctgggg 120
   ctgcggctgc cccgggagac cgacgaagag cccgaggagc ccggccggag gggcagcttt 180
   gtggagatgg tggacaacct gaggggcaag tcggggcagg gctactacgt ggagatgacc 240
   gtgggcagcc ccccgcagac gctcaacatc ctggtggata caggcagcag taactttgca 300
   gtgggtgctg ccccccaccc cttcctgcat cgctactacc agaggcagct gtccagcaca 360
   taccgggacc tccggaaggg tgtgtatgtg ccctacaccc agggcaagtg ggaaggggag 420
   ctgggcaccg acctggtaag catcccccat ggccccaacg tcactgtgcg tgccaacatt 480
   gctgccatca ctgaatcaga caagttcttc atcaacggct ccaactggga aggcatcctg 540
   gggctggcct atgctgagat tgccaggctt tgtggtgctg gcttccccct caaccagtct 600
   gaagtgctgg cctctgtcgg agggagcatg atcattggag gtatcgacca ctcgctgtac 660
   acaggcagtc tctggtatac acccatccgg cgggagtggt attatgaggt gatcattgtg 720
   cgggtggaga tcaatggaca ggatctgaaa atggactgca aggagtacaa ctatgacaag 780
   agcattgtgg acagtggcac caccaacctt cgtttgccca agaaagtgtt tgaagctgca 840
   gtcaaatcca tcaaggcagc ctcctccacg gagaagttcc ctgatggttt ctggctagga 900
   gagcagctgg tgtgctggca agcaggcacc accccttgga acattttccc agtcatctca 960
   ctctacctaa tgggtgaggt taccaaccag tccttccgca tcaccatcct tccgcagcaa 1020
   tacctgcggc cagtggaaga tgtggccacg tcccaagacg actgttacaa gtttgccatc 1080
   tcacagtcat ccacgggcac tgttatggga gctgttatca tggagggctt ctacgttgtc 1140
   tttgatcggg cccgaaaacg aattggcttt gctgtcagcg cttgccatgt gcacgatgag 1200
   ttcaggacgg cagcggtgga aggccctttt gtcaccttgg acatggaaga ctgtggctac 1260
   aacattccac agacagatga gtcaaccctc atgaccatag cctatgtcat ggctgccatc 1320
   tgcgccctct tcatgctgcc actctgcctc atggtgtgtc agtggcgctg cctccgctgc 1380
   ctgcgccagc agcatgatga ctttgctgat gacatctccc tgctgaagtg aggaggccca 1440
   tgggcagaag atagagattc ccctggacca cacctccgtg gttcactttg gtcacaagta 1500
   ggagacacag atggcacctg tggccagagc acctcaggac cctccccacc caccaaatgc 1560
   ctctgccttg atggagaagg aaaaggctgg caaggtgggt tccagggact gtacctgtag 1620
   gaaacagaaa agagaagaaa gaagcactct gctggcggga atactcttgg tcacctcaaa 1680
   tttaagtcgg gaaattctgc tgcttgaaac ttcagccctg aacctttgtc caccattcct 1740
   ttaaattctc caacccaaag tattcttctt ttcttagttt cagaagtact ggcatcacac 1800
   gcaggttacc ttggcgtgtg tccctgtggt accctggcag agaagagacc aagcttgttt 1860
   ccctgctggc caaagtcagt aggagaggat gcacagtttg ctatttgctt tagagacagg 1920
   gactgtataa acaagcctaa cattggtgca aagattgcct cttgaaaaaa aaaaaaa 1977
<210> 6
   <211> 476
   <212> PRT
   <213> Homo sapiens
<400> 6
   Met Ala Gln Ala Leu Pro Trp Leu Leu Leu Trp Met Gly Ala Gly Val
    1               5                  10                  15
   Leu Pro Ala His Gly Thr Gln His Gly Ile Arg Leu Pro Leu Arg Ser
               20                  25                  30
   Gly Leu Gly Gly Ala Pro Leu Gly Leu Arg Leu Pro Arg Glu Thr Asp
           35                  40                  45
   Glu Glu Pro Glu Glu Pro Gly Arg Arg Gly Ser Phe Val Glu Met Val
       50                  55                  60
   Asp Asn Leu Arg Gly Lys Ser Gly Gln Gly Tyr Tyr Val Glu Met Thr
   65                  70                  75                  80
   Val Gly Ser Pro Pro Gln Thr Leu Asn Ile Leu Val Asp Thr Gly Ser
                   85                  90                  95
   Ser Asn Phe Ala Val Gly Ala Ala Pro His Pro Phe Leu His Arg Tyr
               100                 105                 110
   Tyr Gln Arg Gln Leu Ser Ser Thr Tyr Arg Asp Leu Arg Lys Gly Val
           115                 120                 125
   Tyr Val Pro Tyr Thr Gln Gly Lys Trp Glu Gly Glu Leu Gly Thr Asp
       130                 135                 140
   Leu Val Ser Ile Pro His Gly Pro Asn Val Thr Val Arg Ala Asn Ile
   145                 150                 155                 160
   Ala Ala Ile Thr Glu Ser Asp Lys Phe Phe Ile Asn Gly Ser Asn Trp
                   165                 170                 175
   Glu Gly Ile Leu Gly Leu Ala Tyr Ala Glu Ile Ala Arg Leu Cys Gly
               180                 185                 190
   Ala Gly Phe Pro Leu Asn Gln Ser Glu Val Leu Ala Ser Val Gly Gly
           195                 200                 205
   Ser Met Ile Ile Gly Gly Ile Asp His Ser Leu Tyr Thr Gly Ser Leu
       210                 215                 220
   Trp Tyr Thr Pro Ile Arg Arg Glu Trp Tyr Tyr Glu Val Ile Ile Val
   225                 230                 235                 240
   Arg Val Glu Ile Asn Gly Gln Asp Leu Lys Met Asp Cys Lys Glu Tyr
                   245                 250                 255
   Asn Tyr Asp Lys Ser Ile Val Asp Ser Gly Thr Thr Asn Leu Arg Leu
               260                 265                 270
   Pro Lys Lys Val Phe Glu Ala Ala Val Lys Ser Ile Lys Ala Ala Ser
           275                 280                 285
   Ser Thr Glu Lys Phe Pro Asp Gly Phe Trp Leu Gly Glu Gln Leu Val
       290                 295                 300
   Cys Trp Gln Ala Gly Thr Thr Pro Trp Asn Ile Phe Pro Val Ile Ser
   305                 310                 315                 320
   Leu Tyr Leu Met Gly Glu Val Thr Asn Gln Ser Phe Arg Ile Thr Ile
                   325                 330                 335
   Leu Pro Gln Gln Tyr Leu Arg Pro Val Glu Asp Val Ala Thr Ser Gln
               340                 345                 350
   Asp Asp Cys Tyr Lys Phe Ala Ile Ser Gln Ser Ser Thr Gly Thr Val
           355                 360                 365
   Met Gly Ala Val Ile Met Glu Gly Phe Tyr Val Val Phe Asp Arg Ala
       370                 375                 380
   Arg Lys Arg Ile Gly Phe Ala Val Ser Ala Cys His Val His Asp Glu
   385                 390                 395                 400
   Phe Arg Thr Ala Ala Val Glu Gly Pro Phe Val Thr Leu Asp Met Glu
                   405                 410                 415
   Asp Cys Gly Tyr Asn Ile Pro Gln Thr Asp Glu Ser Thr Leu Met Thr
               420                 425                 430
   Ile Ala Tyr Val Met Ala Ala Ile Cys Ala Leu Phe Met Leu Pro Leu
           435                 440                 445
   Cys Leu Met Val Cys Gln Trp Arg Cys Leu Arg Cys Leu Arg Gln Gln
       450                 455                 460
   His Asp Asp Phe Ala Asp Asp Ile Ser Leu Leu Lys
   465                 470                 475
<210> 7
   <211> 2043
   <212> DNA
   <213> Mus musculus
<400> 7
   atggccccag cgctgcactg gctcctgcta tgggtgggct cgggaatgct gcctgcccag 60
   ggaacccatc tcggcatccg gctgcccctt cgcagcggcc tggcagggcc acccctgggc 120
   ctgaggctgc cccgggagac tgacgaggaa tcggaggagc ctggccggag aggcagcttt 180
   gtggagatgg tggacaacct gaggggaaag tccggccagg gctactatgt ggagatgacc 240
   gtaggcagcc ccccacagac gctcaacatc ctggtggaca cgggcagtag taactttgca 300
   gtgggggctg ccccacaccc tttcctgcat cgctactacc agaggcagct gtccagcaca 360
   tatcgagacc tccgaaaggg tgtgtatgtg ccctacaccc agggcaagtg ggagggggaa 420
   ctgggcaccg acctggtgag catccctcat ggccccaacg tcactgtgcg tgccaacatt 480
   gctgccatca ctgaatcgga caagttcttc atcaatggtt ccaactggga gggcatccta 540
   gggctggcct atgctgagat tgccaggccc gacgactctt tggagccctt ctttgactcc 600
   ctggtgaagc agacccacat tcccaacatc ttttccctgc agctctgtgg cgctggcttc 660
   cccctcaacc agaccgaggc actggcctcg gtgggaggga gcatgatcat tggtggtatc 720
   gaccactcgc tatacacggg cagtctctgg tacacaccca tccggcggga gtggtattat 780
   gaagtgatca ttgtacgtgt ggaaatcaat ggtcaagatc tcaagatgga ctgcaaggag 840
   tacaactacg acaagagcat tgtggacagt gggaccacca accttcgctt gcccaagaaa 900
   gtatttgaag ctgccgtcaa gtccatcaag gcagcctcct cgacggagaa gttcccggat 960
   ggcttttggc taggggagca gctggtgtgc tggcaagcag gcacgacccc ttggaacatt 1020
   ttcccagtca tttcacttta cctcatgggt gaagtcacca atcagtcctt ccgcatcacc 1080
   atccttcctc agcaatacct acggccggtg gaggacgtgg ccacgtccca agacgactgt 1140
   tacaagttcg ctgtctcaca gtcatccacg ggcactgtta tgggagccgt catcatggaa 1200
   ggtttctatg tcgtcttcga tcgagcccga aagcgaattg gctttgctgt cagcgcttgc 1260
   catgtgcacg atgagttcag gacggcggca gtggaaggtc cgtttgttac ggcagacatg 1320
   gaagactgtg gctacaacat tccccagaca gatgagtcaa cacttatgac catagcctat 1380
   gtcatggcgg ccatctgcgc cctcttcatg ttgccactct gcctcatggt atgtcagtgg 1440
   cgctgcctgc gttgcctgcg ccaccagcac gatgactttg ctgatgacat ctccctgctc 1500
   aagtaaggag gctcgtgggc agatgatgga gacgcccctg gaccacatct gggtggttcc 1560
   ctttggtcac atgagttgga gctatggatg gtacctgtgg ccagagcacc tcaggaccct 1620
   caccaacctg ccaatgcttc tggcgtgaca gaacagagaa atcaggcaag ctggattaca 1680
   gggcttgcac ctgtaggaca caggagaggg aaggaagcag cgttctggtg gcaggaatat 1740
   ccttaggcac cacaaacttg agttggaaat tttgctgctt gaagcttcag ccctgaccct 1800
   ctgcccagca tcctttagag tctccaacct aaagtattct ttatgtcctt ccagaagtac 1860
   tggcgtcata ctcaggctac ccggcatgtg tccctgtggt accctggcag agaaagggcc 1920
   aatctcattc cctgctggcc aaagtcagca gaagaaggtg aagtttgcca gttgctttag 1980
   tgatagggac tgcagactca agcctacact ggtacaaaga ctgcgtcttg agataaacaa 2040
   gaa 2043
<210> 8
   <211> 501
   <212> PRT
   <213> Mus musculus
<400> 8
   Met Ala Pro Ala Leu His Trp Leu Leu Leu Trp Val Gly Ser Gly Met
    1               5                  10                  15
   Leu Pro Ala Gln Gly Thr His Leu Gly Ile Arg Leu Pro Leu Arg Ser
               20                  25                  30
   Gly Leu Ala Gly Pro Pro Leu Gly Leu Arg Leu Pro Arg Glu Thr Asp
           35                  40                  45
   Glu Glu Ser Glu Glu Pro Gly Arg Arg Gly Ser Phe Val Glu Met Val
       50                  55                  60
   Asp Asn Leu Arg Gly Lys Ser Gly Gln Gly Tyr Tyr Val Glu Met Thr
   65                  70                  75                  80
   Val Gly Ser Pro Pro Gln Thr Leu Asn Ile Leu Val Asp Thr Gly Ser
                   85                  90                  95
   Ser Asn Phe Ala Val Gly Ala Ala Pro His Pro Phe Leu His Arg Tyr
               100                 105                 110
   Tyr Gln Arg Gln Leu Ser Ser Thr Tyr Arg Asp Leu Arg Lys Gly Val
           115                 120                 125
   Tyr Val Pro Tyr Thr Gln Gly Lys Trp Glu Gly Glu Leu Gly Thr Asp
       130                 135                 140
   Leu Val Ser Ile Pro His Gly Pro Asn Val Thr Val Arg Ala Asn Ile
   145                 150                 155                 160
   Ala Ala Ile Thr Glu Ser Asp Lys Phe Phe Ile Asn Gly Ser Asn Trp
                   165                 170                 175
   Glu Gly Ile Leu Gly Leu Ala Tyr Ala Glu Ile Ala Arg Pro Asp Asp
               180                 185                 190
   Ser Leu Glu Pro Phe Phe Asp Ser Leu Val Lys Gln Thr His Ile Pro
           195                 200                 205
   Asn Ile Phe Ser Leu Gln Leu Cys Gly Ala Gly Phe Pro Leu Asn Gln
       210                 215                 220
   Thr Glu Ala Leu Ala Ser Val Gly Gly Ser Met Ile Ile Gly Gly Ile
   225                 230                 235                 240
   Asp His Ser Leu Tyr Thr Gly Ser Leu Trp Tyr Thr Pro Ile Arg Arg
                   245                 250                 255
   Glu Trp Tyr Tyr Glu Val Ile Ile Val Arg Val Glu Ile Asn Gly Gln
               260                 265                 270
   Asp Leu Lys Met Asp Cys Lys Glu Tyr Asn Tyr Asp Lys Ser Ile Val
           275                 280                 285
   Asp Ser Gly Thr Thr Asn Leu Arg Leu Pro Lys Lys Val Phe Glu Ala
       290                 295                 300
   Ala Val Lys Ser Ile Lys Ala Ala Ser Ser Thr Glu Lys Phe Pro Asp
   305                 310                 315                 320
   Gly Phe Trp Leu Gly Glu Gln Leu Val Cys Trp Gln Ala Gly Thr Thr
                   325                 330                 335
   Pro Trp Asn Ile Phe Pro Val Ile Ser Leu Tyr Leu Met Gly Glu Val
               340                 345                 350
   Thr Asn Gln Ser Phe Arg Ile Thr Ile Leu Pro Gln Gln Tyr Leu Arg
           355                 360                 365
   Pro Val Glu Asp Val Ala Thr Ser Gln Asp Asp Cys Tyr Lys Phe Ala
       370                 375                 380
   Val Ser Gln Ser Ser Thr Gly Thr Val Met Gly Ala Val Ile Met Glu
   385                 390                 395                 400
   Gly Phe Tyr Val Val Phe Asp Arg Ala Arg Lys Arg Ile Gly Phe Ala
                   405                 410                 415
   Val Ser Ala Cys His Val His Asp Glu Phe Arg Thr Ala Ala Val Glu
               420                 425                 430
   Gly Pro Phe Val Thr Ala Asp Met Glu Asp Cys Gly Tyr Asn Ile Pro
           435                 440                 445
   Gln Thr Asp Glu Ser Thr Leu Met Thr Ile Ala Tyr Val Met Ala Ala
       450                 455                 460
   Ile Cys Ala Leu Phe Met Leu Pro Leu Cys Leu Met Val Cys Gln Trp
   465                 470                 475                 480
   Arg Cys Leu Arg Cys Leu Arg His Gln His Asp Asp Phe Ala Asp Asp
                   485                 490                 495
   Ile Ser Leu Leu Lys
               500
<210> 9
   <211> 2088
   <212> DNA
   <213> Homo sapiens
<400> 9
   atgctgcccg gtttggcact gctcctgctg gccgcctgga cggctcgggc gctggaggta 60
   cccactgatg gtaatgctgg cctgctggct gaaccccaga ttgccatgtt ctgtggcaga 120
   ctgaacatgc acatgaatgt ccagaatggg aagtgggatt cagatccatc agggaccaaa 180
   acctgcattg ataccaagga aggcatcctg cagtattgcc aagaagtcta ccctgaactg 240
   cagatcacca atgtggtaga agccaaccaa ccagtgacca tccagaactg gtgcaagcgg 300
   ggccgcaagc agtgcaagac ccatccccac tttgtgattc cctaccgctg cttagttggt 360
   gagtttgtaa gtgatgccct tctcgttcct gacaagtgca aattcttaca ccaggagagg 420
   atggatgttt gcgaaactca tcttcactgg cacaccgtcg ccaaagagac atgcagtgag 480
   aagagtacca acttgcatga ctacggcatg ttgctgccct gcggaattga caagttccga 540
   ggggtagagt ttgtgtgttg cccactggct gaagaaagtg acaatgtgga ttctgctgat 600
   gcggaggagg atgactcgga tgtctggtgg ggcggagcag acacagacta tgcagatggg 660
   agtgaagaca aagtagtaga agtagcagag gaggaagaag tggctgaggt ggaagaagaa 720
   gaagccgatg atgacgagga cgatgaggat ggtgatgagg tagaggaaga ggctgaggaa 780
   ccctacgaag aagccacaga gagaaccacc agcattgcca ccaccaccac caccaccaca 840
   gagtctgtgg aagaggtggt tcgagttcct acaacagcag ccagtacccc tgatgccgtt 900
   gacaagtatc tcgagacacc tggggatgag aatgaacatg cccatttcca gaaagccaaa 960
   gagaggcttg aggccaagca ccgagagaga atgtcccagg tcatgagaga atgggaagag 1020
   gcagaacgtc aagcaaagaa cttgcctaaa gctgataaga aggcagttat ccagcatttc 1080
   caggagaaag tggaatcttt ggaacaggaa gcagccaacg agagacagca gctggtggag 1140
   acacacatgg ccagagtgga agccatgctc aatgaccgcc gccgcctggc cctggagaac 1200
   tacatcaccg ctctgcaggc tgttcctcct cggcctcgtc acgtgttcaa tatgctaaag 1260
   aagtatgtcc gcgcagaaca gaaggacaga cagcacaccc taaagcattt cgagcatgtg 1320
   cgcatggtgg atcccaagaa agccgctcag atccggtccc aggttatgac acacctccgt 1380
   gtgatttatg agcgcatgaa tcagtctctc tccctgctct acaacgtgcc tgcagtggcc 1440
   gaggagattc aggatgaagt tgatgagctg cttcagaaag agcaaaacta ttcagatgac 1500
   gtcttggcca acatgattag tgaaccaagg atcagttacg gaaacgatgc tctcatgcca 1560
   tctttgaccg aaacgaaaac caccgtggag ctccttcccg tgaatggaga gttcagcctg 1620
   gacgatctcc agccgtggca ttcttttggg gctgactctg tgccagccaa cacagaaaac 1680
   gaagttgagc ctgttgatgc ccgccctgct gccgaccgag gactgaccac tcgaccaggt 1740
   tctgggttga caaatatcaa gacggaggag atctctgaag tgaagatgga tgcagaattc 1800
   cgacatgact caggatatga agttcatcat caaaaattgg tgttctttgc agaagatgtg 1860
   ggttcaaaca aaggtgcaat cattggactc atggtgggcg gtgttgtcat agcgacagtg 1920
   atcgtcatca ccttggtgat gctgaagaag aaacagtaca catccattca tcatggtgtg 1980
   gtggaggttg acgccgctgt caccccagag gagcgccacc tgtccaagat gcagcagaac 2040
   ggctacgaaa atccaaccta caagttcttt gagcagatgc agaactag 2088
<210> 10
   <211> 695
   <212> PRT
   <213> Homo sapiens
<400> 10
   Met Leu Pro Gly Leu Ala Leu Leu Leu Leu Ala Ala Trp Thr Ala Arg
    1               5                  10                  15
   Ala Leu Glu Val Pro Thr Asp Gly Asn Ala Gly Leu Leu Ala Glu Pro
               20                  25                  30
   Gln Ile Ala Met Phe Cys Gly Arg Leu Asn Met His Met Asn Val Gln
           35                  40                  45
   Asn Gly Lys Trp Asp Ser Asp Pro Ser Gly Thr Lys Thr Cys Ile Asp
       50                  55                  60
   Thr Lys Glu Gly Ile Leu Gln Tyr Cys Gln Glu Val Tyr Pro Glu Leu
   65                  70                  75                  80
   Gln Ile Thr Asn Val Val Glu Ala Asn Gln Pro Val Thr Ile Gln Asn
                   85                  90                  95
   Trp Cys Lys Arg Gly Arg Lys Gln Cys Lys Thr His Pro His Phe Val
               100                 105                 110
   Ile Pro Tyr Arg Cys Leu Val Gly Glu Phe Val Ser Asp Ala Leu Leu
           115                 120                 125
   Val Pro Asp Lys Cys Lys Phe Leu His Gln Glu Arg Met Asp Val Cys
       130                 135                 140
   Glu Thr His Leu His Trp His Thr Val Ala Lys Glu Thr Cys Ser Glu
   145                 150                 155                 160
   Lys Ser Thr Asn Leu His Asp Tyr Gly Met Leu Leu Pro Cys Gly Ile
                   165                 170                 175
   Asp Lys Phe Arg Gly Val Glu Phe Val Cys Cys Pro Leu Ala Glu Glu
               180                 185                 190
   Ser Asp Asn Val Asp Ser Ala Asp Ala Glu Glu Asp Asp Ser Asp Val
           195                 200                 205
   Trp Trp Gly Gly Ala Asp Thr Asp Tyr Ala Asp Gly Ser Glu Asp Lys
       210                 215                 220
   Val Val Glu Val Ala Glu Glu Glu Glu Val Ala Glu Val Glu Glu Glu
   225                 230                 235                 240
   Glu Ala Asp Asp Asp Glu Asp Asp Glu Asp Gly Asp Glu Val Glu Glu
                   245                 250                 255
   Glu Ala Glu Glu Pro Tyr Glu Glu Ala Thr Glu Arg Thr Thr Ser Ile
               260                 265                 270
   Ala Thr Thr Thr Thr Thr Thr Thr Glu Ser Val Glu Glu Val Val Arg
           275                 280                 285
   Val Pro Thr Thr Ala Ala Ser Thr Pro Asp Ala Val Asp Lys Tyr Leu
       290                 295                 300
   Glu Thr Pro Gly Asp Glu Asn Glu His Ala His Phe Gln Lys Ala Lys
   305                 310                 315                 320
   Glu Arg Leu Glu Ala Lys His Arg Glu Arg Met Ser Gln Val Met Arg
                   325                 330                 335
   Glu Trp Glu Glu Ala Glu Arg Gln Ala Lys Asn Leu Pro Lys Ala Asp
               340                 345                 350
   Lys Lys Ala Val Ile Gln His Phe Gln Glu Lys Val Glu Ser Leu Glu
           355                 360                 365
   Gln Glu Ala Ala Asn Glu Arg Gln Gln Leu Val Glu Thr His Met Ala
       370                 375                 380
   Arg Val Glu Ala Met Leu Asn Asp Arg Arg Arg Leu Ala Leu Glu Asn
   385                 390                 395                 400
   Tyr Ile Thr Ala Leu Gln Ala Val Pro Pro Arg Pro Arg His Val Phe
                   405                 410                 415
   Asn Met Leu Lys Lys Tyr Val Arg Ala Glu Gln Lys Asp Arg Gln His
               420                 425                 430
   Thr Leu Lys His Phe Glu His Val Arg Met Val Asp Pro Lys Lys Ala
           435                 440                 445
   Ala Gln Ile Arg Ser Gln Val Met Thr His Leu Arg Val Ile Tyr Glu
       450                 455                 460
   Arg Met Asn Gln Ser Leu Ser Leu Leu Tyr Asn Val Pro Ala Val Ala
   465                 470                 475                 480
   Glu Glu Ile Gln Asp Glu Val Asp Glu Leu Leu Gln Lys Glu Gln Asn
                   485                 490                 495
   Tyr Ser Asp Asp Val Leu Ala Asn Met Ile Ser Glu Pro Arg Ile Ser
               500                 505                 510
   Tyr Gly Asn Asp Ala Leu Met Pro Ser Leu Thr Glu Thr Lys Thr Thr
           515                 520                 525
   Val Glu Leu Leu Pro Val Asn Gly Glu Phe Ser Leu Asp Asp Leu Gln
       530                 535                 540
   Pro Trp His Ser Phe Gly Ala Asp Ser Val Pro Ala Asn Thr Glu Asn
   545                 550                 555                 560
   Glu Val Glu Pro Val Asp Ala Arg Pro Ala Ala Asp Arg Gly Leu Thr
                   565                 570                 575
   Thr Arg Pro Gly Ser Gly Leu Thr Asn Ile Lys Thr Glu Glu Ile Ser
               580                 585                 590
   Glu Val Lys Met Asp Ala Glu Phe Arg His Asp Ser Gly Tyr Glu Val
           595                 600                 605
   His His Gln Lys Leu Val Phe Phe Ala Glu Asp Val Gly Ser Asn Lys
       610                 615                 620
   Gly Ala Ile Ile Gly Leu Met Val Gly Gly Val Val Ile Ala Thr Val
   625                 630                 635                 640
   Ile Val Ile Thr Leu Val Met Leu Lys Lys Lys Gln Tyr Thr Ser Ile
                   645                 650                 655
   His His Gly Val Val Glu Val Asp Ala Ala Val Thr Pro Glu Glu Arg
               660                 665                 670
   His Leu Ser Lys Met Gln Gln Asn Gly Tyr Glu Asn Pro Thr Tyr Lys
           675                 680                 685
   Phe Phe Glu Gln Met Gln Asn
       690                 695
<210> 11
   <211> 2088
   <212> DNA
   <213> Homo sapiens
<400> 11
   atgctgcccg gtttggcact gctcctgctg gccgcctgga cggctcgggc gctggaggta 60
   cccactgatg gtaatgctgg cctgctggct gaaccccaga ttgccatgtt ctgtggcaga 120
   ctgaacatgc acatgaatgt ccagaatggg aagtgggatt cagatccatc agggaccaaa 180
   acctgcattg ataccaagga aggcatcctg cagtattgcc aagaagtcta ccctgaactg 240
   cagatcacca atgtggtaga agccaaccaa ccagtgacca tccagaactg gtgcaagcgg 300
   ggccgcaagc agtgcaagac ccatccccac tttgtgattc cctaccgctg cttagttggt 360
   gagtttgtaa gtgatgccct tctcgttcct gacaagtgca aattcttaca ccaggagagg 420
   atggatgttt gcgaaactca tcttcactgg cacaccgtcg ccaaagagac atgcagtgag 480
   aagagtacca acttgcatga ctacggcatg ttgctgccct gcggaattga caagttccga 540
   ggggtagagt ttgtgtgttg cccactggct gaagaaagtg acaatgtgga ttctgctgat 600
   gcggaggagg atgactcgga tgtctggtgg ggcggagcag acacagacta tgcagatggg 660
   agtgaagaca aagtagtaga agtagcagag gaggaagaag tggctgaggt ggaagaagaa 720
   gaagccgatg atgacgagga cgatgaggat ggtgatgagg tagaggaaga ggctgaggaa 780
   ccctacgaag aagccacaga gagaaccacc agcattgcca ccaccaccac caccaccaca 840
   gagtctgtgg aagaggtggt tcgagttcct acaacagcag ccagtacccc tgatgccgtt 900
   gacaagtatc tcgagacacc tggggatgag aatgaacatg cccatttcca gaaagccaaa 960
   gagaggcttg aggccaagca ccgagagaga atgtcccagg tcatgagaga atgggaagag 1020
   gcagaacgtc aagcaaagaa cttgcctaaa gctgataaga aggcagttat ccagcatttc 1080
   caggagaaag tggaatcttt ggaacaggaa gcagccaacg agagacagca gctggtggag 1140
   acacacatgg ccagagtgga agccatgctc aatgaccgcc gccgcctggc cctggagaac 1200
   tacatcaccg ctctgcaggc tgttcctcct cggcctcgtc acgtgttcaa tatgctaaag 1260
   aagtatgtcc gcgcagaaca gaaggacaga cagcacaccc taaagcattt cgagcatgtg 1320
   cgcatggtgg atcccaagaa agccgctcag atccggtccc aggttatgac acacctccgt 1380
   gtgatttatg agcgcatgaa tcagtctctc tccctgctct acaacgtgcc tgcagtggcc 1440
   gaggagattc aggatgaagt tgatgagctg cttcagaaag agcaaaacta ttcagatgac 1500
   gtcttggcca acatgattag tgaaccaagg atcagttacg gaaacgatgc tctcatgcca 1560
   tctttgaccg aaacgaaaac caccgtggag ctccttcccg tgaatggaga gttcagcctg 1620
   gacgatctcc agccgtggca ttcttttggg gctgactctg tgccagccaa cacagaaaac 1680
   gaagttgagc ctgttgatgc ccgccctgct gccgaccgag gactgaccac tcgaccaggt 1740
   tctgggttga caaatatcaa gacggaggag atctctgaag tgaatctgga tgcagaattc 1800
   cgacatgact caggatatga agttcatcat caaaaattgg tgttctttgc agaagatgtg 1860
   ggttcaaaca aaggtgcaat cattggactc atggtgggcg gtgttgtcat agcgacagtg 1920
   atcgtcatca ccttggtgat gctgaagaag aaacagtaca catccattca tcatggtgtg 1980
   gtggaggttg acgccgctgt caccccagag gagcgccacc tgtccaagat gcagcagaac 2040
   ggctacgaaa atccaaccta caagttcttt gagcagatgc agaactag          2088
<210> 12
   <211> 695
   <212> PRT
   <213> Homo sapiens
<400> 12
<210> 13
   <211> 2088
   <212> DNA
   <213> Homo sapiens
<400> 13
   atgctgcccg gtttggcact gctcctgctg gccgcctgga cggctcgggc gctggaggta 60
   cccactgatg gtaatgctgg cctgctggct gaaccccaga ttgccatgtt ctgtggcaga 120
   ctgaacatgc acatgaatgt ccagaatggg aagtgggatt cagatccatc agggaccaaa 180
   acctgcattg ataccaagga aggcatcctg cagtattgcc aagaagtcta ccctgaactg 240
   cagatcacca atgtggtaga agccaaccaa ccagtgacca tccagaactg gtgcaagcgg 300
   ggccgcaagc agtgcaagac ccatccccac tttgtgattc cctaccgctg cttagttggt 360
   gagtttgtaa gtgatgccct tctcgttcct gacaagtgca aattcttaca ccaggagagg 420
   atggatgttt gcgaaactca tcttcactgg cacaccgtcg ccaaagagac atgcagtgag 480
   aagagtacca acttgcatga ctacggcatg ttgctgccct gcggaattga caagttccga 540
   ggggtagagt ttgtgtgttg cccactggct gaagaaagtg acaatgtgga ttctgctgat 600
   gcggaggagg atgactcgga tgtctggtgg ggcggagcag acacagacta tgcagatggg 660
   agtgaagaca aagtagtaga agtagcagag gaggaagaag tggctgaggt ggaagaagaa 720
   gaagccgatg atgacgagga cgatgaggat ggtgatgagg tagaggaaga ggctgaggaa 780
   ccctacgaag aagccacaga gagaaccacc agcattgcca ccaccaccac caccaccaca 840
   gagtctgtgg aagaggtggt tcgagttcct acaacagcag ccagtacccc tgatgccgtt 900
   gacaagtatc tcgagacacc tggggatgag aatgaacatg cccatttcca gaaagccaaa 960
   gagaggcttg aggccaagca ccgagagaga atgtcccagg tcatgagaga atgggaagag 1020
   gcagaacgtc aagcaaagaa cttgcctaaa gctgataaga aggcagttat ccagcatttc 1080
   caggagaaag tggaatcttt ggaacaggaa gcagccaacg agagacagca gctggtggag 1140
   acacacatgg ccagagtgga agccatgctc aatgaccgcc gccgcctggc cctggagaac 1200
   tacatcaccg ctctgcaggc tgttcctcct cggcctcgtc acgtgttcaa tatgctaaag 1260
   aagtatgtcc gcgcagaaca gaaggacaga cagcacaccc taaagcattt cgagcatgtg 1320
   cgcatggtgg atcccaagaa agccgctcag atccggtccc aggttatgac acacctccgt 1380
   gtgatttatg agcgcatgaa tcagtctctc tccctgctct acaacgtgcc tgcagtggcc 1440
   gaggagattc aggatgaagt tgatgagctg cttcagaaag agcaaaacta ttcagatgac 1500
   gtcttggcca acatgattag tgaaccaagg atcagttacg gaaacgatgc tctcatgcca 1560
   tctttgaccg aaacgaaaac caccgtggag ctccttcccg tgaatggaga gttcagcctg 1620
   gacgatctcc agccgtggca ttcttttggg gctgactctg tgccagccaa cacagaaaac 1680
   gaagttgagc ctgttgatgc ccgccctgct gccgaccgag gactgaccac tcgaccaggt 1740
   tctgggttga caaatatcaa gacggaggag atctctgaag tgaagatgga tgcagaattc 1800
   cgacatgact caggatatga agttcatcat caaaaattgg tgttctttgc agaagatgtg 1860
   ggttcaaaca aaggtgcaat cattggactc atggtgggcg gtgttgtcat agcgacagtg 1920
   atcttcatca ccttggtgat gctgaagaag aaacagtaca catccattca tcatggtgtg 1980
   gtggaggttg acgccgctgt caccccagag gagcgccacc tgtccaagat gcagcagaac 2040
   ggctacgaaa atccaaccta caagttcttt gagcagatgc agaactag            2088
<210> 14
   <211> 695
   <212> PRT
   <213> Homo sapiens
<400> 14
   Met Leu Pro Gly Leu Ala Leu Leu Leu Leu Ala Ala Trp Thr Ala Arg
    1               5                  10                  15
   Ala Leu Glu Val Pro Thr Asp Gly Asn Ala Gly Leu Leu Ala Glu Pro
               20                  25                  30
   Gln Ile Ala Met Phe Cys Gly Arg Leu Asn Met His Met Asn Val Gln
           35                  40                  45
   Asn Gly Lys Trp Asp Ser Asp Pro Ser Gly Thr Lys Thr Cys Ile Asp
       50                  55                  60
   Thr Lys Glu Gly Ile Leu Gln Tyr Cys Gln Glu Val Tyr Pro Glu Leu
   65                  70                  75                  80
   Gln Ile Thr Asn Val Val Glu Ala Asn Gln Pro Val Thr Ile Gln Asn
                   85                  90                  95
   Trp Cys Lys Arg Gly Arg Lys Gln Cys Lys Thr His Pro His Phe Val
               100                 105                 110
   Ile Pro Tyr Arg Cys Leu Val Gly Glu Phe Val Ser Asp Ala Leu Leu
           115                 120                 125
   Val Pro Asp Lys Cys Lys Phe Leu His Gln Glu Arg Met Asp Val Cys
       130                 135                 140
   Glu Thr His Leu His Trp His Thr Val Ala Lys Glu Thr Cys Ser Glu
   145                 150                 155                 160
   Lys Ser Thr Asn Leu His Asp Tyr Gly Met Leu Leu Pro Cys Gly Ile
                   165                 170                 175
   Asp Lys Phe Arg Gly Val Glu Phe Val Cys Cys Pro Leu Ala Glu Glu
               180                 185                 190
   Ser Asp Asn Val Asp Ser Ala Asp Ala Glu Glu Asp Asp Ser Asp Val
           195                 200                 205
   Trp Trp Gly Gly Ala Asp Thr Asp Tyr Ala Asp Gly Ser Glu Asp Lys
       210                 215                 220
   Val Val Glu Val Ala Glu Glu Glu Glu Val Ala Glu Val Glu Glu Glu
   225                 230                 235                 240
   Glu Ala Asp Asp Asp Glu Asp Asp Glu Asp Gly Asp Glu Val Glu Glu
                   245                 250                 255
   Glu Ala Glu Glu Pro Tyr Glu Glu Ala Thr Glu Arg Thr Thr Ser Ile
               260                 265                 270
   Ala Thr Thr Thr Thr Thr Thr Thr Glu Ser Val Glu Glu Val Val Arg
           275                 280                 285
   Val Pro Thr Thr Ala Ala Ser Thr Pro Asp Ala Val Asp Lys Tyr Leu
       290                 295                 300
   Glu Thr Pro Gly Asp Glu Asn Glu His Ala His Phe Gln Lys Ala Lys
   305                 310                 315                 320
   Glu Arg Leu Glu Ala Lys His Arg Glu Arg Met Ser Gln Val Met Arg
                   325                 330                 335
   Glu Trp Glu Glu Ala Glu Arg Gln Ala Lys Asn Leu Pro Lys Ala Asp
               340                 345                 350
   Lys Lys Ala Val Ile Gln His Phe Gln Glu Lys Val Glu Ser Leu Glu
           355                 360                 365
   Gln Glu Ala Ala Asn Glu Arg Gln Gln Leu Val Glu Thr His Met Ala
       370                 375                 380
   Arg Val Glu Ala Met Leu Asn Asp Arg Arg Arg Leu Ala Leu Glu Asn
   385                 390                 395                 400
   Tyr Ile Thr Ala Leu Gln Ala Val Pro Pro Arg Pro Arg His Val Phe
                   405                 410                 415
   Asn Met Leu Lys Lys Tyr Val Arg Ala Glu Gln Lys Asp Arg Gln His
               420                 425                 430
   Thr Leu Lys His Phe Glu His Val Arg Met Val Asp Pro Lys Lys Ala
           435                 440                 445
   Ala Gln Ile Arg Ser Gln Val Met Thr His Leu Arg Val Ile Tyr Glu
       450                 455                 460
   Arg Met Asn Gln Ser Leu Ser Leu Leu Tyr Asn Val Pro Ala Val Ala
   465                 470                 475                 480
   Glu Glu Ile Gln Asp Glu Val Asp Glu Leu Leu Gln Lys Glu Gln Asn
                   485                 490                 495
   Tyr Ser Asp Asp Val Leu Ala Asn Met Ile Ser Glu Pro Arg Ile Ser
               500                 505                 510
   Tyr Gly Asn Asp Ala Leu Met Pro Ser Leu Thr Glu Thr Lys Thr Thr
           515                 520                 525
   Val Glu Leu Leu Pro Val Asn Gly Glu Phe Ser Leu Asp Asp Leu Gln
       530                 535                 540
   Pro Trp His Ser Phe Gly Ala Asp Ser Val Pro Ala Asn Thr Glu Asn
   545 550 555 560
   Glu Val Glu Pro Val Asp Ala Arg Pro Ala Ala Asp Arg Gly Leu Thr
   565 570 575
   Thr Arg Pro Gly Ser Gly Leu Thr Asn Ile Lys Thr Glu Glu Ile Ser
   580 585 590
   Glu Val Lys Met Asp Ala Glu Phe Arg His Asp Ser Gly Tyr Glu Val
   595 600 605
   His His Gln Lys Leu Val Phe Phe Ala Glu Asp Val Gly Ser Asn Lys
   610 615 620
   Gly Ala Ile Ile Gly Leu Met Val Gly Gly Val Val Ile Ala Thr Val
   625 630 635 640
   Ile Phe Ile Thr Leu Val Met Leu Lys Lys Lys Gln Tyr Thr Ser Ile
   645 650 655
   His His Gly Val Val Glu Val Asp Ala Ala Val Thr Pro Glu Glu Arg
   660 665 670
   His Leu Ser Lys Met Gln Gln Asn Gly Tyr Glu Asn Pro Thr Tyr Lys
   675 680 685
   Phe Phe Glu Gln Met Gln Asn
   690 695
<210> 15
   <211> 2094
   <212> DNA
   <213> Homo sapiens
<400> 15
   atgctgcccg gtttggcact gctcctgctg gccgcctgga cggctcgggc gctggaggta 60
   cccactgatg gtaatgctgg cctgctggct gaaccccaga ttgccatgtt ctgtggcaga 120
   ctgaacatgc acatgaatgt ccagaatggg aagtgggatt cagatccatc agggaccaaa 180
   acctgcattg ataccaagga aggcatcctg cagtattgcc aagaagtcta ccctgaactg 240
   cagatcacca atgtggtaga agccaaccaa ccagtgacca tccagaactg gtgcaagcgg 300
   ggccgcaagc agtgcaagac ccatccccac tttgtgattc cctaccgctg cttagttggt 360
   gagtttgtaa gtgatgccct tctcgttcct gacaagtgca aattcttaca ccaggagagg 420
   atggatgttt gcgaaactca tcttcactgg cacaccgtcg ccaaagagac atgcagtgag 480
   aagagtacca acttgcatga ctacggcatg ttgctgccct gcggaattga caagttccga 540
   ggggtagagt ttgtgtgttg cccactggct gaagaaagtg acaatgtgga ttctgctgat 600
   gcggaggagg atgactcgga tgtctggtgg ggcggagcag acacagacta tgcagatggg 660
   agtgaagaca aagtagtaga agtagcagag gaggaagaag tggctgaggt ggaagaagaa 720
   gaagccgatg atgacgagga cgatgaggat ggtgatgagg tagaggaaga ggctgaggaa 780
   ccctacgaag aagccacaga gagaaccacc agcattgcca ccaccaccac caccaccaca 840
   gagtctgtgg aagaggtggt tcgagttcct acaacagcag ccagtacccc tgatgccgtt 900
   gacaagtatc tcgagacacc tggggatgag aatgaacatg cccatttcca gaaagccaaa 960
   gagaggcttg aggccaagca ccgagagaga atgtcccagg tcatgagaga atgggaagag 1020
   gcagaacgtc aagcaaagaa cttgcctaaa gctgataaga aggcagttat ccagcatttc 1080
   caggagaaag tggaatcttt ggaacaggaa gcagccaacg agagacagca gctggtggag 1140
   acacacatgg ccagagtgga agccatgctc aatgaccgcc gccgcctggc cctggagaac 1200
   tacatcaccg ctctgcaggc tgttcctcct cggcctcgtc acgtgttcaa tatgctaaag 1260
   aagtatgtcc gcgcagaaca gaaggacaga cagcacaccc taaagcattt cgagcatgtg 1320
   cgcatggtgg atcccaagaa agccgctcag atccggtccc aggttatgac acacctccgt 1380
   gtgatttatg agcgcatgaa tcagtctctc tccctgctct acaacgtgcc tgcagtggcc 1440
   gaggagattc aggatgaagt tgatgagctg cttcagaaag agcaaaacta ttcagatgac 1500
   gtcttggcca acatgattag tgaaccaagg atcagttacg gaaacgatgc tctcatgcca 1560
   tctttgaccg aaacgaaaac caccgtggag ctccttcccg tgaatggaga gttcagcctg 1620
   gacgatctcc agccgtggca ttcttttggg gctgactctg tgccagccaa cacagaaaac 1680
   gaagttgagc ctgttgatgc ccgccctgct gccgaccgag gactgaccac tcgaccaggt 1740
   tctgggttga caaatatcaa gacggaggag atctctgaag tgaagatgga tgcagaattc 1800
   cgacatgact caggatatga agttcatcat caaaaattgg tgttctttgc agaagatgtg 1860
   ggttcaaaca aaggtgcaat cattggactc atggtgggcg gtgttgtcat agcgacagtg 1920
   atcgtcatca ccttggtgat gctgaagaag aaacagtaca catccattca tcatggtgtg 1980
   gtggaggttg acgccgctgt caccccagag gagcgccacc tgtccaagat gcagcagaac 2040
   ggctacgaaa atccaaccta caagttcttt gagcagatgc agaacaagaa gtag       2094
<210> 16
   <211> 697
   <212> PRT
   <213> Homo sapiens
<400> 16
   Met Leu Pro Gly Leu Ala Leu Leu Leu Leu Ala Ala Trp Thr Ala Arg
    1               5                  10                  15
   Ala Leu Glu Val Pro Thr Asp Gly Asn Ala Gly Leu Leu Ala Glu Pro
               20                  25                  30
   Gln Ile Ala Met Phe Cys Gly Arg Leu Asn Met His Met Asn Val Gln
           35                  40                  45
   Asn Gly Lys Trp Asp Ser Asp Pro Ser Gly Thr Lys Thr Cys Ile Asp
       50                  55                  60
   Thr Lys Glu Gly Ile Leu Gln Tyr Cys Gln Glu Val Tyr Pro Glu Leu
   65                  70                  75                  80
   Gln Ile Thr Asn Val Val Glu Ala Asn Gln Pro Val Thr Ile Gln Asn
                   85                  90                  95
   Trp Cys Lys Arg Gly Arg Lys Gln Cys Lys Thr His Pro His Phe Val
               100                 105                 110
   Ile Pro Tyr Arg Cys Leu Val Gly Glu Phe Val Ser Asp Ala Leu Leu
           115                 120                 125
   Val Pro Asp Lys Cys Lys Phe Leu His Gln Glu Arg Met Asp Val Cys
       130                 135                 140
   Glu Thr His Leu His Trp His Thr Val Ala Lys Glu Thr Cys Ser Glu
   145                 150                 155                 160
   Lys Ser Thr Asn Leu His Asp Tyr Gly Met Leu Leu Pro Cys Gly Ile
                   165                 170                 175
   Asp Lys Phe Arg Gly Val Glu Phe Val Cys Cys Pro Leu Ala Glu Glu
               180                 185                 190
   Ser Asp Asn Val Asp Ser Ala Asp Ala Glu Glu Asp Asp Ser Asp Val
           195                 200                 205
   Trp Trp Gly Gly Ala Asp Thr Asp Tyr Ala Asp Gly Ser Glu Asp Lys
       210                 215                 220
   Val Val Glu Val Ala Glu Glu Glu Glu Val Ala Glu Val Glu Glu Glu
   225                 230                 235                 240
   Glu Ala Asp Asp Asp Glu Asp Asp Glu Asp Gly Asp Glu Val Glu Glu
                   245                 250                 255
   Glu Ala Glu Glu Pro Tyr Glu Glu Ala Thr Glu Arg Thr Thr Ser Ile
               260                 265                 270
   Ala Thr Thr Thr Thr Thr Thr Thr Glu Ser Val Glu Glu Val Val Arg
           275                 280                 285
   Val Pro Thr Thr Ala Ala Ser Thr Pro Asp Ala Val Asp Lys Tyr Leu
       290                 295                 300
   Glu Thr Pro Gly Asp Glu Asn Glu His Ala His Phe Gln Lys Ala Lys
   305                 310                 315                 320
   Glu Arg Leu Glu Ala Lys His Arg Glu Arg Met Ser Gln Val Met Arg
                   325                 330                 335
   Glu Trp Glu Glu Ala Glu Arg Gln Ala Lys Asn Leu Pro Lys Ala Asp
               340                 345                 350
   Lys Lys Ala Val Ile Gln His Phe Gln Glu Lys Val Glu Ser Leu Glu
           355                 360                 365
   Gln Glu Ala Ala Asn Glu Arg Gln Gln Leu Val Glu Thr His Met Ala
       370                 375                 380
   Arg Val Glu Ala Met Leu Asn Asp Arg Arg Arg Leu Ala Leu Glu Asn
   385                 390                 395                 400
   Tyr Ile Thr Ala Leu Gln Ala Val Pro Pro Arg Pro Arg His Val Phe
                   405                 410                 415
   Asn Met Leu Lys Lys Tyr Val Arg Ala Glu Gln Lys Asp Arg Gln His
               420                 425                 430
   Thr Leu Lys His Phe Glu His Val Arg Met Val Asp Pro Lys Lys Ala
           435                 440                 445
   Ala Gln Ile Arg Ser Gln Val Met Thr His Leu Arg Val Ile Tyr Glu
       450                 455                 460
   Arg Met Asn Gln Ser Leu Ser Leu Leu Tyr Asn Val Pro Ala Val Ala
   465                 470                 475                 480
   Glu Glu Ile Gln Asp Glu Val Asp Glu Leu Leu Gln Lys Glu Gln Asn
                   485                 490                 495
   Tyr Ser Asp Asp Val Leu Ala Asn Met Ile Ser Glu Pro Arg Ile Ser
               500                 505                 510
   Tyr Gly Asn Asp Ala Leu Met Pro Ser Leu Thr Glu Thr Lys Thr Thr
           515                 520                 525
   Val Glu Leu Leu Pro Val Asn Gly Glu Phe Ser Leu Asp Asp Leu Gln
       530                 535                 540
   Pro Trp His Ser Phe Gly Ala Asp Ser Val Pro Ala Asn Thr Glu Asn
   545                 550                 555                 560
   Glu Val Glu Pro Val Asp Ala Arg Pro Ala Ala Asp Arg Gly Leu Thr
                   565                 570                 575
   Thr Arg Pro Gly Ser Gly Leu Thr Asn Ile Lys Thr Glu Glu Ile Ser
               580                 585                 590
   Glu Val Lys Met Asp Ala Glu Phe Arg His Asp Ser Gly Tyr Glu Val
           595                 600                 605
   His His Gln Lys Leu Val Phe Phe Ala Glu Asp Val Gly Ser Asn Lys
       610                 615                 620
   Gly Ala Ile Ile Gly Leu Met Val Gly Gly Val Val Ile Ala Thr Val
   625                 630                 635                 640
   Ile Val Ile Thr Leu Val Met Leu Lys Lys Lys Gln Tyr Thr Ser Ile
                   645                 650                 655
   His His Gly Val Val Glu Val Asp Ala Ala Val Thr Pro Glu Glu Arg
               660                 665                 670
   His Leu Ser Lys Met Gln Gln Asn Gly Tyr Glu Asn Pro Thr Tyr Lys
           675                 680                 685
   Phe Phe Glu Gln Met Gln Asn Lys Lys
       690                 695
<210> 17
   <211> 2094
   <212> DNA
   <213> Homo sapiens
<400> 17
   atgctgcccg gtttggcact gctcctgctg gccgcctgga cggctcgggc gctggaggta 60
   cccactgatg gtaatgctgg cctgctggct gaaccccaga ttgccatgtt ctgtggcaga 120
   ctgaacatgc acatgaatgt ccagaatggg aagtgggatt cagatccatc agggaccaaa 180
   acctgcattg ataccaagga aggcatcctg cagtattgcc aagaagtcta ccctgaactg 240
   cagatcacca atgtggtaga agccaaccaa ccagtgacca tccagaactg gtgcaagcgg 300
   ggccgcaagc agtgcaagac ccatccccac tttgtgattc cctaccgctg cttagttggt 360
   gagtttgtaa gtgatgccct tctcgttcct gacaagtgca aattcttaca ccaggagagg 420
   atggatgttt gcgaaactca tcttcactgg cacaccgtcg ccaaagagac atgcagtgag 480
   aagagtacca acttgcatga ctacggcatg ttgctgccct gcggaattga caagttccga 540
   ggggtagagt ttgtgtgttg cccactggct gaagaaagtg acaatgtgga ttctgctgat 600
   gcggaggagg atgactcgga tgtctggtgg ggcggagcag acacagacta tgcagatggg 660
   agtgaagaca aagtagtaga agtagcagag gaggaagaag tggctgaggt ggaagaagaa 720
   gaagccgatg atgacgagga cgatgaggat ggtgatgagg tagaggaaga ggctgaggaa 780
   ccctacgaag aagccacaga gagaaccacc agcattgcca ccaccaccac caccaccaca 840
   gagtctgtgg aagaggtggt tcgagttcct acaacagcag ccagtacccc tgatgccgtt 900
   gacaagtatc tcgagacacc tggggatgag aatgaacatg cccatttcca gaaagccaaa 960
   gagaggcttg aggccaagca ccgagagaga atgtcccagg tcatgagaga atgggaagag 1020
   gcagaacgtc aagcaaagaa cttgcctaaa gctgataaga aggcagttat ccagcatttc 1080
   caggagaaag tggaatcttt ggaacaggaa gcagccaacg agagacagca gctggtggag 1140
   acacacatgg ccagagtgga agccatgctc aatgaccgcc gccgcctggc cctggagaac 1200
   tacatcaccg ctctgcaggc tgttcctcct cggcctcgtc acgtgttcaa tatgctaaag 1260
   aagtatgtcc gcgcagaaca gaaggacaga cagcacaccc taaagcattt cgagcatgtg 1320
   cgcatggtgg atcccaagaa agccgctcag atccggtccc aggttatgac acacctccgt 1380
   gtgatttatg agcgcatgaa tcagtctctc tccctgctct acaacgtgcc tgcagtggcc 1440
   gaggagattc aggatgaagt tgatgagctg cttcagaaag agcaaaacta ttcagatgac 1500
   gtcttggcca acatgattag tgaaccaagg atcagttacg gaaacgatgc tctcatgcca 1560
   tctttgaccg aaacgaaaac caccgtggag ctccttcccg tgaatggaga gttcagcctg 1620
   gacgatctcc agccgtggca ttcttttggg gctgactctg tgccagccaa cacagaaaac 1680
   gaagttgagc ctgttgatgc ccgccctgct gccgaccgag gactgaccac tcgaccaggt 1740
   tctgggttga caaatatcaa gacggaggag atctctgaag tgaatctgga tgcagaattc 1800
   cgacatgact caggatatga agttcatcat caaaaattgg tgttctttgc agaagatgtg 1860
   ggttcaaaca aaggtgcaat cattggactc atggtgggcg gtgttgtcat agcgacagtg 1920
   atcgtcatca ccttggtgat gctgaagaag aaacagtaca catccattca tcatggtgtg 1980
   gtggaggttg acgccgctgt caccccagag gagcgccacc tgtccaagat gcagcagaac 2040
   ggctacgaaa atccaaccta caagttcttt gagcagatgc agaacaagaa gtag       2094
<210> 18
   <211> 697
   <212> PRT
   <213> Homo sapiens
<400> 18
   Met Leu Pro Gly Leu Ala Leu Leu Leu Leu Ala Ala Trp Thr Ala Arg
   1               5                   10                  15
   Ala Leu Glu Val Pro Thr Asp Gly Asn Ala Gly Leu Leu Ala Glu Pro
               20                  25                  30
   Gln Ile Ala Met Phe Cys Gly Arg Leu Asn Met His Met Asn Val Gln
           35                  40                  45
   Asn Gly Lys Trp Asp Ser Asp Pro Ser Gly Thr Lys Thr Cys Ile Asp
       50                  55                  60
   Thr Lys Glu Gly Ile Leu Gln Tyr Cys Gln Glu Val Tyr Pro Glu Leu
   65                  70                  75                  80
   Gln Ile Thr Asn Val Val Glu Ala Asn Gln Pro Val Thr Ile Gln Asn
                   85                  90                  95
   Trp Cys Lys Arg Gly Arg Lys Gln Cys Lys Thr His Pro His Phe Val
               100                 105                 110
   Ile Pro Tyr Arg Cys Leu Val Gly Glu Phe Val Ser Asp Ala Leu Leu
           115                 120                 125
   Val Pro Asp Lys Cys Lys Phe Leu His Gln Glu Arg Met Asp Val Cys
       130                 135                 140
   Glu Thr His Leu His Trp His Thr Val Ala Lys Glu Thr Cys Ser Glu
   145                 150                 155                 160
   Lys Ser Thr Asn Leu His Asp Tyr Gly Met Leu Leu Pro Cys Gly Ile
                   165                 170                 175
   Asp Lys Phe Arg Gly Val Glu Phe Val Cys Cys Pro Leu Ala Glu Glu
               180                 185                 190
   Ser Asp Asn Val Asp Ser Ala Asp Ala Glu Glu Asp Asp Ser Asp Val
           195                 200                 205
   Trp Trp Gly Gly Ala Asp Thr Asp Tyr Ala Asp Gly Ser Glu Asp Lys
       210                 215                 220
   Val Val Glu Val Ala Glu Glu Glu Glu Val Ala Glu Val Glu Glu Glu
   225                 230                 235                 240
   Glu Ala Asp Asp Asp Glu Asp Asp Glu Asp Gly Asp Glu Val Glu Glu
                   245                 250                 255
   Glu Ala Glu Glu Pro Tyr Glu Glu Ala Thr Glu Arg Thr Thr Ser Ile
               260                 265                 270
   Ala Thr Thr Thr Thr Thr Thr Thr Glu Ser Val Glu Glu Val Val Arg
           275                 280                 285
   Val Pro Thr Thr Ala Ala Ser Thr Pro Asp Ala Val Asp Lys Tyr Leu
       290                 295                 300
   Glu Thr Pro Gly Asp Glu Asn Glu His Ala His Phe Gln Lys Ala Lys
   305                 310                 315                 320
   Glu Arg Leu Glu Ala Lys His Arg Glu Arg Met Ser Gln Val Met Arg
                   325                 330                 335
   Glu Trp Glu Glu Ala Glu Arg Gln Ala Lys Asn Leu Pro Lys Ala Asp
               340                 345                 350
   Lys Lys Ala Val Ile Gln His Phe Gln Glu Lys Val Glu Ser Leu Glu
           355                 360                 365
   Gln Glu Ala Ala Asn Glu Arg Gln Gln Leu Val Glu Thr His Met Ala
       370                 375                 380
   Arg Val Glu Ala Met Leu Asn Asp Arg Arg Arg Leu Ala Leu Glu Asn
   385                 390                 395                 400
   Tyr Ile Thr Ala Leu Gln Ala Val Pro Pro Arg Pro Arg His Val Phe
                   405                 410                 415
   Asn Met Leu Lys Lys Tyr Val Arg Ala Glu Gln Lys Asp Arg Gln His
               420                 425                 430
   Thr Leu Lys His Phe Glu His Val Arg Met Val Asp Pro Lys Lys Ala
           435                 440                 445
   Ala Gln Ile Arg Ser Gln Val Met Thr His Leu Arg Val Ile Tyr Glu
       450                 455                 460
   Arg Met Asn Gln Ser Leu Ser Leu Leu Tyr Asn Val Pro Ala Val Ala
   465                 470                 475                 480
   Glu Glu Ile Gln Asp Glu Val Asp Glu Leu Leu Gln Lys Glu Gln Asn
                   485                 490                 495
   Tyr Ser Asp Asp Val Leu Ala Asn Met Ile Ser Glu Pro Arg Ile Ser
               500                 505                 510
   Tyr Gly Asn Asp Ala Leu Met Pro Ser Leu Thr Glu Thr Lys Thr Thr
           515                 520                 525
   Val Glu Leu Leu Pro Val Asn Gly Glu Phe Ser Leu Asp Asp Leu Gln
       530                 535                 540
   Pro Trp His Ser Phe Gly Ala Asp Ser Val Pro Ala Asn Thr Glu Asn
   545                 550                 555                 560
   Glu Val Glu Pro Val Asp Ala Arg Pro Ala Ala Asp Arg Gly Leu Thr
                   565                 570                 575
   Thr Arg Pro Gly Ser Gly Leu Thr Asn Ile Lys Thr Glu Glu Ile Ser
               580                 585                 590
   Glu Val Asn Leu Asp Ala Glu Phe Arg His Asp Ser Gly Tyr Glu Val
           595                 600                 605
   His His Gln Lys Leu Val Phe Phe Ala Glu Asp Val Gly Ser Asn Lys
       610                 615                 620
   Gly Ala Ile Ile Gly Leu Met Val Gly Gly Val Val Ile Ala Thr Val
   625                 630                 635                 640
   Ile Val Ile Thr Leu Val Met Leu Lys Lys Lys Gln Tyr Thr Ser Ile
                   645                 650                 655
   His His Gly Val Val Glu Val Asp Ala Ala Val Thr Pro Glu Glu Arg
               660                 665                 670
   His Leu Ser Lys Met Gln Gln Asn Gly Tyr Glu Asn Pro Thr Tyr Lys
           675                 680                 685
   Phe Phe Glu Gln Met Gln Asn Lys Lys
       690                 695
<210> 19
   <211> 2094
   <212> DNA
   <213> Homo sapiens
<400> 19
   atgctgcccg gtttggcact gctcctgctg gccgcctgga cggctcgggc gctggaggta 60
   cccactgatg gtaatgctgg cctgctggct gaaccccaga ttgccatgtt ctgtggcaga 120
   ctgaacatgc acatgaatgt ccagaatggg aagtgggatt cagatccatc agggaccaaa 180
   acctgcattg ataccaagga aggcatcctg cagtattgcc aagaagtcta ccctgaactg 240
   cagatcacca atgtggtaga agccaaccaa ccagtgacca tccagaactg gtgcaagcgg 300
   ggccgcaagc agtgcaagac ccatccccac tttgtgattc cctaccgctg cttagttggt 360
   gagtttgtaa gtgatgccct tctcgttcct gacaagtgca aattcttaca ccaggagagg 420
   atggatgttt gcgaaactca tcttcactgg cacaccgtcg ccaaagagac atgcagtgag 480
   aagagtacca acttgcatga ctacggcatg ttgctgccct gcggaattga caagttccga 540
   ggggtagagt ttgtgtgttg cccactggct gaagaaagtg acaatgtgga ttctgctgat 600
   gcggaggagg atgactcgga tgtctggtgg ggcggagcag acacagacta tgcagatggg 660
   agtgaagaca aagtagtaga agtagcagag gaggaagaag tggctgaggt ggaagaagaa 720
   gaagccgatg atgacgagga cgatgaggat ggtgatgagg tagaggaaga ggctgaggaa 780
   ccctacgaag aagccacaga gagaaccacc agcattgcca ccaccaccac caccaccaca 840
   gagtctgtgg aagaggtggt tcgagttcct acaacagcag ccagtacccc tgatgccgtt 900
   gacaagtatc tcgagacacc tggggatgag aatgaacatg cccatttcca gaaagccaaa 960
   gagaggcttg aggccaagca ccgagagaga atgtcccagg tcatgagaga atgggaagag 1020
   gcagaacgtc aagcaaagaa cttgcctaaa gctgataaga aggcagttat ccagcatttc 1080
   caggagaaag tggaatcttt ggaacaggaa gcagccaacg agagacagca gctggtggag 1140
   acacacatgg ccagagtgga agccatgctc aatgaccgcc gccgcctggc cctggagaac 1200
   tacatcaccg ctctgcaggc tgttcctcct cggcctcgtc acgtgttcaa tatgctaaag 1260
   aagtatgtcc gcgcagaaca gaaggacaga cagcacaccc taaagcattt cgagcatgtg 1320
   cgcatggtgg atcccaagaa agccgctcag atccggtccc aggttatgac acacctccgt 1380
   gtgatttatg agcgcatgaa tcagtctctc tccctgctct acaacgtgcc tgcagtggcc 1440
   gaggagattc aggatgaagt tgatgagctg cttcagaaag agcaaaacta ttcagatgac 1500
   gtcttggcca acatgattag tgaaccaagg atcagttacg gaaacgatgc tctcatgcca 1560
   tctttgaccg aaacgaaaac caccgtggag ctccttcccg tgaatggaga gttcagcctg 1620
   gacgatctcc agccgtggca ttcttttggg gctgactctg tgccagccaa cacagaaaac 1680
   gaagttgagc ctgttgatgc ccgccctgct gccgaccgag gactgaccac tcgaccaggt 1740
   tctgggttga caaatatcaa gacggaggag atctctgaag tgaagatgga tgcagaattc 1800
   cgacatgact caggatatga agttcatcat caaaaattgg tgttctttgc agaagatgtg 1860
   ggttcaaaca aaggtgcaat cattggactc atggtgggcg gtgttgtcat agcgacagtg 1920
   atcttcatca ccttggtgat gctgaagaag aaacagtaca catccattca tcatggtgtg 1980
   gtggaggttg acgccgctgt caccccagag gagcgccacc tgtccaagat gcagcagaac 2040
   ggctacgaaa atccaaccta caagttcttt gagcagatgc agaacaagaa gtag       2094
<210> 20
   <211> 697
   <212> PRT
   <213> Homo sapiens
<400> 20
   Met Leu Pro Gly Leu Ala Leu Leu Leu Leu Ala Ala Trp Thr Ala Arg
    1               5                  10                  15
   Ala Leu Glu Val Pro Thr Asp Gly Asn Ala Gly Leu Leu Ala Glu Pro
               20                  25                  30
   Gln Ile Ala Met Phe Cys Gly Arg Leu Asn Met His Met Asn Val Gln
           35                  40                  45
   Asn Gly Lys Trp Asp Ser Asp Pro Ser Gly Thr Lys Thr Cys Ile Asp
       50                  55                  60
   Thr Lys Glu Gly Ile Leu Gln Tyr Cys Gln Glu Val Tyr Pro Glu Leu
   65                  70                  75                  80
   Gln Ile Thr Asn Val Val Glu Ala Asn Gln Pro Val Thr Ile Gln Asn
                   85                  90                  95
   Trp Cys Lys Arg Gly Arg Lys Gln Cys Lys Thr His Pro His Phe Val
               100                 105                 110
   Ile Pro Tyr Arg Cys Leu Val Gly Glu Phe Val Ser Asp Ala Leu Leu
           115                 120                 125
   Val Pro Asp Lys Cys Lys Phe Leu His Gln Glu Arg Met Asp Val Cys
       130                 135                 140
   Glu Thr His Leu His Trp His Thr Val Ala Lys Glu Thr Cys Ser Glu
   145                 150                 155                 160
   Lys Ser Thr Asn Leu His Asp Tyr Gly Met Leu Leu Pro Cys Gly Ile
                   165                 170                 175
   Asp Lys Phe Arg Gly Val Glu Phe Val Cys Cys Pro Leu Ala Glu Glu
               180                 185                 190
   Ser Asp Asn Val Asp Ser Ala Asp Ala Glu Glu Asp Asp Ser Asp Val
           195                 200                 205
   Trp Trp Gly Gly Ala Asp Thr Asp Tyr Ala Asp Gly Ser Glu Asp Lys
       210                 215                 220
   Val Val Glu Val Ala Glu Glu Glu Glu Val Ala Glu Val Glu Glu Glu
   225                 230                 235                 240
   Glu Ala Asp Asp Asp Glu Asp Asp Glu Asp Gly Asp Glu Val Glu Glu
                   245                 250                 255
   Glu Ala Glu Glu Pro Tyr Glu Glu Ala Thr Glu Arg Thr Thr Ser Ile
               260                 265                 270
   Ala Thr Thr Thr Thr Thr Thr Thr Glu Ser Val Glu Glu Val Val Arg
           275                 280                 285
   Val Pro Thr Thr Ala Ala Ser Thr Pro Asp Ala Val Asp Lys Tyr Leu
       290                 295                 300
   Glu Thr Pro Gly Asp Glu Asn Glu His Ala His Phe Gln Lys Ala Lys
   305                 310                 315                 320
   Glu Arg Leu Glu Ala Lys His Arg Glu Arg Met Ser Gln Val Met Arg
                   325                 330                 335
   Glu Trp Glu Glu Ala Glu Arg Gln Ala Lys Asn Leu Pro Lys Ala Asp
               340                 345                 350
   Lys Lys Ala Val Ile Gln His Phe Gln Glu Lys Val Glu Ser Leu Glu
           355                 360                 365
   Gln Glu Ala Ala Asn Glu Arg Gln Gln Leu Val Glu Thr His Met Ala
       370                 375                 380
   Arg Val Glu Ala Met Leu Asn Asp Arg Arg Arg Leu Ala Leu Glu Asn
   385                 390                 395                 400
   Tyr Ile Thr Ala Leu Gln Ala Val Pro Pro Arg Pro Arg His Val Phe
                   405                 410                 415
   Asn Met Leu Lys Lys Tyr Val Arg Ala Glu Gln Lys Asp Arg Gln His
               420                 425                 430
   Thr Leu Lys His Phe Glu His Val Arg Met Val Asp Pro Lys Lys Ala
           435                 440                 445
   Ala Gln Ile Arg Ser Gln Val Met Thr His Leu Arg Val Ile Tyr Glu
       450                 455                 460
   Arg Met Asn Gln Ser Leu Ser Leu Leu Tyr Asn Val Pro Ala Val Ala
   465                 470                 475                 480
   Glu Glu Ile Gln Asp Glu Val Asp Glu Leu Leu Gln Lys Glu Gln Asn
                   485                 490                 495
   Tyr Ser Asp Asp Val Leu Ala Asn Met Ile Ser Glu Pro Arg Ile Ser
               500                 505                 510
   Tyr Gly Asn Asp Ala Leu Met Pro Ser Leu Thr Glu Thr Lys Thr Thr
           515                 520                 525
   Val Glu Leu Leu Pro Val Asn Gly Glu Phe Ser Leu Asp Asp Leu Gln
       530                 535                 540
   Pro Trp His Ser Phe Gly Ala Asp Ser Val Pro Ala Asn Thr Glu Asn
   545                 550                 555                 560
   Glu Val Glu Pro Val Asp Ala Arg Pro Ala Ala Asp Arg Gly Leu Thr
                   565                 570                 575
   Thr Arg Pro Gly Ser Gly Leu Thr Asn Ile Lys Thr Glu Glu Ile Ser
               580                 585                 590
   Glu Val Lys Met Asp Ala Glu Phe Arg His Asp Ser Gly Tyr Glu Val
           595                 600                 605
   His His Gln Lys Leu Val Phe Phe Ala Glu Asp Val Gly Ser Asn Lys
       610                 615                 620
   Gly Ala Ile Ile Gly Leu Met Val Gly Gly Val Val Ile Ala Thr Val
   625                 630                 635                 640
   Ile Phe Ile Thr Leu Val Met Leu Lys Lys Lys Gln Tyr Thr Ser Ile
                   645                 650                 655
   His His Gly Val Val Glu Val Asp Ala Ala Val Thr Pro Glu Glu Arg
               660                 665                 670
   His Leu Ser Lys Met Gln Gln Asn Gly Tyr Glu Asn Pro Thr Tyr Lys
           675                 680                 685
   Phe Phe Glu Gln Met Gln Asn Lys Lys
       690                 695
<210> 21
   <211> 1341
   <212> DNA
   <213> Homo sapiens
<400> 21
   atggctagca tgactggtgg acagcaaatg ggtcgcggat ccacccagca cggcatccgg 60
   ctgcccctgc gcagcggcct ggggggcgcc cccctggggc tgcggctgcc ccgggagacc 120
   gacgaagagc ccgaggagcc cggccggagg ggcagctttg tggagatggt ggacaacctg 180
   aggggcaagt cggggcaggg ctactacgtg gagatgaccg tgggcagccc cccgcagacg 240
   ctcaacatcc tggtggatac aggcagcagt aactttgcag tgggtgctgc cccccacccc 300
   ttcctgcatc gctactacca gaggcagctg tccagcacat accgggacct ccggaagggt 360
   gtgtatgtgc cctacaccca gggcaagtgg gaaggggagc tgggcaccga cctggtaagc 420
   atcccccatg gccccaacgt cactgtgcgt gccaacattg ctgccatcac tgaatcagac 480
   aagttcttca tcaacggctc caactgggaa ggcatcctgg ggctggccta tgctgagatt 540
   gccaggcctg acgactccct ggagcctttc tttgactctc tggtaaagca gacccacgtt 600
   cccaacctct tctccctgca cctttgtggt gctggcttcc ccctcaacca gtctgaagtg 660
   ctggcctctg tcggagggag catgatcatt ggaggtatcg accactcgct gtacacaggc 720
   agtctctggt atacacccat ccggcgggag tggtattatg aggtcatcat tgtgcgggtg 780
   gagatcaatg gacaggatct gaaaatggac tgcaaggagt acaactatga caagagcatt 840
   gtggacagtg gcaccaccaa ccttcgtttg cccaagaaag tgtttgaagc tgcagtcaaa 900
   tccatcaagg cagcctcctc cacggagaag ttccctgatg gtttctggct aggagagcag 960
   ctggtgtgct ggcaagcagg caccacccct tggaacattt tcccagtcat ctcactctac 1020
   ctaatgggtg aggttaccaa ccagtccttc cgcatcacca tccttccgca gcaatacctg 1080
   cggccagtgg aagatgtggc cacgtcccaa gacgactgtt acaagtttgc catctcacag 1140
   tcatccacgg gcactgttat gggagctgtt atcatggagg gcttctacgt tgtctttgat 1200
   cgggcccgaa aacgaattgg ctttgctgtc agcgcttgcc atgtgcacga tgagttcagg 1260
   acggcagcgg tggaaggccc ttttgtcacc ttggacatgg aagactgtgg ctacaacatt 1320
   ccacagacag atgagtcatg a                                           1341
<210> 22
   <211> 446
   <212> PRT
   <213> Homo sapiens
<400> 22
   Met Ala Ser Met Thr Gly Gly Gln Gln Met Gly Arg Gly Ser Thr Gln
    1               5                  10                  15
   His Gly Ile Arg Leu Pro Leu Arg Ser Gly Leu Gly Gly Ala Pro Leu
               20                  25                  30
   Gly Leu Arg Leu Pro Arg Glu Thr Asp Glu Glu Pro Glu Glu Pro Gly
           35                  40                  45
   Arg Arg Gly Ser Phe Val Glu Met Val Asp Asn Leu Arg Gly Lys Ser
       50                  55                  60
   Gly Gln Gly Tyr Tyr Val Glu Met Thr Val Gly Ser Pro Pro Gln Thr
    65                 70                  75                  80
   Leu Asn Ile Leu Val Asp Thr Gly Ser Ser Asn Phe Ala Val Gly Ala
                   85                  90                  95
   Ala Pro His Pro Phe Leu His Arg Tyr Tyr Gln Arg Gln Leu Ser Ser
               100                 105                 110
   Thr Tyr Arg Asp Leu Arg Lys Gly Val Tyr Val Pro Tyr Thr Gln Gly
           115                 120                 125
   Lys Trp Glu Gly Glu Leu Gly Thr Asp Leu Val Ser Ile Pro His Gly
       130                 135                 140
   Pro Asn Val Thr Val Arg Ala Asn Ile Ala Ala Ile Thr Glu Ser Asp
   145                 150                 155                 160
   Lys Phe Phe Ile Asn Gly Ser Asn Trp Glu Gly Ile Leu Gly Leu Ala
                   165                 170                 175
   Tyr Ala Glu Ile Ala Arg Pro Asp Asp Ser Leu Glu Pro Phe Phe Asp
               180                 185                 190
   Ser Leu Val Lys Gln Thr His Val Pro Asn Leu Phe Ser Leu His Leu
           195                 200                 205
   Cys Gly Ala Gly Phe Pro Leu Asn Gln Ser Glu Val Leu Ala Ser Val
       210                 215                 220
   Gly Gly Ser Met Ile Ile Gly Gly Ile Asp His Ser Leu Tyr Thr Gly
   225                 230                 235                 240
   Ser Leu Trp Tyr Thr Pro Ile Arg Arg Glu Trp Tyr Tyr Glu Val Ile
                   245                 250                 255
   Ile Val Arg Val Glu Ile Asn Gly Gln Asp Leu Lys Met Asp Cys Lys
               260                 265                 270
   Glu Tyr Asn Tyr Asp Lys Ser Ile Val Asp Ser Gly Thr Thr Asn Leu
           275                 280                 285
   Arg Leu Pro Lys Lys Val Phe Glu Ala Ala Val Lys Ser Ile Lys Ala
       290                 295                 300
   Ala Ser Ser Thr Glu Lys Phe Pro Asp Gly Phe Trp Leu Gly Glu Gln
   305                 310                 315                 320
   Leu Val Cys Trp Gln Ala Gly Thr Thr Pro Trp Asn Ile Phe Pro Val
                   325                 330                 335
   Ile Ser Leu Tyr Leu Met Gly Glu Val Thr Asn Gln Ser Phe Arg Ile
               340                 345                 350
   Thr Ile Leu Pro Gln Gln Tyr Leu Arg Pro Val Glu Asp Val Ala Thr
           355                 360                 365
   Ser Gln Asp Asp Cys Tyr Lys Phe Ala Ile Ser Gln Ser Ser Thr Gly
       370                 375                 380
   Thr Val Met Gly Ala Val Ile Met Glu Gly Phe Tyr Val Val Phe Asp
   385                 390                 395                 400
   Arg Ala Arg Lys Arg Ile Gly Phe Ala Val Ser Ala Cys His Val His
                   405                 410                 415
   Asp Glu Phe Arg Thr Ala Ala Val Glu Gly Pro Phe Val Thr Leu Asp
               420                 425                 430
   Met Glu Asp Cys Gly Tyr Asn Ile Pro Gln Thr Asp Glu Ser
           435                 440                 445
<210> 23
   <211> 1380
   <212> DNA
   <213> Homo sapiens
<400> 23
   atggctagca tgactggtgg acagcaaatg ggtcgcggat cgatgactat ctctgactct 60
   ccgcgtgaac aggacggatc cacccagcac ggcatccggc tgcccctgcg cagcggcctg 120
   gggggcgccc ccctggggct gcggctgccc cgggagaccg acgaagagcc cgaggagccc 180
   ggccggaggg gcagctttgt ggagatggtg gacaacctga ggggcaagtc ggggcagggc 240
   tactacgtgg agatgaccgt gggcagcccc ccgcagacgc tcaacatcct ggtggataca 300
   ggcagcagta actttgcagt gggtgctgcc ccccacccct tcctgcatcg ctactaccag 360
   aggcagctgt ccagcacata ccgggacctc cggaagggtg tgtatgtgcc ctacacccag 420
   ggcaagtggg aaggggagct gggcaccgac ctggtaagca tcccccatgg ccccaacgtc 480
   actgtgcgtg ccaacattgc tgccatcact gaatcagaca agttcttcat caacggctcc 540
   aactgggaag gcatcctggg gctggcctat gctgagattg ccaggcctga cgactccctg 600
   gagcctttct ttgactctct ggtaaagcag acccacgttc ccaacctctt ctccctgcac 660
   ctttgtggtg ctggcttccc cctcaaccag tctgaagtgc tggcctctgt cggagggagc 720
   atgatcattg gaggtatcga ccactcgctg tacacaggca gtctctggta tacacccatc 780
   cggcgggagt ggtattatga ggtcatcatt gtgcgggtgg agatcaatgg acaggatctg 840
   aaaatggact gcaaggagta caactatgac aagagcattg tggacagtgg caccaccaac 900
   cttcgtttgc ccaagaaagt gtttgaagct gcagtcaaat ccatcaaggc agcctcctcc 960
   acggagaagt tccctgatgg tttctggcta ggagagcagc tggtgtgctg gcaagcaggc 1020
   accacccctt ggaacatttt cccagtcatc tcactctacc taatgggtga ggttaccaac 1080
   cagtccttcc gcatcaccat ccttccgcag caatacctgc ggccagtgga agatgtggcc 1140
   acgtcccaag acgactgtta caagtttgcc atctcacagt catccacggg cactgttatg 1200
   ggagctgtta tcatggaggg cttctacgtt gtctttgatc gggcccgaaa acgaattggc 1260
   tttgctgtca gcgcttgcca tgtgcacgat gagttcagga cggcagcggt ggaaggccct 1320
   tttgtcacct tggacatgga agactgtggc tacaacattc cacagacaga tgagtcatga 1380
<210> 24
   <211> 459
   <212> PRT
   <213> Homo sapiens
<400> 24
   Met Ala Ser Met Thr Gly Gly Gln Gln Met Gly Arg Gly Ser Met Thr
    1               5                  10                  15
   Ile Ser Asp Ser Pro Arg Glu Gln Asp Gly Ser Thr Gln His Gly Ile
               20                  25                  30
   Arg Leu Pro Leu Arg Ser Gly Leu Gly Gly Ala Pro Leu Gly Leu Arg
           35                  40                  45
   Leu Pro Arg Glu Thr Asp Glu Glu Pro Glu Glu Pro Gly Arg Arg Gly
       50                  55                  60
   Ser Phe Val Glu Met Val Asp Asn Leu Arg Gly Lys Ser Gly Gln Gly
   65                  70                  75                  80
   Tyr Tyr Val Glu Met Thr Val Gly Ser Pro Pro Gln Thr Leu Asn Ile
                   85                  90                  95
   Leu Val Asp Thr Gly Ser Ser Asn Phe Ala Val Gly Ala Ala Pro His
   100 105 110
   Pro Phe Leu His Arg Tyr Tyr Gln Arg Gln Leu Ser Ser Thr Tyr Arg
   115 120 125
   Asp Leu Arg Lys Gly Val Tyr Val Pro Tyr Thr Gln Gly Lys Trp Glu
   130 135 140
   Gly Glu Leu Gly Thr Asp Leu Val Ser Ile Pro His Gly Pro Asn Val
   145 150 155 160
   Thr Val Arg Ala Asn Ile Ala Ala Ile Thr Glu Ser Asp Lys Phe Phe
   165 170 175
   Ile Asn Gly Ser Asn Trp Glu Gly Ile Leu Gly Leu Ala Tyr Ala Glu
   180 185 190
   Ile Ala Arg Pro Asp Asp Ser Leu Glu Pro Phe Phe Asp Ser Leu Val
   195 200 205
   Lys Gln Thr His Val Pro Asn Leu Phe Ser Leu His Leu Cys Gly Ala
   210 215 220
   Gly Phe Pro Leu Asn Gln Ser Glu Val Leu Ala Ser Val Gly Gly Ser
   225 230 235 240
   Met Ile Ile Gly Gly Ile Asp His Ser Leu Tyr Thr Gly Ser Leu Trp
   245 250 255
   Tyr Thr Pro Ile Arg Arg Glu Trp Tyr Tyr Glu Val Ile Ile Val Arg
   260 265 270
   Val Glu Ile Asn Gly Gln Asp Leu Lys Met Asp Cys Lys Glu Tyr Asn
   275 280 285
   Tyr Asp Lys Ser Ile Val Asp Ser Gly Thr Thr Asn Leu Arg Leu Pro
   290 295 300
   Lys Lys Val Phe Glu Ala Ala Val Lys Ser Ile Lys Ala Ala Ser Ser
   305 310 315 320
   Thr Glu Lys Phe Pro Asp Gly Phe Trp Leu Gly Glu Gln Leu Val Cys
   325 330 335
   Trp Gln Ala Gly Thr Thr Pro Trp Asn Ile Phe Pro Val Ile Ser Leu
   340 345 350
   Tyr Leu Met Gly Glu Val Thr Asn Gln Ser Phe Arg Ile Thr Ile Leu
   355 360 365
   Pro Gln Gln Tyr Leu Arg Pro Val Glu Asp Val Ala Thr Ser Gln Asp
   370 375 380
   Asp Cys Tyr Lys Phe Ala Ile Ser Gln Ser Ser Thr Gly Thr Val Met
   385 390 395 400
   Gly Ala Val Ile Met Glu Gly Phe Tyr Val Val Phe Asp Arg Ala Arg
   405 410 415
   Lys Arg Ile Gly Phe Ala Val Ser Ala Cys His Val His Asp Glu Phe
   420 425 430
   Arg Thr Ala Ala Val Glu Gly Pro Phe Val Thr Leu Asp Met Glu Asp
           435                 440                 445
   Cys Gly Tyr Asn Ile Pro Gln Thr Asp Glu Ser
       450                 455
<210> 25
   <211> 1302
   <212> DNA
   <213> Homo sapiens
<400> 25
   atgactcagc atggtattcg tctgccactg cgtagcggtc tgggtggtgc tccactgggt 60
   ctgcgtctgc cccgggagac cgacgaagag cccgaggagc ccggccggag gggcagcttt 120
   gtggagatgg tggacaacct gaggggcaag tcggggcagg gctactacgt ggagatgacc 180
   gtgggcagcc ccccgcagac gctcaacatc ctggtggata caggcagcag taactttgca 240
   gtgggtgctg ccccccaccc cttcctgcat cgctactacc agaggcagct gtccagcaca 300
   taccgggacc tccggaaggg tgtgtatgtg ccctacaccc agggcaagtg ggaaggggag 360
   ctgggcaccg acctggtaag catcccccat ggccccaacg tcactgtgcg tgccaacatt 420
   gctgccatca ctgaatcaga caagttcttc atcaacggct ccaactggga aggcatcctg 480
   gggctggcct atgctgagat tgccaggcct gacgactccc tggagccttt ctttgactct 540
   ctggtaaagc agacccacgt tcccaacctc ttctccctgc acctttgtgg tgctggcttc 600
   cccctcaacc agtctgaagt gctggcctct gtcggaggga gcatgatcat tggaggtatc 660
   gaccactcgc tgtacacagg cagtctctgg tatacaccca tccggcggga gtggtattat 720
   gaggtcatca ttgtgcgggt ggagatcaat ggacaggatc tgaaaatgga ctgcaaggag 780
   tacaactatg acaagagcat tgtggacagt ggcaccacca accttcgttt gcccaagaaa 840
   gtgtttgaag ctgcagtcaa atccatcaag gcagcctcct ccacggagaa gttccctgat 900
   ggtttctggc taggagagca gctggtgtgc tggcaagcag gcaccacccc ttggaacatt 960
   ttcccagtca tctcactcta cctaatgggt gaggttacca accagtcctt ccgcatcacc 1020
   atccttccgc agcaatacct gcggccagtg gaagatgtgg ccacgtccca agacgactgt 1080
   tacaagtttg ccatctcaca gtcatccacg ggcactgtta tgggagctgt tatcatggag 1140
   ggcttctacg ttgtctttga tcgggcccga aaacgaattg gctttgctgt cagcgcttgc 1200
   catgtgcacg atgagttcag gacggcagcg gtggaaggcc cttttgtcac cttggacatg 1260
   gaagactgtg gctacaacat tccacagaca gatgagtcat ga                    1302
<210> 26
   <211> 433
   <212> PRT
   <213> Homo sapiens
<400> 26
   Met Thr Gln His Gly Ile Arg Leu Pro Leu Arg Ser Gly Leu Gly Gly
    1               5                  10                  15
   Ala Pro Leu Gly Leu Arg Leu Pro Arg Glu Thr Asp Glu Glu Pro Glu
               20                  25                  30
   Glu Pro Gly Arg Arg Gly Ser Phe Val Glu Met Val Asp Asn Leu Arg
           35                  40                  45
   Gly Lys Ser Gly Gln Gly Tyr Tyr Val Glu Met Thr Val Gly Ser Pro
       50                  55                  60
   Pro Gln Thr Leu Asn Ile Leu Val Asp Thr Gly Ser Ser Asn Phe Ala
   65                  70                  75                  80
   Val Gly Ala Ala Pro His Pro Phe Leu His Arg Tyr Tyr Gln Arg Gln
                   85                  90                  95
   Leu Ser Ser Thr Tyr Arg Asp Leu Arg Lys Gly Val Tyr Val Pro Tyr
               100                 105                 110
   Thr Gln Gly Lys Trp Glu Gly Glu Leu Gly Thr Asp Leu Val Ser Ile
   115 120 125
   Pro His Gly Pro Asn Val Thr Val Arg Ala Asn Ile Ala Ala Ile Thr
   130 135 140
   Glu Ser Asp Lys Phe Phe Ile Asn Gly Ser Asn Trp Glu Gly Ile Leu
   145 150 155 160
   Gly Leu Ala Tyr Ala Glu Ile Ala Arg Pro Asp Asp Ser Leu Glu Pro
   165 170 175
   Phe Phe Asp Ser Leu Val Lys Gln Thr His Val Pro Asn Leu Phe Ser
   180 185 190
   Leu His Leu Cys Gly Ala Gly Phe Pro Leu Asn Gln Ser Glu Val Leu
   195 200 205
   Ala Ser Val Gly Gly Ser Met Ile Ile Gly Gly Ile Asp His Ser Leu
   210 215 220
   Tyr Thr Gly Ser Leu Trp Tyr Thr Pro Ile Arg Arg Glu Trp Tyr Tyr
   225 230 235 240
   Glu Val Ile Ile Val Arg Val Glu Ile Asn Gly Gln Asp Leu Lys Met
   245 250 255
   Asp Cys Lys Glu Tyr Asn Tyr Asp Lys Ser Ile Val Asp Ser Gly Thr
   260 265 270
   Thr Asn Leu Arg Leu Pro Lys Lys Val Phe Glu Ala Ala Val Lys Ser
   275 280 285
   Ile Lys Ala Ala Ser Ser Thr Glu Lys Phe Pro Asp Gly Phe Trp Leu
   290 295 300
   Gly Glu Gln Leu Val Cys Trp Gln Ala Gly Thr Thr Pro Trp Asn Ile
   305 310 315 320
   Phe Pro Val Ile Ser Leu Tyr Leu Met Gly Glu Val Thr Asn Gln Ser
   325 330 335
   Phe Arg Ile Thr Ile Leu Pro Gln Gln Tyr Leu Arg Pro Val Glu Asp
   340 345 350
   Val Ala Thr Ser Gln Asp Asp Cys Tyr Lys Phe Ala Ile Ser Gln Ser
   355 360 365
   Ser Thr Gly Thr Val Met Gly Ala Val Ile Met Glu Gly Phe Tyr Val
   370 375 380
   Val Phe Asp Arg Ala Arg Lys Arg Ile Gly Phe Ala Val Ser Ala Cys
   385 390 395 400
   His Val His Asp Glu Phe Arg Thr Ala Ala Val Glu Gly Pro Phe Val
   405 410 415
   Thr Leu Asp Met Glu Asp Cys Gly Tyr Asn Ile Pro Gln Thr Asp Glu
   420 425 430
   Ser
<210> 27
   <211> 1278
   <212> DNA
   <213> Homo sapiens
<400> 27
   atggctagca tgactggtgg acagcaaatg ggtcgcggat cgatgactat ctctgactct 60
   ccgctggact ctggtatcga aaccgacgga tcctttgtgg agatggtgga caacctgagg 120
   ggcaagtcgg ggcagggcta ctacgtggag atgaccgtgg gcagcccccc gcagacgctc 180
   aacatcctgg tggatacagg cagcagtaac tttgcagtgg gtgctgcccc ccaccccttc 240
   ctgcatcgct actaccagag gcagctgtcc agcacatacc gggacctccg gaagggtgtg 300
   tatgtgccct acacccaggg caagtgggaa ggggagctgg gcaccgacct ggtaagcatc 360
   ccccatggcc ccaacgtcac tgtgcgtgcc aacattgctg ccatcactga atcagacaag 420
   ttcttcatca acggctccaa ctgggaaggc atcctggggc tggcctatgc tgagattgcc 480
   aggcctgacg actccctgga gcctttcttt gactctctgg taaagcagac ccacgttccc 540
   aacctcttct ccctgcacct ttgtggtgct ggcttccccc tcaaccagtc tgaagtgctg 600
   gcctctgtcg gagggagcat gatcattgga ggtatcgacc actcgctgta cacaggcagt 660
   ctctggtata cacccatccg gcgggagtgg tattatgagg tcatcattgt gcgggtggag 720
   atcaatggac aggatctgaa aatggactgc aaggagtaca actatgacaa gagcattgtg 780
   gacagtggca ccaccaacct tcgtttgccc aagaaagtgt ttgaagctgc agtcaaatcc 840
   atcaaggcag cctcctccac ggagaagttc cctgatggtt tctggctagg agagcagctg 900
   gtgtgctggc aagcaggcac caccccttgg aacattttcc cagtcatctc actctaccta 960
   atgggtgagg ttaccaacca gtccttccgc atcaccatcc ttccgcagca atacctgcgg 1020
   ccagtggaag atgtggccac gtcccaagac gactgttaca agtttgccat ctcacagtca 1080
   tccacgggca ctgttatggg agctgttatc atggagggct tctacgttgt ctttgatcgg 1140
   gcccgaaaac gaattggctt tgctgtcagc gcttgccatg tgcacgatga gttcaggacg 1200
   gcagcggtgg aaggcccttt tgtcaccttg gacatggaag actgtggcta caacattcca 1260
   cagacagatg agtcatga                                                1278
<210> 28
   <211> 425
   <212> PRT
   <213> Homo sapiens
<400> 28
   Met Ala Ser Met Thr Gly Gly Gln Gln Met Gly Arg Gly Ser Met Thr
    1               5                  10                  15
   Ile Ser Asp Ser Pro Leu Asp Ser Gly Ile Glu Thr Asp Gly Ser Phe
               20                  25                  30
   Val Glu Met Val Asp Asn Leu Arg Gly Lys Ser Gly Gln Gly Tyr Tyr
           35                  40                  45
   Val Glu Met Thr Val Gly Ser Pro Pro Gln Thr Leu Asn Ile Leu Val
       50                  55                  60
   Asp Thr Gly Ser Ser Asn Phe Ala Val Gly Ala Ala Pro His Pro Phe
   65                  70                  75                  80
   Leu His Arg Tyr Tyr Gln Arg Gln Leu Ser Ser Thr Tyr Arg Asp Leu
                   85                  90                  95
   Arg Lys Gly Val Tyr Val Pro Tyr Thr Gln Gly Lys Trp Glu Gly Glu
               100                 105                 110
   Leu Gly Thr Asp Leu Val Ser Ile Pro His Gly Pro Asn Val Thr Val
           115                 120                 125
   Arg Ala Asn Ile Ala Ala Ile Thr Glu Ser Asp Lys Phe Phe Ile Asn
       130                 135                 140
   Gly Ser Asn Trp Glu Gly Ile Leu Gly Leu Ala Tyr Ala Glu Ile Ala
   145                 150                 155                 160
   Arg Pro Asp Asp Ser Leu Glu Pro Phe Phe Asp Ser Leu Val Lys Gln
                   165                 170                 175
   Thr His Val Pro Asn Leu Phe Ser Leu His Leu Cys Gly Ala Gly Phe
               180                 185                 190
   Pro Leu Asn Gln Ser Glu Val Leu Ala Ser Val Gly Gly Ser Met Ile
           195                 200                 205
   Ile Gly Gly Ile Asp His Ser Leu Tyr Thr Gly Ser Leu Trp Tyr Thr
       210                 215                 220
   Pro Ile Arg Arg Glu Trp Tyr Tyr Glu Val Ile Ile Val Arg Val Glu
   225                 230                 235                 240
   Ile Asn Gly Gln Asp Leu Lys Met Asp Cys Lys Glu Tyr Asn Tyr Asp
                   245                 250                 255
   Lys Ser Ile Val Asp Ser Gly Thr Thr Asn Leu Arg Leu Pro Lys Lys
               260                 265                 270
   Val Phe Glu Ala Ala Val Lys Ser Ile Lys Ala Ala Ser Ser Thr Glu
           275                 280                 285
   Lys Phe Pro Asp Gly Phe Trp Leu Gly Glu Gln Leu Val Cys Trp Gln
       290                 295                 300
   Ala Gly Thr Thr Pro Trp Asn Ile Phe Pro Val Ile Ser Leu Tyr Leu
   305                 310                 315                 320
   Met Gly Glu Val Thr Asn Gln Ser Phe Arg Ile Thr Ile Leu Pro Gln
                   325                 330                 335
   Gln Tyr Leu Arg Pro Val Glu Asp Val Ala Thr Ser Gln Asp Asp Cys
               340                 345                 350
   Tyr Lys Phe Ala Ile Ser Gln Ser Ser Thr Gly Thr Val Met Gly Ala
           355                 360                 365
   Val Ile Met Glu Gly Phe Tyr Val Val Phe Asp Arg Ala Arg Lys Arg
       370                 375                 380
   Ile Gly Phe Ala Val Ser Ala Cys His Val His Asp Glu Phe Arg Thr
   385                 390                 395                 400
   Ala Ala Val Glu Gly Pro Phe Val Thr Leu Asp Met Glu Asp Cys Gly
                   405                 410                 415
   Tyr Asn Ile Pro Gln Thr Asp Glu Ser
               420                 425
<210> 29
   <211> 1362
   <212> DNA
   <213> Homo sapiens
<400> 29
   atggcccaag ccctgccctg gctcctgctg tggatgggcg cgggagtgct gcctgcccac 60
   ggcacccagc acggcatccg gctgcccctg cgcagcggcc tggggggcgc ccccctgggg 120
   ctgcggctgc cccgggagac cgacgaagag cccgaggagc ccggccggag gggcagcttt 180
   gtggagatgg tggacaacct gaggggcaag tcggggcagg gctactacgt ggagatgacc 240
   gtgggcagcc ccccgcagac gctcaacatc ctggtggata caggcagcag taactttgca 300
   gtgggtgctg ccccccaccc cttcctgcat cgctactacc agaggcagct gtccagcaca 360
   taccgggacc tccggaaggg tgtgtatgtg ccctacaccc agggcaagtg ggaaggggag 420
   ctgggcaccg acctggtaag catcccccat ggccccaacg tcactgtgcg tgccaacatt 480
   gctgccatca ctgaatcaga caagttcttc atcaacggct ccaactggga aggcatcctg 540
   gggctggcct atgctgagat tgccaggcct gacgactccc tggagccttt ctttgactct 600
   ctggtaaagc agacccacgt tcccaacctc ttctccctgc acctttgtgg tgctggcttc 660
   cccctcaacc agtctgaagt gctggcctct gtcggaggga gcatgatcat tggaggtatc 720
   gaccactcgc tgtacacagg cagtctctgg tatacaccca tccggcggga gtggtattat 780
   gaggtcatca ttgtgcgggt ggagatcaat ggacaggatc tgaaaatgga ctgcaaggag 840
   tacaactatg acaagagcat tgtggacagt ggcaccacca accttcgttt gcccaagaaa 900
   gtgtttgaag ctgcagtcaa atccatcaag gcagcctcct ccacggagaa gttccctgat 960
   ggtttctggc taggagagca gctggtgtgc tggcaagcag gcaccacccc ttggaacatt 1020
   ttcccagtca tctcactcta cctaatgggt gaggttacca accagtcctt ccgcatcacc 1080
   atccttccgc agcaatacct gcggccagtg gaagatgtgg ccacgtccca agacgactgt 1140
   tacaagtttg ccatctcaca gtcatccacg ggcactgtta tgggagctgt tatcatggag 1200
   ggcttctacg ttgtctttga tcgggcccga aaacgaattg gctttgctgt cagcgcttgc 1260
   catgtgcacg atgagttcag gacggcagcg gtggaaggcc cttttgtcac cttggacatg 1320
   gaagactgtg gctacaacat tccacagaca gatgagtcat ga                    1362
<210> 30
   <211> 453
   <212> PRT
   <213> Homo sapiens
<400> 30
   Met Ala Gln Ala Leu Pro Trp Leu Leu Leu Trp Met Gly Ala Gly Val
    1               5                  10                  15
   Leu Pro Ala His Gly Thr Gln His Gly Ile Arg Leu Pro Leu Arg Ser
               20                  25                  30
   Gly Leu Gly Gly Ala Pro Leu Gly Leu Arg Leu Pro Arg Glu Thr Asp
           35                  40                  45
   Glu Glu Pro Glu Glu Pro Gly Arg Arg Gly Ser Phe Val Glu Met Val
       50                  55                  60
   Asp Asn Leu Arg Gly Lys Ser Gly Gln Gly Tyr Tyr Val Glu Met Thr
   65                  70                  75                  80
   Val Gly Ser Pro Pro Gln Thr Leu Asn Ile Leu Val Asp Thr Gly Ser
                   85                  90                  95
   Ser Asn Phe Ala Val Gly Ala Ala Pro His Pro Phe Leu His Arg Tyr
               100                 105                 110
   Tyr Gln Arg Gln Leu Ser Ser Thr Tyr Arg Asp Leu Arg Lys Gly Val
           115                 120                 125
   Tyr Val Pro Tyr Thr Gln Gly Lys Trp Glu Gly Glu Leu Gly Thr Asp
       130                 135                 140
   Leu Val Ser Ile Pro His Gly Pro Asn Val Thr Val Arg Ala Asn Ile
   145                 150                 155                 160
   Ala Ala Ile Thr Glu Ser Asp Lys Phe Phe Ile Asn Gly Ser Asn Trp
                   165                 170                 175
   Glu Gly Ile Leu Gly Leu Ala Tyr Ala Glu Ile Ala Arg Pro Asp Asp
               180                 185                 190
   Ser Leu Glu Pro Phe Phe Asp Ser Leu Val Lys Gln Thr His Val Pro
           195                 200                 205
   Asn Leu Phe Ser Leu Gln Leu Cys Gly Ala Gly Phe Pro Leu Asn Gln
       210                 215                 220
   Ser Glu Val Leu Ala Ser Val Gly Gly Ser Met Ile Ile Gly Gly Ile
   225                 230                 235                 240
   Asp His Ser Leu Tyr Thr Gly Ser Leu Trp Tyr Thr Pro Ile Arg Arg
                   245                 250                 255
   Glu Trp Tyr Tyr Glu Val Ile Ile Val Arg Val Glu Ile Asn Gly Gln
               260                 265                 270
   Asp Leu Lys Met Asp Cys Lys Glu Tyr Asn Tyr Asp Lys Ser Ile Val
           275                 280                 285
   Asp Ser Gly Thr Thr Asn Leu Arg Leu Pro Lys Lys Val Phe Glu Ala
       290                 295                 300
   Ala Val Lys Ser Ile Lys Ala Ala Ser Ser Thr Glu Lys Phe Pro Asp
   305                 310                 315                 320
   Gly Phe Trp Leu Gly Glu Gln Leu Val Cys Trp Gln Ala Gly Thr Thr
                   325                 330                 335
   Pro Trp Asn Ile Phe Pro Val Ile Ser Leu Tyr Leu Met Gly Glu Val
               340                 345                 350
   Thr Asn Gln Ser Phe Arg Ile Thr Ile Leu Pro Gln Gln Tyr Leu Arg
           355                 360                 365
   Pro Val Glu Asp Val Ala Thr Ser Gln Asp Asp Cys Tyr Lys Phe Ala
       370                 375                 380
   Ile Ser Gln Ser Ser Thr Gly Thr Val Met Gly Ala Val Ile Met Glu
   385                 390                 395                 400
   Gly Phe Tyr Val Val Phe Asp Arg Ala Arg Lys Arg Ile Gly Phe Ala
                   405                 410                 415
   Val Ser Ala Cys His Val His Asp Glu Phe Arg Thr Ala Ala Val Glu
               420                 425                 430
   Gly Pro Phe Val Thr Leu Asp Met Glu Asp Cys Gly Tyr Asn Ile Pro
           435                 440                 445
   Gln Thr Asp Glu Ser
       450
<210> 31
   <211> 1380
   <212> DNA
   <213> Homo sapiens
<400> 31
   atggcccaag ccctgccctg gctcctgctg tggatgggcg cgggagtgct gcctgcccac 60
   ggcacccagc acggcatccg gctgcccctg cgcagcggcc tggggggcgc ccccctgggg 120
   ctgcggctgc cccgggagac cgacgaagag cccgaggagc ccggccggag gggcagcttt 180
   gtggagatgg tggacaacct gaggggcaag tcggggcagg gctactacgt ggagatgacc 240
   gtgggcagcc ccccgcagac gctcaacatc ctggtggata caggcagcag taactttgca 300
   gtgggtgctg ccccccaccc cttcctgcat cgctactacc agaggcagct gtccagcaca 360
   taccgggacc tccggaaggg tgtgtatgtg ccctacaccc agggcaagtg ggaaggggag 420
   ctgggcaccg acctggtaag catcccccat ggccccaacg tcactgtgcg tgccaacatt 480
   gctgccatca ctgaatcaga caagttcttc atcaacggct ccaactggga aggcatcctg 540
   gggctggcct atgctgagat tgccaggcct gacgactccc tggagccttt ctttgactct 600
   ctggtaaagc agacccacgt tcccaacctc ttctccctgc acctttgtgg tgctggcttc 660
   cccctcaacc agtctgaagt gctggcctct gtcggaggga gcatgatcat tggaggtatc 720
   gaccactcgc tgtacacagg cagtctctgg tatacaccca tccggcggga gtggtattat 780
   gaggtcatca ttgtgcgggt ggagatcaat ggacaggatc tgaaaatgga ctgcaaggag 840
   tacaactatg acaagagcat tgtggacagt ggcaccacca accttcgttt gcccaagaaa 900
   gtgtttgaag ctgcagtcaa atccatcaag gcagcctcct ccacggagaa gttccctgat 960
   ggtttctggc taggagagca gctggtgtgc tggcaagcag gcaccacccc ttggaacatt 1020
   ttcccagtca tctcactcta cctaatgggt gaggttacca accagtcctt ccgcatcacc 1080
   atccttccgc agcaatacct gcggccagtg gaagatgtgg ccacgtccca agacgactgt 1140
   tacaagtttg ccatctcaca gtcatccacg ggcactgtta tgggagctgt tatcatggag 1200
   ggcttctacg ttgtctttga tcgggcccga aaacgaattg gctttgctgt cagcgcttgc 1260
   catgtgcacg atgagttcag gacggcagcg gtggaaggcc cttttgtcac cttggacatg 1320
   gaagactgtg gctacaacat tccacagaca gatgagtcac agcagcagca gcagcagtga 1380
<210> 32
   <211> 459
   <212> PRT
   <213> Homo sapiens
<400> 32
   Met Ala Gln Ala Leu Pro Trp Leu Leu Leu Trp Met Gly Ala Gly Val
    1               5                  10                  15
   Leu Pro Ala His Gly Thr Gln His Gly Ile Arg Leu Pro Leu Arg Ser
               20                  25                  30
   Gly Leu Gly Gly Ala Pro Leu Gly Leu Arg Leu Pro Arg Glu Thr Asp
           35                  40                  45
   Glu Glu Pro Glu Glu Pro Gly Arg Arg Gly Ser Phe Val Glu Met Val
       50                  55                  60
   Asp Asn Leu Arg Gly Lys Ser Gly Gln Gly Tyr Tyr Val Glu Met Thr
   65                  70                  75                  80
   Val Gly Ser Pro Pro Gln Thr Leu Asn Ile Leu Val Asp Thr Gly Ser
                   85                  90                  95
   Ser Asn Phe Ala Val Gly Ala Ala Pro His Pro Phe Leu His Arg Tyr
               100                 105                 110
   Tyr Gln Arg Gln Leu Ser Ser Thr Tyr Arg Asp Leu Arg Lys Gly Val
           115                 120                 125
   Tyr Val Pro Tyr Thr Gln Gly Lys Trp Glu Gly Glu Leu Gly Thr Asp
       130                 135                 140
   Leu Val Ser Ile Pro His Gly Pro Asn Val Thr Val Arg Ala Asn Ile
   145                 150                 155                 160
   Ala Ala Ile Thr Glu Ser Asp Lys Phe Phe Ile Asn Gly Ser Asn Trp
                   165                 170                 175
   Glu Gly Ile Leu Gly Leu Ala Tyr Ala Glu Ile Ala Arg Pro Asp Asp
               180                 185                 190
   Ser Leu Glu Pro Phe Phe Asp Ser Leu Val Lys Gln Thr His Val Pro
           195                 200                 205
   Asn Leu Phe Ser Leu Gln Leu Cys Gly Ala Gly Phe Pro Leu Asn Gln
       210                 215                 220
   Ser Glu Val Leu Ala Ser Val Gly Gly Ser Met Ile Ile Gly Gly Ile
   225                 230                 235                 240
   Asp His Ser Leu Tyr Thr Gly Ser Leu Trp Tyr Thr Pro Ile Arg Arg
                   245                 250                 255
   Glu Trp Tyr Tyr Glu Val Ile Ile Val Arg Val Glu Ile Asn Gly Gln
               260                 265                 270
   Asp Leu Lys Met Asp Cys Lys Glu Tyr Asn Tyr Asp Lys Ser Ile Val
           275                 280                 285
   Asp Ser Gly Thr Thr Asn Leu Arg Leu Pro Lys Lys Val Phe Glu Ala
       290                 295                 300
   Ala Val Lys Ser Ile Lys Ala Ala Ser Ser Thr Glu Lys Phe Pro Asp
   305                 310                 315                 320
   Gly Phe Trp Leu Gly Glu Gln Leu Val Cys Trp Gln Ala Gly Thr Thr
                   325                 330                 335
   Pro Trp Asn Ile Phe Pro Val Ile Ser Leu Tyr Leu Met Gly Glu Val
               340                 345                 350
   Thr Asn Gln Ser Phe Arg Ile Thr Ile Leu Pro Gln Gln Tyr Leu Arg
           355                 360                 365
   Pro Val Glu Asp Val Ala Thr Ser Gln Asp Asp Cys Tyr Lys Phe Ala
       370                 375                 380
   Ile Ser Gln Ser Ser Thr Gly Thr Val Met Gly Ala Val Ile Met Glu
   385                 390                 395                 400
   Gly Phe Tyr Val Val Phe Asp Arg Ala Arg Lys Arg Ile Gly Phe Ala
                   405                 410                 415
   Val Ser Ala Cys His Val His Asp Glu Phe Arg Thr Ala Ala Val Glu
               420                 425                 430
   Gly Pro Phe Val Thr Leu Asp Met Glu Asp Cys Gly Tyr Asn Ile Pro
           435                 440                 445
   Gln Thr Asp Glu Ser His His His His His His
       450                 455
<210> 33
   <211> 25
   <212> PRT
   <213> Homo sapiens
<400> 33
   Ser Glu Gln Gln Arg Arg Pro Arg Asp Pro Glu Val Val Asn Asp Glu
   1 5 10 15
   Ser Ser Leu Val Arg His Arg Trp Lys
   20 25
<210> 34
   <211> 19
   <212> PRT
   <213> Homo sapiens
<400> 34
   Ser Glu Gln Leu Arg Gln Gln His Asp Asp Phe Ala Asp Asp Ile Ser
   1 5 10 15
   Leu Leu Lys
<210> 35
   <211> 29
   <212> DNA
   <213> Homo sapiens
<400> 35
   gtggatccac ccagcacggc atccggctg 29
<210> 36
   <211> 36
   <212> DNA
   <213> Homo sapiens
<400> 36
   gaaagctttc atgactcatc tgtctgtgga atgttg 36
<210> 37
   <211> 39
   <212> DNA
   <213> Homo sapiens
<400> 37
   gatcgatgac tatctctgac tctccgcgtg aacaggacg 39
<210> 38
   <211> 39
   <212> DNA
   <213> Homo sapiens
<400> 38
   gatccgtcct gttcacgcgg agagtcagag atagtcatc 39
<210> 39
   <211> 77
   <212> DNA
   <213> Artificial Sequence
<220>
   <223> Description of Artificial Sequence: Hu-Asp2
<400> 39
   cggcatccgg ctgcccctgc gtagcggtct gggtggtgct ccactgggtc tgcgtctgcc 60
   ccgggagacc gacgaag 77
<210> 40
   <211> 77
   <212> DNA
   <213> Artificial Sequence
<220>
   <223> Description of Artificial Sequence: Hu-Asp2
<400> 40
   cttcgtcggt ctcccggggc agacgcagac ccagtggagc accacccaga ccgctacgca 60
   ggggcagccg gatgccg 77
<210> 41
   <211> 51
   <212> DNA
   <213> Artificial Sequence
<220>
<223> Description of Artificial Sequence: Caspase 8
   Cleavage Site
<400> 41
   gatcgatgac tatctctgac tctccgctgg actctggtat cgaaaccgac g 51
<210> 42
   <211> 51
   <212> DNA
   <213> Artificial Sequence
<220>
   <223> Description of Artificial Sequence: Caspase 8
   Cleavage Site
<400> 42
   gatccgtcgg tttcgatacc agagtccagc ggagagtcag agatagtcat c 51
<210> 43
   <211> 32
   <212> DNA
   <213> Homo sapiens
<400> 43
   aaggatcctt tgtggagatg gtggacaacc tg 32
<210> 44
   <211> 36
   <212> DNA
   <213> Homo sapiens
<400> 44
   gaaagctttc atgactcatc tgtctgtgga atgttg 36
<210> 45
   <211> 24
   <212> DNA
   <213> Artificial Sequence
<220>
   <223> Description of Artificial Sequence: 6-His tag
<400> 45
   gatcgcatca tcaccatcac catg 24
<210> 46
   <211> 24
   <212> DNA
   <213> Artificial Sequence
<220>
   <223> Description of Artificial Sequence: 6-His tag
<400> 46
   gatccatggt gatggtgatg atgc 24
<210> 47
   <211> 22
   <212> DNA
   <213> Artificial Sequence
<220>
   <223> Description of Artificial Sequence: primer
<400> 47
   gactgaccac tcgaccaggt tc 22
<210> 48
   <211> 51
   <212> DNA
   <213> Artificial Sequence
<220>
   <223> Description of Artificial Sequence: primer
<400> 48
   cgaattaaat tccagcacac tggctacttc ttgttctgca tctcaaagaa c 51
<210> 49
   <211> 26
   <212> DNA
   <213> Artificial Sequence
<220>
   <223> Description of Artificial Sequence: primer
<400> 49
   cgaattaaat tccagcacac tggcta 26
<210> 50
   <211> 1287
   <212> DNA
   <213> Artificial Sequence
<220>
   <223> Description of Artificial Sequence: Hu-Asp2(b)
   delta TM
<400> 50
   atggcccaag ccctgccctg gctcctgctg tggatgggcg cgggagtgct gcctgcccac 60
   ggcacccagc acggcatccg gctgcccctg cgcagcggcc tggggggcgc ccccctgggg 120
   ctgcggctgc cccgggagac cgacgaagag cccgaggagc ccggccggag gggcagcttt 180
   gtggagatgg tggacaacct gaggggcaag tcggggcagg gctactacgt ggagatgacc 240
   gtgggcagcc ccccgcagac gctcaacatc ctggtggata caggcagcag taactttgca 300
   gtgggtgctg ccccccaccc cttcctgcat cgctactacc agaggcagct gtccagcaca 360
   taccgggacc tccggaaggg tgtgtatgtg ccctacaccc agggcaagtg ggaaggggag 420
   ctgggcaccg acctggtaag catcccccat ggccccaacg tcactgtgcg tgccaacatt 480
   gctgccatca ctgaatcaga caagttcttc atcaacggct ccaactggga aggcatcctg 540
   gggctggcct atgctgagat tgccaggctt tgtggtgctg gcttccccct caaccagtct 600
   gaagtgctgg cctctgtcgg agggagcatg atcattggag gtatcgacca ctcgctgtac 660
   acaggcagtc tctggtatac acccatccgg cgggagtggt attatgaggt catcattgtg 720
   cgggtggaga tcaatggaca ggatctgaaa atggactgca aggagtacaa ctatgacaag 780
   agcattgtgg acagtggcac caccaacctt cgtttgccca agaaagtgtt tgaagctgca 840
   gtcaaatcca tcaaggcagc ctcctccacg gagaagttcc ctgatggttt ctggctagga 900
   gagcagctgg tgtgctggca agcaggcacc accccttgga acattttccc agtcatctca 960
   ctctacctaa tgggtgaggt taccaaccag tccttccgca tcaccatcct tccgcagcaa 1020
   tacctgcggc cagtggaaga tgtggccacg tcccaagacg actgttacaa gtttgccatc 1080
   tcacagtcat ccacgggcac tgttatggga gctgttatca tggagggctt ctacgttgtc 1140
   tttgatcggg cccgaaaacg aattggcttt gctgtcagcg cttgccatgt gcacgatgag 1200
   ttcaggacgg cagcggtgga aggccctttt gtcaccttgg acatggaaga ctgtggctac 1260
   aacattccac agacagatga gtcatga 1287
<210> 51
   <211> 428
   <212> PRT
   <213> Artificial Sequence
<220>
   <223> Description of Artificial Sequence: Hu-Asp2(b) delta TM
<400> 51
   Met Ala Gln Ala Leu Pro Trp Leu Leu Leu Trp Met Gly Ala Gly Val
    1               5                  10                  15
   Leu Pro Ala His Gly Thr Gln His Gly Ile Arg Leu Pro Leu Arg Ser
               20                  25                  30
   Gly Leu Gly Gly Ala Pro Leu Gly Leu Arg Leu Pro Arg Glu Thr Asp
           35                  40                  45
   Glu Glu Pro Glu Glu Pro Gly Arg Arg Gly Ser Phe Val Glu Met Val
       50                  55                  60
   Asp Asn Leu Arg Gly Lys Ser Gly Gln Gly Tyr Tyr Val Glu Met Thr
   65                  70                  75                  80
   Val Gly Ser Pro Pro Gln Thr Leu Asn Ile Leu Val Asp Thr Gly Ser
                   85                  90                  95
   Ser Asn Phe Ala Val Gly Ala Ala Pro His Pro Phe Leu His Arg Tyr
               100                 105                 110
   Tyr Gln Arg Gln Leu Ser Ser Thr Tyr Arg Asp Leu Arg Lys Gly Val
           115                 120                 125
   Tyr Val Pro Tyr Thr Gln Gly Lys Trp Glu Gly Glu Leu Gly Thr Asp
       130                 135                 140
   Leu Val Ser Ile Pro His Gly Pro Asn Val Thr Val Arg Ala Asn Ile
   145                 150                 155                 160
   Ala Ala Ile Thr Glu Ser Asp Lys Phe Phe Ile Asn Gly Ser Asn Trp
                   165                 170                 175
   Glu Gly Ile Leu Gly Leu Ala Tyr Ala Glu Ile Ala Arg Leu Cys Gly
               180                 185                 190
   Ala Gly Phe Pro Leu Asn Gln Ser Glu Val Leu Ala Ser Val Gly Gly
           195                 200                 205
   Ser Met Ile Ile Gly Gly Ile Asp His Ser Leu Tyr Thr Gly Ser Leu
       210                 215                 220
   Trp Tyr Thr Pro Ile Arg Arg Glu Trp Tyr Tyr Glu Val Ile Ile Val
   225                 230                 235                 240
   Arg Val Glu Ile Asn Gly Gln Asp Leu Lys Met Asp Cys Lys Glu Tyr
                   245                 250                 255
   Asn Tyr Asp Lys Ser Ile Val Asp Ser Gly Thr Thr Asn Leu Arg Leu
               260                 265                 270
   Pro Lys Lys Val Phe Glu Ala Ala Val Lys Ser Ile Lys Ala Ala Ser
           275                 280                 285
   Ser Thr Glu Lys Phe Pro Asp Gly Phe Trp Leu Gly Glu Gln Leu Val
       290                 295                 300
   Cys Trp Gln Ala Gly Thr Thr Pro Trp Asn Ile Phe Pro Val Ile Ser
   305                 310                 315                 320
   Leu Tyr Leu Met Gly Glu Val Thr Asn Gln Ser Phe Arg Ile Thr Ile
                   325                 330                 335
   Leu Pro Gln Gln Tyr Leu Arg Pro Val Glu Asp Val Ala Thr Ser Gln
               340                 345                 350
   Asp Asp Cys Tyr Lys Phe Ala Ile Ser Gln Ser Ser Thr Gly Thr Val
           355                 360                 365
   Met Gly Ala Val Ile Met Glu Gly Phe Tyr Val Val Phe Asp Arg Ala
       370                 375                 380
   Arg Lys Arg Ile Gly Phe Ala Val Ser Ala Cys His Val His Asp Glu
   385                 390                 395                 400
   Phe Arg Thr Ala Ala Val Glu Gly Pro Phe Val Thr Leu Asp Met Glu
                   405                 410                 415
   Asp Cys Gly Tyr Asn Ile Pro Gln Thr Asp Glu Ser
               420                 425
<210> 52
   <211> 1305
   <212> DNA
   <213> Artificial Sequence
<220>
   <223> Description of Artificial Sequence: Hu-Asp2 (b) delta TM
<400> 52
   atggcccaag ccctgccctg gctcctgctg tggatgggcg cgggagtgct gcctgcccac 60
   ggcacccagc acggcatccg gctgcccctg cgcagcggcc tggggggcgc ccccctgggg 120
   ctgcggctgc cccgggagac cgacgaagag cccgaggagc ccggccggag gggcagcttt 180
   gtggagatgg tggacaacct gaggggcaag tcggggcagg gctactacgt ggagatgacc 240
   gtgggcagcc ccccgcagac gctcaacatc ctggtggata caggcagcag taactttgca 300
   gtgggtgctg ccccccaccc cttcctgcat cgctactacc agaggcagct gtccagcaca 360
   taccgggacc tccggaaggg tgtgtatgtg ccctacaccc agggcaagtg ggaaggggag 420
   ctgggcaccg acctggtaag catcccccat ggccccaacg tcactgtgcg tgccaacatt 480
   gctgccatca ctgaatcaga caagttcttc atcaacggct ccaactggga aggcatcctg 540
   gggctggcct atgctgagat tgccaggctt tgtggtgctg gcttccccct caaccagtct 600
   gaagtgctgg cctctgtcgg agggagcatg atcattggag gtatcgacca ctcgctgtac 660
   acaggcagtc tctggtatac acccatccgg cgggagtggt attatgaggt catcattgtg 720
   cgggtggaga tcaatggaca ggatctgaaa atggactgca aggagtacaa ctatgacaag 780
   agcattgtgg acagtggcac caccaacctt cgtttgccca agaaagtgtt tgaagctgca 840
   gtcaaatcca tcaaggcagc ctcctccacg gagaagttcc ctgatggttt ctggctagga 900
   gagcagctgg tgtgctggca agcaggcacc accccttgga acattttccc agtcatctca 960
   ctctacctaa tgggtgaggt taccaaccag tccttccgca tcaccatcct tccgcagcaa 1020
   tacctgcggc cagtggaaga tgtggccacg tcccaagacg actgttacaa gtttgccatc 1080
   tcacagtcat ccacgggcac tgttatggga gctgttatca tggagggctt ctacgttgtc 1140
   tttgatcggg cccgaaaacg aattggcttt gctgtcagcg cttgccatgt gcacgatgag 1200
   ttcaggacgg cagcggtgga aggccctttt gtcaccttgg acatggaaga ctgtggctac 1260
   aacattccac agacagatga gtcacagcag cagcagcagc agtga             1305
<210> 53
   <211> 434
   <212> PRT
   <213> Artificial Sequence
<220>
   <223> Description of Artificial Sequence: Hu-Asp2(b) delta TM
<400> 53
   Met Ala Gln Ala Leu Pro Trp Leu Leu Leu Trp Met Gly Ala Gly Val
    1               5                  10                  15
   Leu Pro Ala His Gly Thr Gln His Gly Ile Arg Leu Pro Leu Arg Ser
               20                  25                  30
   Gly Leu Gly Gly Ala Pro Leu Gly Leu Arg Leu Pro Arg Glu Thr Asp
           35                  40                  45
   Glu Glu Pro Glu Glu Pro Gly Arg Arg Gly Ser Phe Val Glu Met Val
       50                  55                  60
   Asp Asn Leu Arg Gly Lys Ser Gly Gln Gly Tyr Tyr Val Glu Met Thr
   65                  70                  75                  80
   Val Gly Ser Pro Pro Gln Thr Leu Asn Ile Leu Val Asp Thr Gly Ser
                   85                  90                  95
   Ser Asn Phe Ala Val Gly Ala Ala Pro His Pro Phe Leu His Arg Tyr
               100                 105                 110
   Tyr Gln Arg Gln Leu Ser Ser Thr Tyr Arg Asp Leu Arg Lys Gly Val
           115                 120                 125
   Tyr Val Pro Tyr Thr Gln Gly Lys Trp Glu Gly Glu Leu Gly Thr Asp
       130                 135                 140
   Leu Val Ser Ile Pro His Gly Pro Asn Val Thr Val Arg Ala Asn Ile
   145                 150                 155                 160
   Ala Ala Ile Thr Glu Ser Asp Lys Phe Phe Ile Asn Gly Ser Asn Trp
                   165                 170                 175
   Glu Gly Ile Leu Gly Leu Ala Tyr Ala Glu Ile Ala Arg Leu Cys Gly
               180                 185                 190
   Ala Gly Phe Pro Leu Asn Gln Ser Glu Val Leu Ala Ser Val Gly Gly
           195                 200                 205
   Ser Met Ile Ile Gly Gly Ile Asp His Ser Leu Tyr Thr Gly Ser Leu
       210                 215                 220
   Trp Tyr Thr Pro Ile Arg Arg Glu Trp Tyr Tyr Glu Val Ile Ile Val
   225                 230                 235                 240
   Arg Val Glu Ile Asn Gly Gln Asp Leu Lys Met Asp Cys Lys Glu Tyr
                   245                 250                 255
   Asn Tyr Asp Lys Ser Ile Val Asp Ser Gly Thr Thr Asn Leu Arg Leu
               260                 265                 270
   Pro Lys Lys Val Phe Glu Ala Ala Val Lys Ser Ile Lys Ala Ala Ser
           275                 280                 285
   Ser Thr Glu Lys Phe Pro Asp Gly Phe Trp Leu Gly Glu Gln Leu Val
       290                 295                 300
   Cys Trp Gln Ala Gly Thr Thr Pro Trp Asn Ile Phe Pro Val Ile Ser
   305                 310                 315                 320
   Leu Tyr Leu Met Gly Glu Val Thr Asn Gln Ser Phe Arg Ile Thr Ile
                   325                 330                 335
   Leu Pro Gln Gln Tyr Leu Arg Pro Val Glu Asp Val Ala Thr Ser Gln
               340                 345                 350
   Asp Asp Cys Tyr Lys Phe Ala Ile Ser Gln Ser Ser Thr Gly Thr Val
           355                 360                 365
   Met Gly Ala Val Ile Met Glu Gly Phe Tyr Val Val Phe Asp Arg Ala
       370                 375                 380
   Arg Lys Arg Ile Gly Phe Ala Val Ser Ala Cys His Val His Asp Glu
   385                 390                 395                 400
   Phe Arg Thr Ala Ala Val Glu Gly Pro Phe Val Thr Leu Asp Met Glu
                   405                 410                 415
   Asp Cys Gly Tyr Asn Ile Pro Gln Thr Asp Glu Ser His His His His
               420                 425                 430
   His His
<210> 54
   <211> 2310
   <212> DNA
   <213> Homo sapiens
<400> 54
   atgctgcccg gtttggcact gctcctgctg gccgcctgga cggctcgggc gctggaggta 60
   cccactgatg gtaatgctgg cctgctggct gaaccccaga ttgccatgtt ctgtggcaga 120
   ctgaacatgc acatgaatgt ccagaatggg aagtgggatt cagatccatc agggaccaaa 180
   acctgcattg ataccaagga aggcatcctg cagtattgcc aagaagtcta ccctgaactg 240
   cagatcacca atgtggtaga agccaaccaa ccagtgacca tccagaactg gtgcaagcgg 300
   ggccgcaagc agtgcaagac ccatccccac tttgtgattc cctaccgctg cttagttggt 360
   gagtttgtaa gtgatgccct tctcgttcct gacaagtgca aattcttaca ccaggagagg 420
   atggatgttt gcgaaactca tcttcactgg cacaccgtcg ccaaagagac atgcagtgag 480
   aagagtacca acttgcatga ctacggcatg ttgctgccct gcggaattga caagttccga 540
   ggggtagagt ttgtgtgttg cccactggct gaagaaagtg acaatgtgga ttctgctgat 600
   gcggaggagg atgactcgga tgtctggtgg ggcggagcag acacagacta tgcagatggg 660
   agtgaagaca aagtagtaga agtagcagag gaggaagaag tggctgaggt ggaagaagaa 720
   gaagccgatg atgacgagga cgatgaggat ggtgatgagg tagaggaaga ggctgaggaa 780
   ccctacgaag aagccacaga gagaaccacc agcattgcca ccaccaccac caccaccaca 840
   gagtctgtgg aagaggtggt tcgagaggtg tgctctgaac aagccgagac ggggccgtgc 900
   cgagcaatga tctcccgctg gtactttgat gtgactgaag ggaagtgtgc cccattcttt 960
   tacggcggat gtggcggcaa ccggaacaac tttgacacag aagagtactg catggccgtg 1020
   tgtggcagcg ccatgtccca aagtttactc aagactaccc aggaacctct tggccgagat 1080
   cctgttaaac ttcctacaac agcagccagt acccctgatg ccgttgacaa gtatctcgag 1140
   acacctgggg atgagaatga acatgcccat ttccagaaag ccaaagagag gcttgaggcc 1200
   aagcaccgag agagaatgtc ccaggtcatg agagaatggg aagaggcaga acgtcaagca 1260
   aagaacttgc ctaaagctga taagaaggca gttatccagc atttccagga gaaagtggaa 1320
   tctttggaac aggaagcagc caacgagaga cagcagctgg tggagacaca catggccaga 1380
   gtggaagcca tgctcaatga ccgccgccgc ctggccctgg agaactacat caccgctctg 1440
   caggctgttc ctcctcggcc tcgtcacgtg ttcaatatgc taaagaagta tgtccgcgca 1500
   gaacagaagg acagacagca caccctaaag catttcgagc atgtgcgcat ggtggatccc 1560
   aagaaagccg ctcagatccg gtcccaggtt atgacacacc tccgtgtgat ttatgagcgc 1620
   atgaatcagt ctctctccct gctctacaac gtgcctgcag tggccgagga gattcaggat 1680
   gaagttgatg agctgcttca gaaagagcaa aactattcag atgacgtctt ggccaacatg 1740
   attagtgaac caaggatcag ttacggaaac gatgctctca tgccatcttt gaccgaaacg 1800
   aaaaccaccg tggagctcct tcccgtgaat ggagagttca gcctggacga tctccagccg 1860
   tggcattctt ttggggctga ctctgtgcca gccaacacag aaaacgaagt tgagcctgtt 1920
   gatgcccgcc ctgctgccga ccgaggactg accactcgac caggttctgg gttgacaaat 1980
   atcaagacgg aggagatctc tgaagtgaag atggatgcag aattccgaca tgactcagga 2040
   tatgaagttc atcatcaaaa attggtgttc tttgcagaag atgtgggttc aaacaaaggt 2100
   gcaatcattg gactcatggt gggcggtgtt gtcatagcga cagtgatcgt catcaccttg 2160
   gtgatgctga agaagaaaca gtacacatcc attcatcatg gtgtggtgga ggttgacgcc 2220
   gctgtcaccc cagaggagcg ccacctgtcc aagatgcagc agaacggcta cgaaaatcca 2280
   acctacaagt tctttgagca gatgcagaac                                  2310
<210> 55
   <211> 770
   <212> PRT
   <213> Homo sapiens
<400> 55
<210> 56
   <211> 2253
   <212> DNA
   <213> Homo sapiens
<400> 56
   atgctgcccg gtttggcact gctcctgctg gccgcctgga cggctcgggc gctggaggta 60
   cccactgatg gtaatgctgg cctgctggct gaaccccaga ttgccatgtt ctgtggcaga 120
   ctgaacatgc acatgaatgt ccagaatggg aagtgggatt cagatccatc agggaccaaa 180
   acctgcattg ataccaagga aggcatcctg cagtattgcc aagaagtcta ccctgaactg 240
   cagatcacca atgtggtaga agccaaccaa ccagtgacca tccagaactg gtgcaagcgg 300
   ggccgcaagc agtgcaagac ccatccccac tttgtgattc cctaccgctg cttagttggt 360
   gagtttgtaa gtgatgccct tctcgttcct gacaagtgca aattcttaca ccaggagagg 420
   atggatgttt gcgaaactca tcttcactgg cacaccgtcg ccaaagagac atgcagtgag 480
   aagagtacca acttgcatga ctacggcatg ttgctgccct gcggaattga caagttccga 540
   ggggtagagt ttgtgtgttg cccactggct gaagaaagtg acaatgtgga ttctgctgat 600
   gcggaggagg atgactcgga tgtctggtgg ggcggagcag acacagacta tgcagatggg 660
   agtgaagaca aagtagtaga agtagcagag gaggaagaag tggctgaggt ggaagaagaa 720
   gaagccgatg atgacgagga cgatgaggat ggtgatgagg tagaggaaga ggctgaggaa 780
   ccctacgaag aagccacaga gagaaccacc agcattgcca ccaccaccac caccaccaca 840
   gagtctgtgg aagaggtggt tcgagaggtg tgctctgaac aagccgagac ggggccgtgc 900
   cgagcaatga tctcccgctg gtactttgat gtgactgaag ggaagtgtgc cccattcttt 960
   tacggcggat gtggcggcaa ccggaacaac tttgacacag aagagtactg catggccgtg 1020
   tgtggcagcg ccattcctac aacagcagcc agtacccctg atgccgttga caagtatctc 1080
   gagacacctg gggatgagaa tgaacatgcc catttccaga aagccaaaga gaggcttgag 1140
   gccaagcacc gagagagaat gtcccaggtc atgagagaat gggaagaggc agaacgtcaa 1200
   gcaaagaact tgcctaaagc tgataagaag gcagttatcc agcatttcca ggagaaagtg 1260
   gaatctttgg aacaggaagc agccaacgag agacagcagc tggtggagac acacatggcc 1320
   agagtggaag ccatgctcaa tgaccgccgc cgcctggccc tggagaacta catcaccgct 1380
   ctgcaggctg ttcctcctcg gcctcgtcac gtgttcaata tgctaaagaa gtatgtccgc 1440
   gcagaacaga aggacagaca gcacacccta aagcatttcg agcatgtgcg catggtggat 1500
   cccaagaaag ccgctcagat ccggtcccag gttatgacac acctccgtgt gatttatgag 1560
   cgcatgaatc agtctctctc cctgctctac aacgtgcctg cagtggccga ggagattcag 1620
   gatgaagttg atgagctgct tcagaaagag caaaactatt cagatgacgt cttggccaac 1680
   atgattagtg aaccaaggat cagttacgga aacgatgctc tcatgccatc tttgaccgaa 1740
   acgaaaacca ccgtggagct ccttcccgtg aatggagagt tcagcctgga cgatctccag 1800
   ccgtggcatt cttttggggc tgactctgtg ccagccaaca cagaaaacga agttgagcct 1860
   gttgatgccc gccctgctgc cgaccgagga ctgaccactc gaccaggttc tgggttgaca 1920
   aatatcaaga cggaggagat ctctgaagtg aagatggatg cagaattccg acatgactca 1980
   ggatatgaag ttcatcatca aaaattggtg ttctttgcag aagatgtggg ttcaaacaaa 2040
   ggtgcaatca ttggactcat ggtgggcggt gttgtcatag cgacagtgat cgtcatcacc 2100
   ttggtgatgc tgaagaagaa acagtacaca tccattcatc atggtgtggt ggaggttgac 2160
   gccgctgtca ccccagagga gcgccacctg tccaagatgc agcagaacgg ctacgaaaat 2220
   ccaacctaca agttctttga gcagatgcag aac                              2253
<210> 57
   <211> 751
   <212> PRT
   <213> Homo sapiens
<400> 57
   Met Leu Pro Gly Leu Ala Leu Leu Leu Leu Ala Ala Trp Thr Ala Arg
    1               5                  10                  15
   Ala Leu Glu Val Pro Thr Asp Gly Asn Ala Gly Leu Leu Ala Glu Pro
               20                  25                  30
   Gln Ile Ala Met Phe Cys Gly Arg Leu Asn Met His Met Asn Val Gln
           35                  40                  45
   Asn Gly Lys Trp Asp Ser Asp Pro Ser Gly Thr Lys Thr Cys Ile Asp
       50                  55                  60
   Thr Lys Glu Gly Ile Leu Gln Tyr Cys Gln Glu Val Tyr Pro Glu Leu
   65                  70                  75                  80
   Gln Ile Thr Asn Val Val Glu Ala Asn Gln Pro Val Thr Ile Gln Asn
   85 90 95
   Trp Cys Lys Arg Gly Arg Lys Gln Cys Lys Thr His Pro His Phe Val
   100 105 110
   Ile Pro Tyr Arg Cys Leu Val Gly Glu Phe Val Ser Asp Ala Leu Leu
   115 120 125
   Val Pro Asp Lys Cys Lys Phe Leu His Gln Glu Arg Met Asp Val Cys
   130 135 140
   Glu Thr His Leu His Trp His Thr Val Ala Lys Glu Thr Cys Ser Glu
   145 150 155 160
   Lys Ser Thr Asn Leu His Asp Tyr Gly Met Leu Leu Pro Cys Gly Ile
   165 170 175
   Asp Lys Phe Arg Gly Val Glu Phe Val Cys Cys Pro Leu Ala Glu Glu
   180 185 190
   Ser Asp Asn Val Asp Ser Ala Asp Ala Glu Glu Asp Asp Ser Asp Val
   195 200 205
   Trp Trp Gly Gly Ala Asp Thr Asp Tyr Ala Asp Gly Ser Glu Asp Lys
   210 215 220
   Val Val Glu Val Ala Glu Glu Glu Glu Val Ala Glu Val Glu Glu Glu
   225 230 235 240
   Glu Ala Asp Asp Asp Glu Asp Asp Glu Asp Gly Asp Glu Val Glu Glu
   245 250 255
   Glu Ala Glu Glu Pro Tyr Glu Glu Ala Thr Glu Arg Thr Thr Ser Ile
   260 265 270
   Ala Thr Thr Thr Thr Thr Thr Thr Glu Ser Val Glu Glu Val Val Arg
   275 280 285
   Glu Val Cys Ser Glu Gln Ala Glu Thr Gly Pro Cys Arg Ala Met Ile
   290 295 300
   Ser Arg Trp Tyr Phe Asp Val Thr Glu Gly Lys Cys Ala Pro Phe Phe
   305 310 315 320
   Tyr Gly Gly Cys Gly Gly Asn Arg Asn Asn Phe Asp Thr Glu Glu Tyr
   325 330 335
   Cys Met Ala Val Cys Gly Ser Ala Ile Pro Thr Thr Ala Ala Ser Thr
   340 345 350
   Pro Asp Ala Val Asp Lys Tyr Leu Glu Thr Pro Gly Asp Glu Asn Glu
   355 360 365
   His Ala His Phe Gln Lys Ala Lys Glu Arg Leu Glu Ala Lys His Arg
   370 375 380
   Glu Arg Met Ser Gln Val Met Arg Glu Trp Glu Glu Ala Glu Arg Gln
   385 390 395 400
   Ala Lys Asn Leu Pro Lys Ala Asp Lys Lys Ala Val Ile Gln His Phe
   405 410 415
   Gln Glu Lys Val Glu Ser Leu Glu Gln Glu Ala Ala Asn Glu Arg Gln
   420 425 430
   Gln Leu Val Glu Thr His Met Ala Arg Val Glu Ala Met Leu Asn Asp
   435 440 445
   Arg Arg Arg Leu Ala Leu Glu Asn Tyr Ile Thr Ala Leu Gln Ala Val
   450 455 460
   Pro Pro Arg Pro Arg His Val Phe Asn Met Leu Lys Lys Tyr Val Arg
   465 470 475 480
   Ala Glu Gln Lys Asp Arg Gln His Thr Leu Lys His Phe Glu His Val
   485 490 495
   Arg Met Val Asp Pro Lys Lys Ala Ala Gln Ile Arg Ser Gln Val Met
   500 505 510
   Thr His Leu Arg Val Ile Tyr Glu Arg Met Asn Gln Ser Leu Ser Leu
   515 520 525
   Leu Tyr Asn Val Pro Ala Val Ala Glu Glu Ile Gln Asp Glu Val Asp
   530 535 540
   Glu Leu Leu Gln Lys Glu Gln Asn Tyr Ser Asp Asp Val Leu Ala Asn
   545 550 555 560
   Met Ile Ser Glu Pro Arg Ile Ser Tyr Gly Asn Asp Ala Leu Met Pro
   565 570 575
   Ser Leu Thr Glu Thr Lys Thr Thr Val Glu Leu Leu Pro Val Asn Gly
   580 585 590
   Glu Phe Ser Leu Asp Asp Leu Gln Pro Trp His Ser Phe Gly Ala Asp
   595 600 605
   Ser Val Pro Ala Asn Thr Glu Asn Glu Val Glu Pro Val Asp Ala Arg
   610 615 620
   Pro Ala Ala Asp Arg Gly Leu Thr Thr Arg Pro Gly Ser Gly Leu Thr
   625 630 635 640
   Asn Ile Lys Thr Glu Glu Ile Ser Glu Val Lys Met Asp Ala Glu Phe
   645 650 655
   Arg His Asp Ser Gly Tyr Glu Val His His Gln Lys Leu Val Phe Phe
   660 665 670
   Ala Glu Asp Val Gly Ser Asn Lys Gly Ala Ile Ile Gly Leu Met Val
   675 680 685
   Gly Gly Val Val Ile Ala Thr Val Ile Val Ile Thr Leu Val Met Leu
   690 695 700
   Lys Lys Lys Gln Tyr Thr Ser Ile His His Gly Val Val Glu Val Asp
   705 710 715 720
   Ala Ala Val Thr Pro Glu Glu Arg His Leu Ser Lys Met Gln Gln Asn
   725 730 735
   Gly Tyr Glu Asn Pro Thr Tyr Lys Phe Phe Glu Gln Met Gln Asn
   740 745 750
<210> 58
   <211> 2316
   <212> DNA
   <213> Homo sapiens
<400> 58
   atgctgcccg gtttggcact gctcctgctg gccgcctgga cggctcgggc gctggaggta 60
   cccactgatg gtaatgctgg cctgctggct gaaccccaga ttgccatgtt ctgtggcaga 120
   ctgaacatgc acatgaatgt ccagaatggg aagtgggatt cagatccatc agggaccaaa 180
   acctgcattg ataccaagga aggcatcctg cagtattgcc aagaagtcta ccctgaactg 240
   cagatcacca atgtggtaga agccaaccaa ccagtgacca tccagaactg gtgcaagcgg 300
   ggccgcaagc agtgcaagac ccatccccac tttgtgattc cctaccgctg cttagttggt 360
   gagtttgtaa gtgatgccct tctcgttcct gacaagtgca aattcttaca ccaggagagg 420
   atggatgttt gcgaaactca tcttcactgg cacaccgtcg ccaaagagac atgcagtgag 480
   aagagtacca acttgcatga ctacggcatg ttgctgccct gcggaattga caagttccga 540
   ggggtagagt ttgtgtgttg cccactggct gaagaaagtg acaatgtgga ttctgctgat 600
   gcggaggagg atgactcgga tgtctggtgg ggcggagcag acacagacta tgcagatggg 660
   agtgaagaca aagtagtaga agtagcagag gaggaagaag tggctgaggt ggaagaagaa 720
   gaagccgatg atgacgagga cgatgaggat ggtgatgagg tagaggaaga ggctgaggaa 780
   ccctacgaag aagccacaga gagaaccacc agcattgcca ccaccaccac caccaccaca 840
   gagtctgtgg aagaggtggt tcgagaggtg tgctctgaac aagccgagac ggggccgtgc 900
   cgagcaatga tctcccgctg gtactttgat gtgactgaag ggaagtgtgc cccattcttt 960
   tacggcggat gtggcggcaa ccggaacaac tttgacacag aagagtactg catggccgtg 1020
   tgtggcagcg ccatgtccca aagtttactc aagactaccc aggaacctct tggccgagat 1080
   cctgttaaac ttcctacaac agcagccagt acccctgatg ccgttgacaa gtatctcgag 1140
   acacctgggg atgagaatga acatgcccat ttccagaaag ccaaagagag gcttgaggcc 1200
   aagcaccgag agagaatgtc ccaggtcatg agagaatggg aagaggcaga acgtcaagca 1260
   aagaacttgc ctaaagctga taagaaggca gttatccagc atttccagga gaaagtggaa 1320
   tctttggaac aggaagcagc caacgagaga cagcagctgg tggagacaca catggccaga 1380
   gtggaagcca tgctcaatga ccgccgccgc ctggccctgg agaactacat caccgctctg 1440
   caggctgttc ctcctcggcc tcgtcacgtg ttcaatatgc taaagaagta tgtccgcgca 1500
   gaacagaagg acagacagca caccctaaag catttcgagc atgtgcgcat ggtggatccc 1560
   aagaaagccg ctcagatccg gtcccaggtt atgacacacc tccgtgtgat ttatgagcgc 1620
   atgaatcagt ctctctccct gctctacaac gtgcctgcag tggccgagga gattcaggat 1680
   gaagttgatg agctgcttca gaaagagcaa aactattcag atgacgtctt ggccaacatg 1740
   attagtgaac caaggatcag ttacggaaac gatgctctca tgccatcttt gaccgaaacg 1800
   aaaaccaccg tggagctcct tcccgtgaat ggagagttca gcctggacga tctccagccg 1860
   tggcattctt ttggggctga ctctgtgcca gccaacacag aaaacgaagt tgagcctgtt 1920
   gatgcccgcc ctgctgccga ccgaggactg accactcgac caggttctgg gttgacaaat 1980
   atcaagacgg aggagatctc tgaagtgaag atggatgcag aattccgaca tgactcagga 2040
   tatgaagttc atcatcaaaa attggtgttc tttgcagaag atgtgggttc aaacaaaggt 2100
   gcaatcattg gactcatggt gggcggtgtt gtcatagcga cagtgatcgt catcaccttg 2160
   gtgatgctga agaagaaaca gtacacatcc attcatcatg gtgtggtgga ggttgacgcc 2220
   gctgtcaccc cagaggagcg ccacctgtcc aagatgcagc agaacggcta cgaaaatcca 2280
   acctacaagt tctttgagca gatgcagaac aagaag                           2316
<210> 59
   <211> 772
   <212> PRT
   <213> Homo sapiens
<400> 59
   Met Leu Pro Gly Leu Ala Leu Leu Leu Leu Ala Ala Trp Thr Ala Arg
    1               5                  10                  15
   Ala Leu Glu Val Pro Thr Asp Gly Asn Ala Gly Leu Leu Ala Glu Pro
               20                  25                  30
   Gln Ile Ala Met Phe Cys Gly Arg Leu Asn Met His Met Asn Val Gln
           35                  40                  45
   Asn Gly Lys Trp Asp Ser Asp Pro Ser Gly Thr Lys Thr Cys Ile Asp
   50 55 60
   Thr Lys Glu Gly Ile Leu Gln Tyr Cys Gln Glu Val Tyr Pro Glu Leu
   65 70 75 80
   Gln Ile Thr Asn Val Val Glu Ala Asn Gln Pro Val Thr Ile Gln Asn
   85 90 95
   Trp Cys Lys Arg Gly Arg Lys Gln Cys Lys Thr His Pro His Phe Val
   100 105 110
   Ile Pro Tyr Arg Cys Leu Val Gly Glu Phe Val Ser Asp Ala Leu Leu
   115 120 125
   Val Pro Asp Lys Cys Lys Phe Leu His Gln Glu Arg Met Asp Val Cys
   130 135 140
   Glu Thr His Leu His Trp His Thr Val Ala Lys Glu Thr Cys Ser Glu
   145 150 155 160
   Lys Ser Thr Asn Leu His Asp Tyr Gly Met Leu Leu Pro Cys Gly Ile
   165 170 175
   Asp Lys Phe Arg Gly Val Glu Phe Val Cys Cys Pro Leu Ala Glu Glu
   180 185 190
   Ser Asp Asn Val Asp Ser Ala Asp Ala Glu Glu Asp Asp Ser Asp Val
   195 200 205
   Trp Trp Gly Gly Ala Asp Thr Asp Tyr Ala Asp Gly Ser Glu Asp Lys
   210 215 220
   Val Val Glu Val Ala Glu Glu Glu Glu Val Ala Glu Val Glu Glu Glu
   225 230 235 240
   Glu Ala Asp Asp Asp Glu Asp Asp Glu Asp Gly Asp Glu Val Glu Glu
   245 250 255
   Glu Ala Glu Glu Pro Tyr Glu Glu Ala Thr Glu Arg Thr Thr Ser Ile
   260 265 270
   Ala Thr Thr Thr Thr Thr Thr Thr Glu Ser Val Glu Glu Val Val Arg
   275 280 285
   Glu Val Cys Ser Glu Gln Ala Glu Thr Gly Pro Cys Arg Ala Met Ile
   290 295 300
   Ser Arg Trp Tyr Phe Asp Val Thr Glu Gly Lys Cys Ala Pro Phe Phe
   305 310 315 320
   Tyr Gly Gly Cys Gly Gly Asn Arg Asn Asn Phe Asp Thr Glu Glu Tyr
   325 330 335
   Cys Met Ala Val Cys Gly Ser Ala Met Ser Gln Ser Leu Leu Lys Thr
   340 345 350
   Thr Gln Glu Pro Leu Ala Arg Asp Pro Val Lys Leu Pro Thr Thr Ala
   355 360 365
   Ala Ser Thr Pro Asp Ala Val Asp Lys Tyr Leu Glu Thr Pro Gly Asp
   370 375 380
   Glu Asn Glu His Ala His Phe Gln Lys Ala Lys Glu Arg Leu Glu Ala
   385 390 395 400
   Lys His Arg Glu Arg Met Ser Gln Val Met Arg Glu Trp Glu Glu Ala
   405 410 415
   Glu Arg Gln Ala Lys Asn Leu Pro Lys Ala Asp Lys Lys Ala Val Ile
   420 425 430
   Gln His Phe Gln Glu Lys Val Glu Ser Leu Glu Gln Glu Ala Ala Asn
   435 440 445
   Glu Arg Gln Gln Leu Val Glu Thr His Met Ala Arg Val Glu Ala Met
   450 455 460
   Leu Asn Asp Arg Arg Arg Leu Ala Leu Glu Asn Tyr Ile Thr Ala Leu
   465 470 475 480
   Gln Ala Val Pro Pro Arg Pro Arg His Val Phe Asn Met Leu Lys Lys
   485 490 495
   Tyr Val Arg Ala Glu Gln Lys Asp Arg Gln His Thr Leu Lys His Phe
   500 505 510
   Glu His Val Arg Met Val Asp Pro Lys Lys Ala Ala Gln Ile Arg Ser
   515 520 525
   Gln Val Met Thr His Leu Arg Val Ile Tyr Glu Arg Met Asn Gln Ser
   530 535 540
   Leu Ser Leu Leu Tyr Asn Val Pro Ala Val Ala Glu Glu Ile Gln Asp
   545 550 555 560
   Glu Val Asp Glu Leu Leu Gln Lys Glu Gln Asn Tyr Ser Asp Asp Val
   565 570 575
   Leu Ala Asn Met Ile Ser Glu Pro Arg Ile Ser Tyr Gly Asn Asp Ala
   580 585 590
   Leu Met Pro Ser Leu Thr Glu Thr Lys Thr Thr Val Glu Leu Leu Pro
   595 600 605
   Val Asn Gly Glu Phe Ser Leu Asp Asp Leu Gln Pro Trp His Ser Phe
   610 615 620
   Gly Ala Asp Ser Val Pro Ala Asn Thr Glu Asn Glu Val Glu Pro Val
   625 630 635 640
   Asp Ala Arg Pro Ala Ala Asp Arg Gly Leu Thr Thr Arg Pro Gly Ser
   645 650 655
   Gly Leu Thr Asn Ile Lys Thr Glu Glu Ile Ser Glu Val Lys Met Asp
   660 665 670
   Ala Glu Phe Arg His Asp Ser Gly Tyr Glu Val His His Gln Lys Leu
   675 680 685
   Val Phe Phe Ala Glu Asp Val Gly Ser Asn Lys Gly Ala Ile Ile Gly
   690 695 700
   Leu Met Val Gly Gly Val Val Ile Ala Thr Val Ile Val Ile Thr Leu
   705 710 715 720
   Val Met Leu Lys Lys Lys Gln Tyr Thr Ser Ile His His Gly Val Val
   725 730 735
   Glu Val Asp Ala Ala Val Thr Pro Glu Glu Arg His Leu Ser Lys Met
   740 745 750
   Gln Gln Asn Gly Tyr Glu Asn Pro Thr Tyr Lys Phe Phe Glu Gln Met
   755 760 765
   Gln Asn Lys Lys
   770
<210> 60
   <211> 2259
   <212> DNA
   <213> Homo sapiens
<400> 60
   atgctgcccg gtttggcact gctcctgctg gccgcctgga cggctcgggc gctggaggta 60
   cccactgatg gtaatgctgg cctgctggct gaaccccaga ttgccatgtt ctgtggcaga 120
   ctgaacatgc acatgaatgt ccagaatggg aagtgggatt cagatccatc agggaccaaa 180
   acctgcattg ataccaagga aggcatcctg cagtattgcc aagaagtcta ccctgaactg 240
   cagatcacca atgtggtaga agccaaccaa ccagtgacca tccagaactg gtgcaagcgg 300
   ggccgcaagc agtgcaagac ccatccccac tttgtgattc cctaccgctg cttagttggt 360
   gagtttgtaa gtgatgccct tctcgttcct gacaagtgca aattcttaca ccaggagagg 420
   atggatgttt gcgaaactca tcttcactgg cacaccgtcg ccaaagagac atgcagtgag 480
   aagagtacca acttgcatga ctacggcatg ttgctgccct gcggaattga caagttccga 540
   ggggtagagt ttgtgtgttg cccactggct gaagaaagtg acaatgtgga ttctgctgat 600
   gcggaggagg atgactcgga tgtctggtgg ggcggagcag acacagacta tgcagatggg 660
   agtgaagaca aagtagtaga agtagcagag gaggaagaag tggctgaggt ggaagaagaa 720
   gaagccgatg atgacgagga cgatgaggat ggtgatgagg tagaggaaga ggctgaggaa 780
   ccctacgaag aagccacaga gagaaccacc agcattgcca ccaccaccac caccaccaca 840
   gagtctgtgg aagaggtggt tcgagaggtg tgctctgaac aagccgagac ggggccgtgc 900
   cgagcaatga tctcccgctg gtactttgat gtgactgaag ggaagtgtgc cccattcttt 960
   tacggcggat gtggcggcaa ccggaacaac tttgacacag aagagtactg catggccgtg 1020
   tgtggcagcg ccattcctac aacagcagcc agtacccctg atgccgttga caagtatctc 1080
   gagacacctg gggatgagaa tgaacatgcc catttccaga aagccaaaga gaggcttgag 1140
   gccaagcacc gagagagaat gtcccaggtc atgagagaat gggaagaggc agaacgtcaa 1200
   gcaaagaact tgcctaaagc tgataagaag gcagttatcc agcatttcca ggagaaagtg 1260
   gaatctttgg aacaggaagc agccaacgag agacagcagc tggtggagac acacatggcc 1320
   agagtggaag ccatgctcaa tgaccgccgc cgcctggccc tggagaacta catcaccgct 1380
   ctgcaggctg ttcctcctcg gcctcgtcac gtgttcaata tgctaaagaa gtatgtccgc 1440
   gcagaacaga aggacagaca gcacacccta aagcatttcg agcatgtgcg catggtggat 1500
   cccaagaaag ccgctcagat ccggtcccag gttatgacac acctccgtgt gatttatgag 1560
   cgcatgaatc agtctctctc cctgctctac aacgtgcctg cagtggccga ggagattcag 1620
   gatgaagttg atgagctgct tcagaaagag caaaactatt cagatgacgt cttggccaac 1680
   atgattagtg aaccaaggat cagttacgga aacgatgctc tcatgccatc tttgaccgaa 1740
   acgaaaacca ccgtggagct ccttcccgtg aatggagagt tcagcctgga cgatctccag 1800
   ccgtggcatt cttttggggc tgactctgtg ccagccaaca cagaaaacga agttgagcct 1860
   gttgatgccc gccctgctgc cgaccgagga ctgaccactc gaccaggttc tgggttgaca 1920
   aatatcaaga cggaggagat ctctgaagtg aagatggatg cagaattccg acatgactca 1980
   ggatatgaag ttcatcatca aaaattggtg ttctttgcag aagatgtggg ttcaaacaaa 2040
   ggtgcaatca ttggactcat ggtgggcggt gttgtcatag cgacagtgat cgtcatcacc 2100
   ttggtgatgc tgaagaagaa acagtacaca tccattcatc atggtgtggt ggaggttgac 2160
   gccgctgtca ccccagagga gcgccacctg tccaagatgc agcagaacgg ctacgaaaat 2220
   ccaacctaca agttctttga gcagatgcag aacaagaag                         2259
<210> 61
   <211> 753
   <212> PRT
   <213> Homo sapiens
<400> 61
   Met Leu Pro Gly Leu Ala Leu Leu Leu Leu Ala Ala Trp Thr Ala Arg
   1               5                   10                  15
   Ala Leu Glu Val Pro Thr Asp Gly Asn Ala Gly Leu Leu Ala Glu Pro
               20                  25                  30
   Gln Ile Ala Met Phe Cys Gly Arg Leu Asn Met His Met Asn Val Gln
           35                  40                  45
   Asn Gly Lys Trp Asp Ser Asp Pro Ser Gly Thr Lys Thr Cys Ile Asp
       50                  55                  60
   Thr Lys Glu Gly Ile Leu Gln Tyr Cys Gln Glu Val Tyr Pro Glu Leu
   65                  70                  75                  80
   Gln Ile Thr Asn Val Val Glu Ala Asn Gln Pro Val Thr Ile Gln Asn
                   85                  90                  95
   Trp Cys Lys Arg Gly Arg Lys Gln Cys Lys Thr His Pro His Phe Val
               100                 105                 110
   Ile Pro Tyr Arg Cys Leu Val Gly Glu Phe Val Ser Asp Ala Leu Leu
           115                 120                 125
   Val Pro Asp Lys Cys Lys Phe Leu His Gln Glu Arg Met Asp Val Cys
       130                 135                 140
   Glu Thr His Leu His Trp His Thr Val Ala Lys Glu Thr Cys Ser Glu
   145                 150                 155                 160
   Lys Ser Thr Asn Leu His Asp Tyr Gly Met Leu Leu Pro Cys Gly Ile
                   165                 170                 175
   Asp Lys Phe Arg Gly Val Glu Phe Val Cys Cys Pro Leu Ala Glu Glu
               180                 185                 190
   Ser Asp Asn Val Asp Ser Ala Asp Ala Glu Glu Asp Asp Ser Asp Val
           195                 200                 205
   Trp Trp Gly Gly Ala Asp Thr Asp Tyr Ala Asp Gly Ser Glu Asp Lys
       210                 215                 220
   Val Val Glu Val Ala Glu Glu Glu Glu Val Ala Glu Val Glu Glu Glu
   225                 230                 235                 240
   Glu Ala Asp Asp Asp Glu Asp Asp Glu Asp Gly Asp Glu Val Glu Glu
                   245                 250                 255
   Glu Ala Glu Glu Pro Tyr Glu Glu Ala Thr Glu Arg Thr Thr Ser Ile
               260                 265                 270
   Ala Thr Thr Thr Thr Thr Thr Thr Glu Ser Val Glu Glu Val Val Arg
           275                 280                 285
   Glu Val Cys Ser Glu Gln Ala Glu Thr Gly Pro Cys Arg Ala Met Ile
       290                 295                 300
   Ser Arg Trp Tyr Phe Asp Val Thr Glu Gly Lys Cys Ala Pro Phe Phe
   305                 310                 315                 320
   Tyr Gly Gly Cys Gly Gly Asn Arg Asn Asn Phe Asp Thr Glu Glu Tyr
   325 330 335
   Cys Met Ala Val Cys Gly Ser Ala Ile Pro Thr Thr Ala Ala Ser Thr
   340 345 350
   Pro Asp Ala Val Asp Lys Tyr Leu Glu Thr Pro Gly Asp Glu Asn Glu
   355 360 365
   His Ala His Phe Gln Lys Ala Lys Glu Arg Leu Glu Ala Lys His Arg
   370 375 380
   Glu Arg Met Ser Gln Val Met Arg Glu Trp Glu Glu Ala Glu Arg Gln
   385 390 395 400
   Ala Lys Asn Leu Pro Lys Ala Asp Lys Lys Ala Val Ile Gln His Phe
   405 410 415
   Gln Glu Lys Val Glu Ser Leu Glu Gln Glu Ala Ala Asn Glu Arg Gln
   420 425 430
   Gln Leu Val Glu Thr His Met Ala Arg Val Glu Ala Met Leu Asn Asp
   435 440 445
   Arg Arg Arg Leu Ala Leu Glu Asn Tyr Ile Thr Ala Leu Gln Ala Val
   450 455 460
   Pro Pro Arg Pro Arg His Val Phe Asn Met Leu Lys Lys Tyr Val Arg
   465 470 475 480
   Ala Glu Gln Lys Asp Arg Gln His Thr Leu Lys His Phe Glu His Val
   485 490 495
   Arg Met Val Asp Pro Lys Lys Ala Ala Gln Ile Arg Ser Gln Val Met
   500 505 510
   Thr His Leu Arg Val Ile Tyr Glu Arg Met Asn Gln Ser Leu Ser Leu
   515 520 525
   Leu Tyr Asn Val Pro Ala Val Ala Glu Glu Ile Gln Asp Glu Val Asp
   530 535 540
   Glu Leu Leu Gln Lys Glu Gln Asn Tyr Ser Asp Asp Val Leu Ala Asn
   545 550 555 560
   Met Ile Ser Glu Pro Arg Ile Ser Tyr Gly Asn Asp Ala Leu Met Pro
   565 570 575
   Ser Leu Thr Glu Thr Lys Thr Thr Val Glu Leu Leu Pro Val Asn Gly
   580 585 590
   Glu Phe Ser Leu Asp Asp Leu Gln Pro Trp His Ser Phe Gly Ala Asp
   595 600 605
   Ser Val Pro Ala Asn Thr Glu Asn Glu Val Glu Pro Val Asp Ala Arg
   610 615 620
   Pro Ala Ala Asp Arg Gly Leu Thr Thr Arg Pro Gly Ser Gly Leu Thr
   625 630 635 640
   Asn Ile Lys Thr Glu Glu Ile Ser Glu Val Lys Met Asp Ala Glu Phe
   645 650 655
   Arg His Asp Ser Gly Tyr Glu Val His His Gln Lys Leu Val Phe Phe
   660 665 670
   Ala Glu Asp Val Gly Ser Asn Lys Gly Ala Ile Ile Gly Leu Met Val
   675 680 685
   Gly Gly Val Val Ile Ala Thr Val Ile Val Ile Thr Leu Val Met Leu
   690 695 700
   Lys Lys Lys Gln Tyr Thr Ser Ile His His Gly Val Val Glu Val Asp
   705 710 715 720
   Ala Ala Val Thr Pro Glu Glu Arg His Leu Ser Lys Met Gln Gln Asn
   725 730 735
   Gly Tyr Glu Asn Pro Thr Tyr Lys Phe Phe Glu Gln Met Gln Asn Lys
   740 745 750
   Lys
<210> 62
   <211> 8
   <212> PRT
   <213> Artificial Sequence
<220>
   <223> Description of Artificial Sequence: synthetic
<400> 62
   Leu Glu Val Leu Phe Gln Gly Pro
   1 5
<210> 63
   <211> 10
   <212> PRT
   <213> Artificial Sequence
<220>
   <223> Description of Artificial Sequence: synthetic
<400> 63
   Ser Glu Val Asn Leu Asp Ala Glu Phe Arg
   1 5 10
<210> 64
   <211> 10
   <212> PRT
   <213> Artificial Sequence
<220>
   <223> Description of Artificial Sequence: synthetic
<400> 64
   Ser Glu Val Lys Met Asp Ala Glu Phe Arg
   1 5 10
<210> 65
   <211> 15
   <212> PRT
   <213> Artificial Sequence
<220>
   <223> Description of Artificial Sequence: synthetic
<400> 65
   Arg Arg Gly Gly Val Val Ile Ala Thr Val Ile Val Gly Glu Arg
   1 5 10 15
<210> 66
   <211> 518
   <212> PRT
   <213> Homo sapiens
<400> 66
   Met Gly Ala Leu Ala Arg Ala Leu Leu Leu Pro Leu Leu Ala Gln Trp
    1               5                  10                  15
   Leu Leu Arg Ala Ala Pro Glu Leu Ala Pro Ala Pro Phe Thr Leu Pro
               20                  25                  30
   Leu Arg Val Ala Ala Ala Thr Asn Arg Val Val Ala Pro Thr Pro Gly
           35                  40                  45
   Pro Gly Thr Pro Ala Glu Arg His Ala Asp Gly Leu Ala Leu Ala Leu
       50                  55                  60
   Glu Pro Ala Leu Ala Ser Pro Ala Gly Ala Ala Asn Phe Leu Ala Met
   65                  70                  75                  80
   Val Asp Asn Leu Gln Gly Asp Ser Gly Arg Gly Tyr Tyr Leu Glu Met
                   85                  90                  95
   Leu Ile Gly Thr Pro Pro Gln Lys Leu Gln Ile Leu Val Asp Thr Gly
               100                 105                 110
   Ser Ser Asn Phe Ala Val Ala Gly Thr Pro His Ser Tyr Ile Asp Thr
           115                 120                 125
   Tyr Phe Asp Thr Glu Arg Ser Ser Thr Tyr Arg Ser Lys Gly Phe Asp
       130                 135                 140
   Val Thr Val Lys Tyr Thr Gln Gly Ser Trp Thr Gly Phe Val Gly Glu
   145                 150                 155                 160
   Asp Leu Val Thr Ile Pro Lys Gly Phe Asn Thr Ser Phe Leu Val Asn
                   165                 170                 175
   Ile Ala Thr Ile Phe Glu Ser Glu Asn Phe Phe Leu Pro Gly Ile Lys
               180                 185                 190
   Trp Asn Gly Ile Leu Gly Leu Ala Tyr Ala Thr Leu Ala Lys Pro Ser
           195                 200                 205
   Ser Ser Leu Glu Thr Phe Phe Asp Ser Leu Val Thr Gln Ala Asn Ile
       210                 215                 220
   Pro Asn Val Phe Ser Met Gln Met Cys Gly Ala Gly Leu Pro Val Ala
   225                 230                 235                 240
   Gly Ser Gly Thr Asn Gly Gly Ser Leu Val Leu Gly Gly Ile Glu Pro
   245 250 255
   Ser Leu Tyr Lys Gly Asp Ile Trp Tyr Thr Pro Ile Lys Glu Glu Trp
   260 265 270
   Tyr Tyr Gln Ile Glu Ile Leu Lys Leu Glu Ile Gly Gly Gln Ser Leu
   275 280 285
   Asn Leu Asp Cys Arg Glu Tyr Asn Ala Asp Lys Ala Ile Val Asp Ser
   290 295 300
   Gly Thr Thr Leu Leu Arg Leu Pro Gln Lys Val Phe Asp Ala Val Val
   305 310 315 320
   Glu Ala Val Ala Arg Ala Ser Leu Ile Pro Glu Phe Ser Asp Gly Phe
   325 330 335
   Trp Thr Gly Ser Gln Leu Ala Cys Trp Thr Asn Ser Glu Thr Pro Trp
   340 345 350
   Ser Tyr Phe Pro Lys Ile Ser Ile Tyr Leu Arg Asp Glu Asn Ser Ser
   355 360 365
   Arg Ser Phe Arg Ile Thr Ile Leu Pro Gln Leu Tyr Ile Gln Pro Met
   370 375 380
   Met Gly Ala Gly Leu Asn Tyr Glu Cys Tyr Arg Phe Gly Ile Ser Pro
   385 390 395 400
   Ser Thr Asn Ala Leu Val Ile Gly Ala Thr Val Met Glu Gly Phe Tyr
   405 410 415
   Val Ile Phe Asp Arg Ala Gln Lys Arg Val Gly Phe Ala Ala Ser Pro
   420 425 430
   Cys Ala Glu Ile Ala Gly Ala Ala Val Ser Glu Ile Ser Gly Pro Phe
   435 440 445
   Ser Thr Glu Asp Val Ala Ser Asn Cys Val Pro Ala Gln Ser Leu Ser
   450 455 460
   Glu Pro Ile Leu Trp Ile Val Ser Tyr Ala Leu Met Ser Val Cys Gly
   465 470 475 480
   Ala Ile Leu Leu Val Leu Ile Val Leu Leu Leu Leu Pro Phe Arg Cys
   485 490 495
   Gln Arg Arg Pro Arg Asp Pro Glu Val Val Asn Asp Glu Ser Ser Leu
   500 505 510
   Val Arg His Arg Trp Lys
   515
<210> 67
   <211> 475
   <212> PRT
   <213> Homo sapiens
<400> 67
   Met Gly Ala Leu Ala Arg Ala Leu Leu Leu Pro Leu Leu Ala Gln Trp
   1 5 10 15
   Leu Leu Arg Ala Ala Pro Glu Leu Ala Pro Ala Pro Phe Thr Leu Pro
                   20                  25                  30
   Leu Arg Val Ala Ala Ala Thr Asn Arg Val Val Ala Pro Thr Pro Gly
               35                  40                  45
   Pro Gly Thr Pro Ala Glu Arg His Ala Asp Gly Leu Ala Leu Ala Leu
       50                  55                  60
   Glu Pro Ala Leu Ala Ser Pro Ala Gly Ala Ala Asn Phe Leu Ala Met
   65                  70                  75                  80
   Val Asp Asn Leu Gln Gly Asp Ser Gly Arg Gly Tyr Tyr Leu Glu Met
                   85                  90                  95
   Leu Ile Gly Thr Pro Pro Gln Lys Leu Gln Ile Leu Val Asp Thr Gly
               100                 105                 110
   Ser Ser Asn Phe Ala Val Ala Gly Thr Pro His Ser Tyr Ile Asp Thr
           115                 120                 125
   Tyr Phe Asp Thr Glu Arg Ser Ser Thr Tyr Arg Ser Lys Gly Phe Asp
       130                 135                 140
   Val Thr Val Lys Tyr Thr Gln Gly Ser Trp Thr Gly Phe Val Gly Glu
   145                 150                 155                 160
   Asp Leu Val Thr Ile Pro Lys Gly Phe Asn Thr Ser Phe Leu Val Asn
                   165                 170                 175
   Ile Ala Thr Ile Phe Glu Ser Glu Asn Phe Phe Leu Pro Gly Ile Lys
               180                 185                 190
   Trp Asn Gly Ile Leu Gly Leu Ala Tyr Ala Thr Leu Ala Lys Pro Ser
           195                 200                 205
   Ser Ser Leu Glu Thr Phe Phe Asp Ser Leu Val Thr Gln Ala Asn Ile
       210                 215                 220
   Pro Asn Val Phe Ser Met Gln Met Cys Gly Ala Gly Leu Pro Val Ala
   225                 230                 235                 240
   Gly Ser Gly Thr Asn Gly Gly Ser Leu Val Leu Gly Gly Ile Glu Pro
                   245                 250                 255
   Ser Leu Tyr Lys Gly Asp Ile Trp Tyr Thr Pro Ile Lys Glu Glu Trp
               260                 265                 270
   Tyr Tyr Gln Ile Glu Ile Leu Lys Leu Glu Ile Gly Gly Gln Ser Leu
           275                 280                 285
   Asn Leu Asp Cys Arg Glu Tyr Asn Ala Asp Lys Ala Ile Val Asp Ser
       290                 295                 300
   Gly Thr Thr Leu Leu Arg Leu Pro Gln Lys Val Phe Asp Ala Val Val
   305                 310                 315                 320
   Glu Ala Val Ala Arg Ala Ser Leu Ile Pro Glu Phe Ser Asp Gly Phe
                   325                 330                 335
   Trp Thr Gly Ser Gln Leu Ala Cys Trp Thr Asn Ser Glu Thr Pro Trp
               340                 345                 350
   Ser Tyr Phe Pro Lys Ile Ser Ile Tyr Leu Arg Asp Glu Asn Ser Ser
           355                 360                 365
   Arg Ser Phe Arg Ile Thr Ile Leu Pro Gln Leu Tyr Ile Gln Pro Met
       370                 375                 380
   Met Gly Ala Gly Leu Asn Tyr Glu Cys Tyr Arg Phe Gly Ile Ser Pro
   385                 390                 395                 400
   Ser Thr Asn Ala Leu Val Ile Gly Ala Thr Val Met Glu Gly Phe Tyr
                   405                 410                 415
   Val Ile Phe Asp Arg Ala Gln Lys Arg Val Gly Phe Ala Ala Ser Pro
               420                 425                 430
   Cys Ala Glu Ile Ala Gly Ala Ala Val Ser Glu Ile Ser Gly Pro Phe
           435                 440                 445
   Ser Thr Glu Asp Val Ala Ser Asn Cys Val Pro Ala Gln Ser Leu Ser
       450                 455                 460
   Glu Pro Ile Leu Trp His His His His His His
   465                 470                 475
<210> 68
   <211> 413
   <212> PRT
   <213> Homo sapiens
<400> 68
   Ala Leu Glu Pro Ala Leu Ala Ser Pro Ala Gly Ala Ala Asn Phe Leu
    1               5                  10                  15
   Ala Met Val Asp Asn Leu Gln Gly Asp Ser Gly Arg Gly Tyr Tyr Leu
               20                  25                  30
   Glu Met Leu Ile Gly Thr Pro Pro Gln Lys Leu Gln Ile Leu Val Asp
           35                  40                  45
   Thr Gly Ser Ser Asn Phe Ala Val Ala Gly Thr Pro His Ser Tyr Ile
       50                  55                  60
   Asp Thr Tyr Phe Asp Thr Glu Arg Ser Ser Thr Tyr Arg Ser Lys Gly
   65                  70                  75                  80
   Phe Asp Val Thr Val Lys Tyr Thr Gln Gly Ser Trp Thr Gly Phe Val
                   85                  90                  95
   Gly Glu Asp Leu Val Thr Ile Pro Lys Gly Phe Asn Thr Ser Phe Leu
               100                 105                 110
   Val Asn Ile Ala Thr Ile Phe Glu Ser Glu Asn Phe Phe Leu Pro Gly
           115                 120                 125
   Ile Lys Trp Asn Gly Ile Leu Gly Leu Ala Tyr Ala Thr Leu Ala Lys
       130                 135                 140
   Pro Ser Ser Ser Leu Glu Thr Phe Phe Asp Ser Leu Val Thr Gln Ala
   145                 150                 155                 160
   Asn Ile Pro Asn Val Phe Ser Met Gln Met Cys Gly Ala Gly Leu Pro
                   165                 170                 175
   Val Ala Gly Ser Gly Thr Asn Gly Gly Ser Leu Val Leu Gly Gly Ile
                   180                 185                 190
   Glu Pro Ser Leu Tyr Lys Gly Asp Ile Trp Tyr Thr Pro Ile Lys Glu
           195                 200                 205
   Glu Trp Tyr Tyr Gln Ile Glu Ile Leu Lys Leu Glu Ile Gly Gly Gln
       210                 215                 220
   Ser Leu Asn Leu Asp Cys Arg Glu Tyr Asn Ala Asp Lys Ala Ile Val
   225                 230                 235                 240
   Asp Ser Gly Thr Thr Leu Leu Arg Leu Pro Gln Lys Val Phe Asp Ala
                   245                 250                 255
   Val Val Glu Ala Val Ala Arg Ala Ser Leu Ile Pro Glu Phe Ser Asp
               260                 265                 270
   Gly Phe Trp Thr Gly Ser Gln Leu Ala Cys Trp Thr Asn Ser Glu Thr
           275                 280                 285
   Pro Trp Ser Tyr Phe Pro Lys Ile Ser Ile Tyr Leu Arg Asp Glu Asn
       290                 295                 300
   Ser Ser Arg Ser Phe Arg Ile Thr Ile Leu Pro Gln Leu Tyr Ile Gln
   305                 310                 315                 320
   Pro Met Met Gly Ala Gly Leu Asn Tyr Glu Cys Tyr Arg Phe Gly Ile
                   325                 330                 335
   Ser Pro Ser Thr Asn Ala Leu Val Ile Gly Ala Thr Val Met Glu Gly
               340                 345                 350
   Phe Tyr Val Ile Phe Asp Arg Ala Gln Lys Arg Val Gly Phe Ala Ala
           355                 360                 365
   Ser Pro Cys Ala Glu Ile Ala Gly Ala Ala Val Ser Glu Ile Ser Gly
       370                 375                 380
   Pro Phe Ser Thr Glu Asp Val Ala Ser Asn Cys Val Pro Ala Gln Ser
   385                 390                 395                 400
   Leu Ser Glu Pro Ile Leu Trp His His His His His His
                   405                 410
<210> 69
   <211> 8
   <212> PRT
   <213> Artificial Sequence
<220>
   <223> Description of Artificial Sequence: Peptide
<400> 69
   Gly Leu Ala Leu Ala Leu Glu Pro
     1               5
<210> 70
   <211> 8
   <212> PRT
   <213> Artificial Sequence
<220>
   <223> Description of Artificial Sequence: Peptide
<400> 70
   Glu Val Lys Met Asp Ala Glu Phe
   1 5
<210> 71
   <211> 8
   <212> PRT
   <213> Artificial Sequence
<220>
   <223> Description of Artificial Sequence: Peptide
<400> 71
   Glu Val Asn Leu Asp Ala Glu Phe
   1 5
<210> 72
   <211> 8
   <212> PRT
   <213> Artificial Sequence
<220>
   <223> Description of Artificial Sequence: Peptide
<400> 72
   Leu Val Phe Phe Ala Glu Asp Val
   1 5
<210> 73
   <211> 8
   <212> PRT
   <213> Artificial Sequence
<220>
   <223> Description of Artificial Sequence: Peptide
<400> 73
   Lys Leu Val Phe Phe Ala Glu Asp
   1 5
<210> 74
   <211> 38
   <212> DNA
   <213> Artificial Sequence
<220>
   <223> Description of Artificial Sequence: Primer
<400> 74
   cgctttaagc ttgccaccat gggcgcactg gcccgggcg 39
<210> 75
   <211> 57
   <212> DNA
   <213> Artificial Sequence
<220>
   <223> Description of Artificial Sequence: Primer
<400> 75
   cgctttctcg agctaatggt gatggtgatg gtgccacaaa atgggctcgc tcaaaga 357
<210> 76
   <211> 15
   <212> PRT
   <213> Artificial Sequence
<220>
   <223> Description of Artificial Sequence: Peptide
<400> 76
   Arg Arg Gly Gly Val Val Ile Ala Thr Val Ile Val Gly Glu Arg
    1               5                  10                  15